# EUROPÄISCHE PATENTSCHRIFT

(11) **EP 2 194 081 B1**
(45) Veröffentlichungstag und Bekanntmachung des Hinweises auf die Patenterteilung: **07.09.2011**
(21) Anmeldenummer: 08170474.4
(22) Anmeldetag: 02.12.2008
(51) Int. Cl.: C08G 18/08, C08G 18/12, C08G 18/32, C08G 18/36, C08G 18/48, C08G 18/66, C08G 18/76, C09J 175/12, C07C 251/08

(54) **Hydroxyaldimin enthaltende Polyurethanzusammensetzung**
Polyurethane composition comprising hydroxyaldimines
Composition de polyuréthane contenant des aldimines comprenant des groupes d'hydroxyle

(43) Veröffentlichungstag der Anmeldung: 09.06.2010
(73) Patentinhaber: Sika Technology AG, 6340 Baar (CH)
(72) Erfinder: Burckhardt, Urs, 8049 Zürich (CH); Stadelmann, Ursula, 8046 Zürich (CH)
(74) Vertreter: Sika Patent Attorneys

(56) Entgegenhaltungen:
- EP-A- 1 975 190
- WO-A-2007/036571
- DE-A1- 1 814 832

## Beschreibung

### Technisches Gebiet

Die vorliegende Erfindung betrifft das Gebiet der Polyurethanzusammensetzungen sowie deren Verwendung, insbesondere als zweikomponentiger Klebstoff, Dichtstoff, Vergussmasse, Beschichtung oder Bodenbelag.

### Stand der Technik

Isocyanatgruppen aufweisende Polyurethanzusammensetzungen werden seit langem in verschiedensten Anwendungen eingesetzt, unter anderem als Klebstoff, Dichtstoff, Vergussmasse, Beschichtung oder Bodenbelag. Während einkomponentige Polyurethanzusammensetzungen als solche eingesetzt werden können und nach ihrer Applikation durch Kontakt mit Feuchtigkeit und/oder mittels Wärme aushärten, bestehen zweikomponentige Polyurethanzusammensetzungen aus zwei separat voneinander gelagerten Komponenten - im allgemeinen einer Komponente mit freien Isocyanatgruppen und einer Komponente mit freien Hydroxylgruppen -, welche zur Verwendung der Zusammensetzung erst kurz vor oder während deren Applikation gut gemischt werden und daraufhin aushärten. Insbesondere für zweikomponentige Polyurethanzusammensetzungen wird vielfach gewünscht, dass die Zusammensetzungen bei einer ausreichend langen Topf- und Verarbeitungszeit möglichst schnell aushärten, d.h. nach der Applikation möglichst rasch Festigkeit aufbauen und dadurch schon nach kurzer Zeit belast- oder bearbeitbar werden, beispielsweise damit mit einem Klebstoff gefügte Teile bewegt oder fixierende Halterungen entfernt werden können, oder damit eine aufgetragene Beschichtung begangen, nachbeschichtet oder abgeschliffen werden kann.

Bei einer zweikomponentigen Polyurethanzusammensetzung kann die Aushärtung beispielsweise durch das Zuführen von Wärme oder durch die Verwendung von starken oder hochdosierten Katalysatoren beschleunigt werden. Die Zufuhr von Wärme kann aber einen erhöhten Applikationsaufwand bedingen, kann die Substrate schädigen und kann zu Blasenbildung und Haftverlust führen, und die Verwendung von starken oder hochdosierten Katalysatoren kann Einbussen bei der Haltbarkeit und der Langzeitstabilität der Zusammensetzung verursachen und führt insbesondere zu einer Verkürzung der Offenzeit der Zusammensetzung.

Zweikomponentige Polyurethanzusammensetzungen, deren Hydroxylgruppen-haltige Komponente zusätzlich freie primäre oder sekundäre Aminogruppen von mitenthaltenen Polyaminen aufweisen, härten besonders rasch aus, neigen dabei kaum zu Blasenbildung und zeigen sehr gute mechanische Eigenschaften, weil mehr Harnstoffgruppen entstehen, welche die Festigkeit und Elastizität von Polyurethanen generell erhöhen. Allerdings ist die Reaktivität von freien Aminogruppen gegenüber Isocyanatgruppen derart hoch, dass solche Zusammensetzungen für die meisten Anwendungen eine zu kurze Topf- und Verarbeitungszeit (Offenzeit) aufweisen. Ausserdem neigen Amine an der Luft oft zur Bildung von Krusten durch Reaktion mit CO₂ ("Blushing") und/oder weisen aufgrund ihrer Toxizität in Bezug auf Arbeitssicherheit und Ökologie Nachteile auf.

Anstelle von freien Aminen können sogenannte blockierte Amine mit hydrolytisch aktivierbaren Aminogruppen eingesetzt werden, zum Beispiel in Form von Iminen oder Oxazolidinen. Diese weisen kein Blushing auf und ermöglichen zweikomponentige Zusammensetzungen mit etwas längeren Offenzeiten. Allerdings führt die Verwendung von blockierten Aminen in Polyurethanzusammensetzungen zu anderen Schwierigkeiten, insbesondere bedingt durch die Tatsache, dass die zur Blockierung der Aminogruppen eingesetzten Substanzen, typischerweise Aldehyde oder Ketone, bei der Aushärtung der Zusammensetzung freigesetzt werden. Diese sind meistens flüchtig und geruchsintensiv und können unter anderem zu Geruchsbelästigungen und Reizungen führen.

Aus WO 2007/036571 A1 sind zweikomponentige Polyurethanzusammensetzungen enthaltend Aldimine mit zusätzlichen Reaktivgruppen bekannt, welche schnell und geruchsfrei aushärten. Diese Aldimine setzen bei der Aushärtung einen relativ schwerflüchtigen Aldehyd frei, welcher in der ausgehärteten Zusammensetzung verbleibt und auf diese eine mehr oder weniger ausgeprägte Weichmacherwirkung ausübt. Dadurch bleiben diese Zusammensetzungen hinsichtlich ihrer mechanischen Eigenschaften, insbesondere der erreichbaren Werte für das Elastizitätsmodul, beschränkt und deshalb nicht allen Anwendungen zugänglich.

### Darstellung der Erfindung

Aufgabe der vorliegenden Erfindung ist es daher, Polyurethanzusammensetzungen zur Verfügung zu stellen, die dank ausreichend langer Offenzeit einfach verarbeitbar sind, die trotzdem schnell und geruchsarm aushärten und damit rasch Festigkeit aufbauen, und die im ausgehärteten Zustand hohe Zugfestigkeiten und hohe Elastizitätsmodule aufweisen.

Überraschenderweise lässt sich diese Aufgabe mittels einer in Anspruch 1 offenbarten Zusammensetzung lösen. Diese Zusammensetzung enthält neben mindestens einem Polyisocyanat mindestens ein spezielles Aldimin. Dieses Aldimin weist zusätzlich zu mehreren Aldiminogruppen mehrere Hydroxyl-, Mercapto- oder sekundäre Aminogruppen auf und ist damit in der Lage, das Polyisocyanat auch in Abwesenheit von Feuchtigkeit zu vernetzen. Beim Zutritt von Feuchtigkeit, welcher spätestens bei der Applikation der Zusammensetzung durch Luftkontakt erfolgt, kommt es zur hydrolytischen Aktivierung der Aldiminogruppen und deren Reaktion mit vorhandenen Isocyanatgruppen. Dabei wird ein geruchsarmer bis geruchsloser Polyaldehyd von geringer Flüchtigkeit freigesetzt, welcher die ausgehärtete Zusammensetzung wenig oder gar nicht weichmacht. Aufgrund der speziellen Struktur des Aldimins, insbesondere in dessen bevorzugten Ausführungsformen, sind insbesondere zweikomponentige Polyurethanzusammensetzungen erhältlich, welche zum einen eine ausreichend lange und damit eine einfache Verarbeitung gewährleistende Offenzeit aufweisen, zum anderen ohne Geruchsbildung schnell und blasenfrei aushärten und sehr rasch Festigkeit aufbauen, und schliesslich im ausgehärteten Zustand über ausgezeichnete mechanische Eigenschaften, insbesondere über eine hohe Zugfestigkeit und ein hohes Elastizitätsmodul, verfügen.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist eine Zusammensetzung, enthaltend
a) mindestens ein Aldimin **A** der Formel (I), 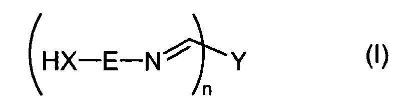 wobei
   n für 2 oder 3 oder 4 steht,
   E entweder für einen zweiwertigen Kohlenwasserstoffrest mit 3 bis 20 C-Atomen steht, oder zusammen mit R¹¹ für einen dreiwertigen Kohlenwasserstoffrest mit 3 bis 20 C-Atomen steht, wobei E gegebenenfalls Heteroatome in der Form von Ether-Sauerstoff oder tertiärem Amin-Stickstoff enthält,
   Y für einen n-wertigen organischen Rest mit 6 bis 30 C-Atomen, welcher gegebenenfalls Stickstoff- und/oder Sauerstoffatome in Form von tertiären Aminogruppen, Ether-, Ester-, Carbonat-, Amid-, Urethan- oder Harnstoffgruppen aufweist, steht,
   X für O oder S oder N-R¹⁰ oder N-R¹¹ steht,
   wobei
   R¹⁰ für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht, und
   R¹¹ zusammen mit E für einen dreiwertigen Kohlenwasserstoffrest mit 3 bis 20 C-Atomen, welcher gegebenenfalls Heteroatome in der Form von Ether-Sauerstoff oder tertiärem Amin-Stickstoff enthält, steht;
   und
b) mindestens ein Polyisocyanat **P.**

Mit "Poly" beginnende Substanznamen wie Polyol, Polyisocyanat oder Polyaldehyd bezeichnen im vorliegenden Dokument Substanzen, die formal zwei oder mehr der in ihrem Namen vorkommenden funktionellen Gruppen pro Molekül enthalten.

Der Begriff "Polyisocyanat" umfasst im vorliegenden Dokument Verbindungen mit zwei oder mehr Isocyanatgruppen, unabhängig davon, ob es sich dabei um monomere Diisocyanate, oligomere Polyisocyanate oder Isocyanatgruppen aufweisende Polymere mit einem relativ hohen Molekulargewicht handelt.

Der Begriff "primäre Aminogruppe" bezeichnet im vorliegenden Dokument eine Aminogruppe in der Form einer NH₂-Gruppe, die an einen organischen Rest gebunden ist. Der Begriff "sekundäre Aminogruppe" bezeichnet eine Aminogruppe, in der das Stickstoffatom an zwei organische Reste gebunden ist, welche auch gemeinsam Teil eines Rings sein können. Der Begriff "tertiäre Aminogruppe" bezeichnet eine Aminogruppe, in der das Stickstoffatom (= tertiärer Amin-Stickstoff) an drei organische Reste gebunden ist, wobei zwei dieser Reste auch gemeinsam Teil eines Rings sein können.

Der Begriff "Polymer" umfasst im vorliegenden Dokument einerseits ein Kollektiv von chemisch einheitlichen, sich aber in Bezug auf Polymerisationsgrad, Molmasse und Kettenlänge unterscheidenden Makromolekülen, das durch eine Polyreaktion (Polymerisation, Polyaddition, Polykondensation) hergestellt wurde. Der Begriff umfasst andererseits auch Derivate eines solchen Kollektivs von Makromolekülen aus Polyreaktionen, Verbindungen also, die durch Umsetzungen, wie beispielsweise Additionen oder Substitutionen, von funktionellen Gruppen an vorgegebenen Makromolekülen erhalten wurden und die chemisch einheitlich oder chemisch uneinheitlich sein können. Der Begriff umfasst im Weiteren auch sogenannte Prepolymere, das heisst reaktive oligomere Voraddukte, deren funktionelle Gruppen am Aufbau von Makromolekülen beteiligt sind.

Der Begriff "Polyurethanpolymer" umfasst sämtliche Polymere, welche nach dem sogenannten Diisocyanat-Polyadditions-Verfahren hergestellt werden. Dies schliesst auch solche Polymere ein, die nahezu oder gänzlich frei sind von Urethangruppen. Beispiele für Polyurethanpolymere sind Polyether-Polyurethane, Polyester-Polyurethane, Polyether-Polyharnstoffe, Polyharnstoffe, Polyester-Polyharnstoffe, Polyisocyanurate und Polycarbodiimide.

Als "aliphatisch" wird ein Amin, ein Aldehyd und ein Isocyanat bezeichnet, deren Amino-, Aldehyd- und Isocyanatgruppen jeweils ausschliesslich an aliphatische, cycloaliphatische oder arylaliphatische Reste gebunden sind; entsprechend werden diese Gruppen als aliphatische Amino-, Aldehyd- und Isocyanatgruppen bezeichnet.

Als "aromatisch" wird ein Amin, ein Aldehyd und ein Isocyanat bezeichnet, deren Amino-, Aldehyd- und Isocyanatgruppen jeweils an einen aromatischen Rest gebunden sind; entsprechend werden diese Gruppen als aromatische Amino-, Aldehyd- und Isocyanatgruppen bezeichnet.

Die Zusammensetzung enthält mindestens ein Aldimin **A** der Formel (I). 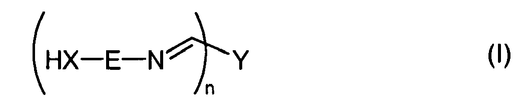 Bevorzugt steht X für O, also für ein Sauerstoffatom.

Als Aldimin **A** der Formel (I) bevorzugt ist ein Aldimin **A1** der Formel (II), 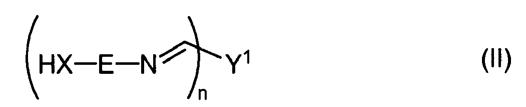 wobei
Y¹ für einen n-wertigen, substituierten oder unsubstituierten Aryl- oder Heteroaryl-Rest, welcher eine Ringgrösse von 5 bis 8, bevorzugt 6, Atomen aufweist, steht;
und n, X und E die bereits genannten Bedeutungen aufweisen.

Als Aldimin **A** der Formel (I) weiterhin bevorzugt ist ein Aldimin **A2** der Formel (III), 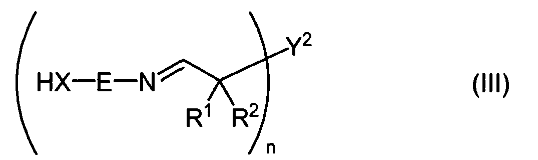 wobei
R¹ und R² entweder
unabhängig voneinander jeweils für einen einwertigen Kohlenwasserstoffrest mit 1 bis 12 C-Atomen stehen,
oder zusammen für einen zweiwertigen Kohlenwasserstoffrest mit 4 bis 12 C-Atomen, der Teil eines, gegebenenfalls substituierten, carbocyclischen Rings mit 5 bis 8, bevorzugt 6, C-Atomen ist, stehen;
Y² für einen n-wertigen organischen Rest mit 1 bis 24 C-Atomen steht, welcher gegebenenfalls Stickstoff- und/oder Sauerstoffatome in Form von tertiären Aminogruppen, Ether-, Ester-, Carbonat-, Amid-, Urethan- oder Harnstoffgruppen aufweist;
und n, X und E die bereits genannten Bedeutungen aufweisen.

Die Aldimine **A2** der Formel (III) sind bevorzugt gegenüber den Aldiminen **A1** der Formel (II).

Als Aldimin **A** der Formel (I), bzw. Aldimin **A2** der Formel (III), besonders bevorzugt sind Aldimine **A3** der Formel (III a), 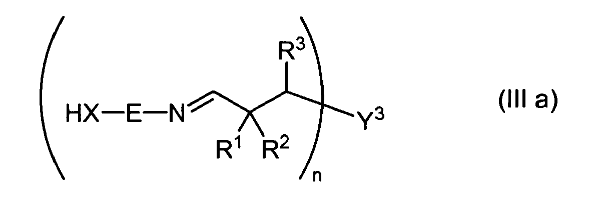 wobei
R³ für ein Wasserstoffatom oder für einen Alkyl-, Cycloalkyl-, Arylalkyl- oder Alkoxycarbonylrest mit 1 bis 12 C-Atomen steht;
Y³ für einen n-wertigen Rest steht, welcher ausgewählt ist aus der Gruppe bestehend aus 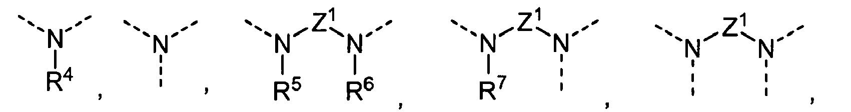 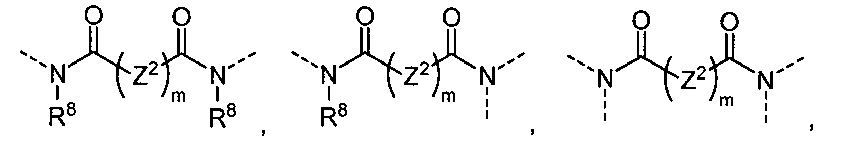 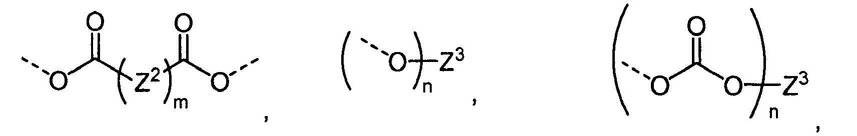 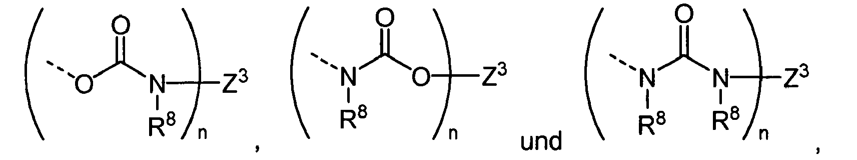 wobei
- m: für 0 oder 1 steht;
- Z¹: entweder für eine Carbonylgruppe oder für einen Alkylenrest mit 2 bis 15 C- Atomen, welcher gegebenenfalls mindestens eine Ether-Gruppe aufweist, steht;
- Z²: für einen zweiwertigen Kohlenwasserstoffrest mit 1 bis 15 C-Atomen, welcher gegebenenfalls mindestens eine Ether-, Carbonyl- oder Carboxylgruppe aufweist, steht;
- Z³: für einen n-wertigen Kohlenwasserstoffrest mit 2 bis 15 C-Atomen, welcher gegebenenfalls mindestens eine Ether- oder Carbonylgruppe aufweist, steht;
- R⁴: für einen Alkyl-, Cycloalkyl- oder Arylalkylrest mit 1 bis 20 C-Atomen steht;
- R⁵ und R⁶: entweder unabhängig voneinander jeweils für einen Alkyl-, Cycloalkyl- oder Arylalkylrest mit 1 bis 12 C-Atomen stehen, oder, für den Fall dass Z¹ für eine Carbonylgruppe steht, unabhängig voneinander jeweils für ein Wasserstoffatom oder für einen Alkyl-, Cycloalkyl- oder Arylalkylrest mit 1 bis 12 C-Atomen stehen; oder zusammen für einen Alkylenrest mit 2 bis 20 C-Atomen, welcher zusammen mit N-Z¹-N einen 5- bis 12-gliedrigen Ring bildet und gegebenenfalls mindestens eine Ether-Gruppe aufweist, steht;
- R⁷: entweder für einen Alkyl-, Cycloalkyl- oder Arylalkylrest mit 1 bis 15 C-Atomen steht; oder, für den Fall dass Z¹ für eine Carbonylgruppe steht, für ein Wasserstoffatom oder für einen Alkyl-, Cycloalkyl- oder Arylalkylrest mit 1 bis 15 C-Atomen steht;
- und R⁸: für ein Wasserstoffatom oder für einen Alkyl-, Cycloalkyl- oder Arylalkylrest mit 1 bis 8 C-Atomen steht;
- und n, X, E, R¹ und R²: die bereits genannten Bedeutungen aufweisen.

Die gestrichelten Linien in den Formeln stellen in diesem Dokument jeweils die Bindung zwischen einem Substituenten und dem zugehörigen Molekülrest dar.

Bevorzugt stehen R¹ und R² jeweils für einen Methylrest.

Bevorzugt steht R³ für ein Wasserstoffatom.

Bevorzugt steht R⁴ für einen Alkyl-, Cycloalkyl- oder Arylalkylrest mit 6 bis 20 C-Atomen.

Bevorzugt bilden R⁵ und R⁶ zusammen mit N-Z¹-N einen Piperazin- oder einen Imidazolidin-2-on- oder einen Hexahydropyrimidin-2-on-Rest, welcher gegebenenfalls substituiert ist.

Für den Fall, dass Z¹ für Carbonyl steht, stehen R⁵ und R⁶ weiterhin bevorzugt für einen Methylrest oder für ein Wasserstoffatom oder für einen Rest R¹³ der Formel (IX).

Bevorzugt steht Y³ für den Rest

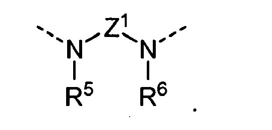

Bei den Aldiminen **A** der Formel (I) handelt es sich um geruchsarme oder geruchsfreie Substanzen.

Bei den Aldiminen **A2** der Formel (III), beziehungsweise den Aldiminen **A3** der Formel (III a), handelt es sich um geruchsfreie Substanzen.

Unter einer "geruchsarmen" Substanz wird eine Substanz verstanden, deren Geruch von menschlichen Individuen nur in geringem Masse wahrnehmbar, d.h. riechbar, ist, die also keinen intensiven Geruch aufweist wie beispielsweise Formaldehyd, Acetaldehyd, Isobutyraldehyd, oder Lösemittel wie Aceton, Methylethylketon oder Methylisobutylketon, und wobei dieser geringe Geruch von den meisten menschlichen Individuen nicht als unangenehm oder abstossend empfunden wird.

Unter einer "geruchsfreien" Substanz wird eine Substanz verstanden, die für die meisten menschlichen Individuen nicht riechbar ist, die also keinen wahrnehmbaren Geruch aufweist.

Ein Aldimin **A** der Formel (I) ist erhältlich aus der Umsetzung von mindestens einem Amin **B** der Formel (IV) mit mindestens einem Polyaldehyd **ALD** der Formel (V).

HX-E-NH₂ (IV)

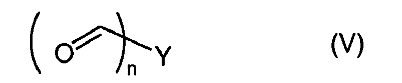

Hierbei weisen n, X, E und Y die bereits genannten Bedeutungen auf.

Die Umsetzung zwischen dem Amin **B** der Formel (IV) und dem Polyaldehyd **ALD** der Formel (V) erfolgt in einer Kondensationsreaktion unter Abspaltung von Wasser. Solche Kondensationsreaktionen sind bestens bekannt und beschrieben, beispielsweise in Houben-Weyl, "Methoden der organischen Chemie", Vol. XI/2, Seite 73ff. Die Aldehydgruppen des Polyaldehyds werden dabei in Bezug auf die primären Aminogruppen des Amins **B** stöchiometrisch oder in stöchiometrischem Überschuss eingesetzt. Üblicherweise werden solche Kondensationsreaktionen in Anwesenheit eines Lösemittels durchgeführt, mittels welchem das bei der Reaktion entstehende Wasser azeotrop entfernt wird. Zur Herstellung eines Aldimins **A** der Formel (I) wird jedoch ein Herstellverfahren ohne Verwendung von Lösemitteln bevorzugt, wobei das bei der Kondensation gebildete Wasser direkt mittels Anlegen von Vakuum aus der Reaktionsmischung entfernt wird. Durch die lösemittelfreie Herstellung erübrigt sich ein Abdestillieren des Lösemittels nach erfolgter Herstellung, was den Herstellungsprozess vereinfacht. Zudem ist das Aldimin so frei von Lösemittelrückständen, welche einen störenden Geruch verursachen könnten.

Als Amin **B** der Formel (IV) geeignet sind Verbindungen, welche sowohl eine primäre Aminogruppe als auch eine Reaktivgruppe in Form einer Hydroxyl-, Mercapto- oder sekundären Aminogruppe aufweisen. Insbesondere geeignet als Amin **B** sind die Folgenden:
- Hydroxyamine, wie insbesondere 2-Aminoethanol, 2-Amino-1-propanol, 1-Amino-2-propanol, 3-Amino-1-propanol, 4-Amino-1-butanol, 4-Amino-2-butanol, 2-Amino-2-methylpropanol, 5-Amino-1-pentanol, 6-Amino-1-hexanol, 7-Amino-1-heptanol, 8-Amino-1-octanol, 10-Amino-1-decanol, 12-Amino-1-dodecanol, 4-(2-Aminoethyl)-2-hydroxyethylbenzol, 3-Aminomethyl-3,5,5-trimethyl-cyclohexanol; eine primäre Aminogruppe tragende Derivate von Glykolen wie Diethylenglykol, Dipropylenglykol, Dibutylenglykol und höhere Oligomere und Polymere dieser Glykole, insbesondere 2-(2-Aminoethoxy)-ethanol, 2-(2-(2-Aminoethoxy)ethoxy)ethanol, α-(2-Hydroxy-methylethyl)-ω-(2-aminomethylethoxy)-poly(oxy(methyl-1,2-ethandiyl)); eine Hydroxylgruppe und eine primäre Aminogruppen tragende Derivate von polyalkoxylierten drei- oder höherwertigen Alkoholen; Produkte aus der einfachen Cyanoethylierung und anschliessender Hydrierung von Glykolen, insbesondere 3-(2-Hydroxyethoxy)-propylamin, 3-(2-(2-Hydroxyethoxy)-ethoxy)-propylamin und 3-(6-Hydroxyhexyloxy)-propylamin;
- aliphatische Mercaptoamine, wie insbesondere 2-Aminoethanthiol (Cysteamin), 3-Aminopropanthiol, 4-Amino-1-butanthiol, 6-Amino-1-hexanthiol, 8-Amino-1-octanthiol, 10-Amino-1-decanthiol und 12-Amino-1-dodecanthiol;
- Verbindungen mit je einer primären und einer sekundären aliphatischen Aminogruppe, wie insbesondere N-Methyl-1,2-ethandiamin, N-Ethyl-1,2-ethandiamin, N-Butyl-1,2-ethandiamin, N-Hexyl-1,2-ethandiamin, N-(2-Ethylhexyl)-1,2-ethandiamin, N-Cyclohexyl-1,2-ethandiamin, 4-Aminomethyl-piperidin, 3-(4-Aminobutyl)-piperidin, N-(2-Aminoethyl)piperazin, Diamine aus der Cyanoethylierung oder Cyanobutylierung und anschliessender Hydrierung von primären Monoaminen, wie insbesondere N-Methyl-1,3-propandiamin, N-Ethyl-1,3-propandiamin, N-Butyl-1,3-propandiamin, N-Hexyl-1,3-propandiamin, N-(2-Ethylhexyl)-1,3-propandiamin, N-Dodecyl-1,3-propandiamin, N-Cyclohexyl-1,3-propandiamin, 3-Methylamino-1-pentyl-amin, 3-Ethylamino-1-pentylamin, 3-Butylamino-1-pentylamin, 3-Hexyl-amino-1-pentylamin, 3-(2-Ethylhexyl)amino-1-pentylamin, 3-Dodecylamino-1-pentylamin, 3-Cyclohexylamino-1-pentylamin, und Fettdiamine wie N-Cocoalkyl-1,3-propandiamin, N-Oleyl-1,3-propandiamin, N-Soyaalkyl-1,3-propandiamin, N-Talgalkyl-1,3-propandiamin und N-(C₁₆₋₂₂-Alkyl)-1,3-propandiamin, wie sie beispielsweise unter dem Handelsnamen Duomeen^{®} von Akzo Nobel erhältlich sind; sowie die Produkte aus der Michael-artigen Addition von aliphatischen primären Diaminen mit Acrylnitril, Malein- oder Fumarsäurediestern, Citraconsäurediestern, Acryl- und Methacrylsäureestern, Acryl- und Methacrylsäureamiden und Itaconsäurediestern, umgesetzt im Molverhältnis 1:1.

Bevorzugt als Hydroxy- oder Mercaptoamine sind solche, in denen die primäre Aminogruppe von der Hydroxyl- oder Mercaptogruppe durch eine Kette von mindestens 5 Atomen, oder durch einen Ring, getrennt sind, insbesondere 5-Amino-1-pentanol, 6-Amino-1-hexanol und höhere Homologe davon, 4-(2-Aminoethyl)-2-hydroxyethylbenzol, 3-Aminomethyl-3,5,5-trimethyl-cyclohexanol, 2-(2-Aminoethoxy)-ethanol, Triethylenglykol-monoamin und höhere Homologe davon, 3-(2-Hydroxyethoxy)-propylamin, 3-(2-(2-Hydroxy-ethoxy)-ethoxy)-propylamin und 3-(6-Hydroxyhexyloxy)-propylamin.

Bevorzugt ist das Amin **B** ausgewählt aus der Gruppe bestehend aus 5-Amino-1-pentanol, 6-Amino-1-hexanol oder höheren Homologen davon, 4-(2-Aminoethyl)-2-hydroxyethylbenzol, 3-Aminomethyl-3,5,5-trimethyl-cyclohexanol, 2-(2-Aminoethoxy)-ethanol, Triethylenglykol-monoamin oder höheren Homologen davon, 3-(2-Hydroxyethoxy)-propylamin, 3-(2-(2-Hydroxyethoxy)-ethoxy)-propylamin, 3-(6-Hydroxyhexyloxy)-propylamin, N-Methyl-1,3-propandiamin, N-Ethyl-1,3-propandiamin, N-Butyl-1,3-propandiamin, N-(2-Ethylhexyl)-1,3-propandiamin, N-Dodecyl-1,3-propandiamin, N-Cyclohexyl-1,3-propandiamin, N-Cocoalkyl-1,3-propandiamin, N-Oleyl-1,3-propandiamin, N-Soyaalkyl-1,3-propandiamin, N-Talgalkyl-1,3-propandiamin und N-(C₁₆₋₂₂-Alkyl)-1,3-propandiamin.

Als Amin **B** besonders bevorzugt sind 5-Amino-1-pentanol und 2-(2-Aminoethoxy)-ethanol. Am meisten bevorzugt ist 2-(2-Aminoethoxy)-ethanol.

Als Polyaldehyd **ALD** der Formel (V) geeignet sind in einer Ausführungsform aliphatische oder cycloaliphatische Polyaldehyde wie insbesondere Korkaldehyd, Azelainaldehyd, Sebacinaldehyd, 1,12-Dodecandialdehyd, Hexahydrophthalaldehyd, Hexahydroisophthalaldehyd, Hexahydroterephthalaldehyd, Octahydro-4,7-methano-1H-indendicarbaidehyd, 3,6,9-Trioxaundecan-1,11-dial und höhere Homologe davon.

Als Polyaldehyd **ALD** der Formel (V) besonders geeignet sind aromatische Polyaldehyde **ALD1** der Formel (VI). 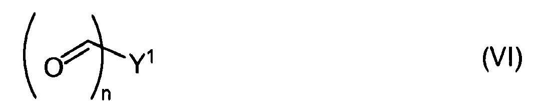 Hierbei weisen n und Y¹ die bereits genannten Bedeutungen auf.

Geeignete Polyaldehyde **ALD1** sind insbesondere aromatische Dialdehyde, wie insbesondere Phthalaldehyd, Isophthalaldehyd, Terephthalaldehyd, 9,10-Anthracendicarbaldehyd sowie 2,3-Naphthalindicarboxaldehyd und Isomere davon.

Ausgehend von Polyaldehyden **ALD1** sind die bevorzugten Aldimine **A1** der Formel (II) erhältlich.

Als Polyaldehyd **ALD** der Formel (V) besonders geeignet sind weiterhin Polyaldehyde **ALD2** der Formel (VII). 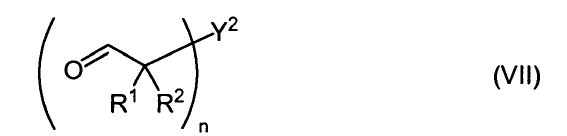 Hierbei weisen n, R¹, R² und Y² die bereits genannten Bedeutungen auf.

Ausgehend von Polyaldehyden **ALD2** sind insbesondere die bevorzugten Aldimine **A2** der Formel (III) erhältlich.

Als Polyaldehyd **ALD2** der Formel (VII) geeignet sind beispielsweise 2,2,6,6-Tetramethylheptan-1,7-dial, 2,2,7,7-Tetramethyloctan-1,8-dial sowie 1,3- und 1,4-Bis-(4,4-dimethyl-5-oxo-2-pentyl)-benzol.

Als Polyaldehyd **ALD2** der Formel (VII) besonders geeignet sind Polyaldehyde **ALD3** der Formel (VIII).

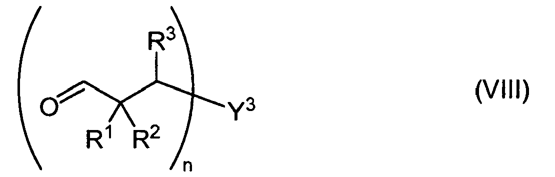

Hierbei weisen n, R¹, R², R³ und Y³ die bereits genannten Bedeutungen auf.

Als Polyaldehyd **ALD3** der Formel (VIII) geeignet ist in einer Ausführungsform ein Polyaldehyd **ALD3a** der Formel (VIII a).

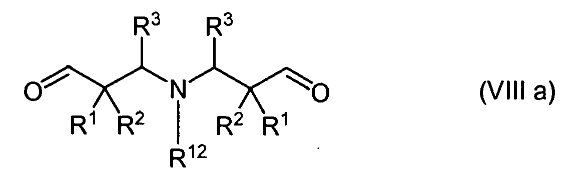

Hierbei steht R¹² entweder für R⁴ oder für einen Rest R¹³ der Formel (IX), und R¹, R², R³ und R⁴ weisen die bereits genannten Bedeutungen auf.

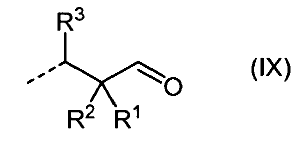

Ein Polyaldehyd **ALD3a** der Formel (VIII a) ist insbesondere erhältlich als Produkt einer Mannich-Reaktion oder einer der Mannich-Reaktion analogen α-Aminoalkylierung, wie sie aus der Fachliteratur bekannt ist; er kann deshalb auch als Mannich-Base bezeichnet werden. Ein Aldehyd **Y1** der Formel (X), ein Aldehyd **Y2** der Formel (XI) und eine Verbindung **C1** der Formel (XII a) werden dabei unter Wasserabspaltung zu einem Polyaldehyd **ALD3a** der Formel (VIII a) umgesetzt.

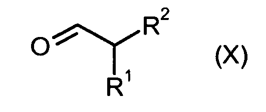

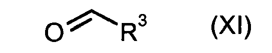

H₂N-R¹⁴ (XII a)

Hierbei steht R¹⁴ entweder für ein Wasserstoffatom oder für R⁴; und R¹, R², R³ und R⁴ weisen die bereits genannten Bedeutungen auf.

Diese Umsetzung kann entweder mit den freien Reagentien **Y1, Y2** und **C1** gemäss den Formeln (X), (XI) und (XII a) geführt werden, oder die Reagentien können teilweise oder vollständig in derivatisierter Form eingesetzt werden. In einer bevorzugten Ausführungsform wird die Umsetzung mit allen Reagentien in freier Form als Eintopfreaktion geführt und der Polyaldehyd **ALD3a** nach erfolgter Umsetzung durch Destillation gereinigt. Bevorzugt werden dabei keine organischen Lösemittel eingesetzt.

Als Aldehyd **Y1** der Formel (X) geeignet sind insbesondere die folgenden Aldehyde: Isobutyraldehyd, 2-Methylbutyraldehyd, 2-Ethylbutyraldehyd, 2-Methylvaleraldehyd, 2-Ethylcapronaldehyd, Cyclopentancarboxaldehyd, Cyclohexancarboxaldehyd, 1,2,3,6-Tetrahydrobenzaldehyd, 2-Methyl-3-phenylpropionaldehyd, 2-Phenylpropionaldehyd und Diphenylacetaldehyd. Bevorzugt ist Isobutyraldehyd.

Als Aldehyd **Y2** der Formel (XI) geeignet sind insbesondere die folgenden Aldehyde: Formaldehyd, Acetaldehyd, Propionaldehyd, Butyraldehyd, Isobutyraldehyd, Phenylacetaldehyd und Glyoxylsäureester, insbesondere Glyoxylsäureethylester. Bevorzugt ist Formaldehyd.

Als Verbindung **C1** der Formel (XII a) geeignet sind in einer ersten Ausführungsform primäre aliphatische Amine, insbesondere die Folgenden: Methylamin, Ethylamin, Propylamin, Isopropylamin, Butylamin, Isobutylamin, sek.-Butylamin, Hexylamin, Cyclohexylamin, Octylamin, 2-Ethyl-1-hexylamin, Benzylamin, 1- oder 2-Phenylethylamin, Decylamin, Dodecylamin, Tetradecylamin, Hexadecylamin, Octadecylamin, Eicosylamin, sowie von natürlichen Fettsäuregemischen abgeleitete Fettamine, wie zum Beispiel Cocoalkylamin, C₁₆-C₂₂-Alkylamin, Soyaalkylamin, Oleylamin und Talgalkylamin, erhältlich beispielsweise unter den Handelsnamen Armeen^{®} (von Akzo Nobel) oder Rofamin^{®} (von Ecogreen Oleochemicals).

Als Verbindung **C1** der Formel (XII a) geeignet ist in einer weiteren Ausführungsform Ammoniak.

Im Fall von Ammoniak als Verbindung **C1** werden pro mol Ammoniak je 3 mol der Aldehyde **Y1** und **Y2** umgesetzt, wobei ein Polyaldehyd **ALD3a** mit drei Aldehydgruppen gebildet wird, während im Fall eines primären aliphatischen Amins als Verbindung **C1** pro mol Amin je 2 mol der Aldehyde **Y1** und **Y2** umgesetzt werden und ein Polyaldehyd **ALD3a** mit zwei Aldehydgruppen gebildet wird.

Als Polyaldehyde **ALD3** der Formel (VIII) geeignet sind in einer weiteren Ausführungsform Polyaldehyde **ALD3b** der Formel (VIII b).

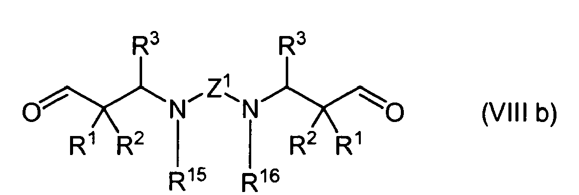

Hierbei stehen in einer Ausführungsform R¹⁵ für R⁵ und R¹⁶ für R⁶; in einer weiteren Ausführungsform stehen R¹⁵ für R⁷ und R¹⁶ für R¹³; in einer weiteren Ausführungsform stehen R¹⁵ und R¹⁶ je für einen Rest R¹³; und Z¹, R¹, R², R³, R⁵, R⁶, R⁷ und R¹³ weisen die bereits genannten Bedeutungen auf.

Ein Polyaldehyd **ALD3b** der Formel (VIII b) ist insbesondere auf dieselbe Weise erhältlich wie für einen Polyaldehyd **ALD3a** der Formel (VIII a) beschrieben, wobei aber anstelle einer Verbindung **C1** der Formel (XII a) eine Verbindung **C2** der Formel (XII b) eingesetzt wird.

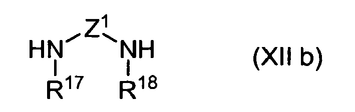

Hierbei stehen in einer Ausführungsform R¹⁷ für R⁵ und R¹⁸ für R⁶; in einer weiteren Ausführungsform stehen R¹⁷ für R⁷ und R¹⁸ für ein Wasserstoffatom; in einer weiteren Ausführungsform stehen R¹⁷ und R¹⁸ je für ein Wasserstoffatom; und Z¹, R⁵, R⁶ und R⁷ weisen die bereits erwähnten Bedeutungen auf.

Als Verbindung **C2** geeignet sind in einer Ausführungsform Polyamine mit zwei sekundären Aminogruppen, wie insbesondere Piperazin, 2,5- und 2,6-Dimethylpiperazin, 1,7-Dioxa-4,10-diazacyclododecan, N,N'-Dibutylethylen-diamin; N,N'-Di-tert.butyl-ethylendiamin, N,N'-Diethyl-1,6-hexandiamin, 1-(1-Methylethyl-amino)-3-(1-methylethyl-aminomethyl)-3,5,5-trimethylcyclohexan (Jefflink^{®} 754 von Huntsman), N4-Cyclohexyl-2-methyl-N2-(2-methylpropyl)-2,4-pentandiamin, N,N'-Dialkyl-1,3-xylylendiamin, Bis-(4-(N-alkylamino)-cyclohexyl)-methan, 4,4'-Trimethylen-dipiperidin und N-alkylierte Polyetheramine, beispielsweise Jeffamine^{®}- SD-231 (von Huntsman); sowie weiterhin disubstituierte Harnstoffe, beispielsweise N,N'-Dialkylharnstoffe wie N,N'-Dimethylharnstoff, N,N'-Diethylharnstoff, N,N'-Dibutylharnstoff, und insbesondere cyclische Harnstoffe wie Imidazolidin-2-on und Hexahydropyrimidin-2-on.

In dieser Ausführungsform werden pro mol der Verbindung **C2** je zwei mol der Aldehyde **Y1** und **Y2** eingesetzt, so dass Polyaldehyde **ALD3b** der Formel (VIII b) mit zwei Aldehydgruppen gebildet werden.

Als Verbindung **C2** geeignet sind in einer weiteren Ausführungsform Polyamine mit einer sekundären und einer primären Aminogruppen, wie insbesondere N-Methyl-1,2-ethandiamin, N-Ethyl-1,2-ethandiamin, N-Butyl-1,2-ethandiamin, N-Hexyl-1,2-ethandiamin, N-(2-Ethylhexyl)-1,2-ethandiamin, N-Cyclohexyl-1,2-ethandiamin, 4-Aminomethyl-piperidin, 3-(4-Aminobutyl)-piperidin, N-(2-Aminoethyl)piperazin, Diamine aus der Cyanoethylierung oder Cyanobutylierung und anschliessender Hydrierung von primären Monoaminen, wie insbesondere N-Methyl-1,3-propandiamin, N-Ethyl-1,3-propandiamin, N-Butyl-1,3-propandiamin, N-Hexyl-1,3-propandiamin, N-(2-Ethylhexyl)-1,3-propandiamin, N-Dodecyl-1,3-propandiamin, N-Cyclohexyl-1,3-propandiamin, 3-Methylamino-1-pentylamin, 3-Ethylamino-1-pentylamin, 3-Butylamino-1-pentylamin, 3-Hexylamino-1-pentylamin, 3-(2-Ethylhexyl)amino-1-pentylamin, 3-Dodecylamino-1-pentylamin, 3-Cyclohexylamino-1-pentylamin, und Fettdiamine wie N-Cocoalkyl-1,3-propandiamin, wie sie beispielsweise unter dem Handelsnamen Duomeen^{®} von Akzo Nobel erhältlich sind; sowie die Produkte aus der Michael-artigen Addition von aliphatischen primären Diaminen mit Acrylnitril, Malein- oder Fumarsäurediestern, Citraconsäurediestern, Acryl- und Methacrylsäureestern, Acryl- und Methacrylsäureamiden und Itaconsäurediestern, umgesetzt im Molverhältnis 1:1. In dieser Ausführungsform werden pro mol der Verbindung **C2** je drei mol der Aldehyde **Y1** und **Y2** eingesetzt, so dass Polyaldehyde **ALD3b** der Formel (VIII b) mit drei Aldehydgruppen gebildet werden.

Als Verbindung **C2** geeignet sind in einer weiteren Ausführungsform monosubstituierte Harnstoffe, beispielsweise N-Alkylharnstoffe wie N-Methylharnstoff, N-Ethylharnstoff oder N-Butylharnstoff. In dieser Ausführungsform werden pro mol der Verbindung **C2** zwei oder drei mol der Aldehyde **Y1** und **Y2** eingesetzt, so dass Polyaldehyde **ALD3b** der Formel (VIII b) mit zwei oder drei Aldehydgruppen gebildet werden.

Geeignet als Verbindung **C2** sind in einer weiteren Ausführungsform Polyamine mit zwei primären Aminogruppen, wie insbesondere Ethylendiamin, 1,2-Propandiamin, 1,3-Propandiamin, 2-Methyl-1,2-propandiamin, 2,2-Dimethyl-1,3-propandiamin, 1,3-Butandiamin, 1,4-Butandiamin, 1,3-Pentandiamin (DAMP), 1,5-Pentandiamin, 1,5-Diamino-2-methylpentan (MPMD), 2-Butyl-2-ethyl-1,5-pentandiamin (C11-Neodiamin), 1,6-Hexandiamin, 2,5-Dimethyl-1,6-hexandiamin, 2,2,4- und 2,4,4-Trimethylhexamethylendiamin (TMD), 1,7-Heptandiamin, 1,8-Octandiamin, 1,9-Nonandiamin, 1,10-Decandiamin, 1,11-Undecandiamin, 1,12-Dodecandiamin, 1,2-, 1,3- und 1,4-Diaminocyclohexan, Bis-(4-aminocyclohexyl)-methan (H₁₂-MDA), Bis-(4-amino-3-methylcyclohexyl)-methan, 1-Amino-3-aminomethyl-3,5,5-trimethylcyclohexan (= Isophorondiamin oder IPDA), 2- und 4-Methyl-1,3-diaminocyclohexan und Mischungen davon, 1,3- und 1,4-Bis-(aminomethyl)cyclohexan, 2,5(2,6)-Bis-(aminomethyl)-bicyclo[2.2.1]heptan (NBDA), 3(4),8(9)-Bis-(aminomethyl)-tri-cyclo[5.2.1.0^{2,6}]decan, 1,4-Diamino-2,2,6-trimethylcyclohexan (TMCDA), 1,8-Menthandiamin, 3,9-Bis-(3-aminopropyl)-2,4,8,10-tetraoxaspiro[5.5]undecan, 1,3- und 1,4-Xylylendiamin, sowie Ethergruppen-haltige aliphatische Diamine, insbesondere Bis-(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin sowie kurzkettige Polyoxyalkylen-Diamine, welche Produkte aus der Aminierung von Polyoxyalkylen-Diolen darstellen und beispielsweise erhältlich sind unter dem Namen Jeffamine^{®} (von Huntsman), unter dem Namen Polyetheramin (von BASF) oder unter dem Namen PC Amine^{®} (von Nitroil), insbesondere geeignete Polyoxyalkylen-Diamine sind Jeffamine^{®} D-230, Jeffamine^{®} XTJ-511, Jeffamine^{®} XTJ-568, Polyetheramin D 230 und PC Amine^{®} DA 250.

In dieser Ausführungsform werden pro mol der Verbindung **C2** je vier mol der Aldehyde **Y1** und **Y2** eingesetzt, so dass Polyaldehyde **ALD3b** der Formel (VIII b) mit vier Aldehydgruppen gebildet werden.

Als Verbindung **C2** geeignet ist in einer weiteren Ausführungsform Harnstoff. In dieser Ausführungsform werden pro mol Harnstoff zwei oder drei oder vier mol der Aldehyde **Y1** und **Y2** eingesetzt, so dass Polyaldehyde **ALD3b** der Formel (VIII b) mit zwei oder drei oder vier Aldehydgruppen gebildet werden.

Bevorzugt ist die Verbindung **C2** der Formel (XII b) ausgewählt aus der Gruppe bestehend aus Piperazin, 2,5- und 2,6-Dimethylpiperazin, N,N'-Dimethylharnstoff, Imidazolidin-2-on, Hexahydropyrimidin-2-on und Harnstoff.

Als Polyaldehyde **ALD3** der Formel (VIII) geeignet sind in einer weiteren Ausführungsform Polyaldehyde **ALD3c** der Formel (VIII c).

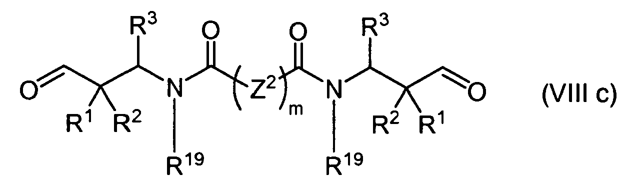

Hierbei stehen die Reste R¹⁹ jeweils unabhängig voneinander entweder für R⁸ oder für R¹³; und m, Z², R¹ R², R³, R⁸ und R¹³ weisen die bereits genannten Bedeutungen auf.

Ein Polyaldehyd **ALD3c** der Formel (VIII c) ist in einer Ausführungsform auf dieselbe Weise erhältlich wie für ein Polyaldehyd **ALD3a** der Formel (VIII a) beschrieben, wobei aber anstelle einer Verbindung **C1** der Formel (XII a) ein Diamid mit zwei oder drei oder vier Amid-Wasserstoffatomen eingesetzt wird.

Bevorzugt zur Herstellung eines Polyaldehyds **ALD3c** der Formel (VIII c) ist aber ein Herstellverfahren über ein Zwischenprodukt **ZW1** der Formel (XIII). 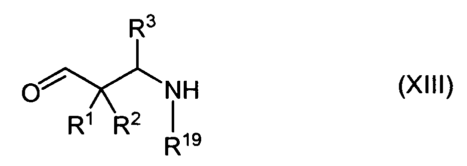 Hierbei weisen R¹, R², R³ und R¹⁹ die bereits erwähnten Bedeutungen auf.

Ein Zwischenprodukt **ZW1** der Formel (XIII) ist auf dieselbe Weise erhältlich wie für ein Polyaldehyd **ALD3a** der Formel (VIII a) beschrieben, wobei die Umsetzung zwischen der Verbindung **C1** der Formel (XII a) und den beiden Aldehyden **Y1** und **Y2** so erfolgt, dass die Amin-Wasserstoffatome gegenüber den Aldehydgruppen überstöchiometrisch eingesetzt werden. Anschliessend wird das Zwischenprodukt **ZW1** mit einer Dicarbonsäure, bevorzugt in Form eines Dicarbonsäuredichlorids oder -diesters, oder mit einem Tetracarbonsäuredianhydrid zum entsprechenden Diamid umgesetzt, wobei ein Polyaldehyd **ALD3c** der Formel (VIII c) gebildet wird. Geeignete Dicarbonsäuren sind insbesondere Oxalsäure, Malonsäure, Bernsteinsäure, Glutarsäure, Adipinsäure, Pimelinsäure, Korksäure, Azelainsäure, Sebacinsäure, 1,12-Dodecandisäure, Maleinsäure, Fumarsäure, Hexahydrophthalsäure, Hexahydroisophthalsäure, Hexahydroterephthalsäure, Methylhexahydrophthalsäure, 3,6,9-Trioxaundecandisäure und höhere Homologe davon, Phthalsäure, Isophthalsäure und Terephthalsäure, sowie Dichloride und Diester, insbesondere Methyl- und Ethylester, der genannten Dicarbonsäuren. Geeignete Tetracarbonsäuredianhydride sind insbesondere 1,2,4,5-Benzoltetracarbonsäuredianhydrid, 1,8,4,5-Naphthalintetracarbonsäuredianhydrid, 3,4,3',4'- und 2,3,3',4'-Biphenyltetracarbonsäuredianhydrid, 3,4,3',4'- und 2,3, 3',4'-Benzophenontetracarbonsäuredianhydrid, Oxydiphthalsäuredianhydrid und Hexahydro-4,8-ethano-1H,3H-benzo[1,2-c:4,5-c']difuran-1,3,5,7-tetron.

Als Polyaldehyde **ALD3** der Formel (VIII) geeignet sind in einer weiteren Ausführungsform Polyaldehyde **ALD3d** der Formel (VIII d).

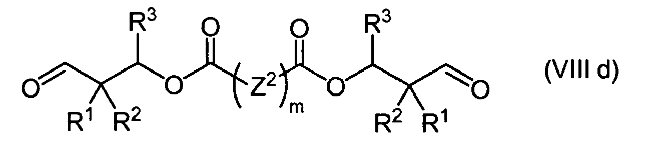

Hierbei weisen m, Z², R¹, R² und R³ die bereits genannten Bedeutungen auf.

Polyaldehyde **ALD3d** der Formel (VIII d) stellen formal Diester von 2,2-disubstituierten 3-Hydroxyaldehyden und Dicarbonsäuren dar. Die 2,2-disubstituierten 3-Hydroxyaldehyde stellen ihrerseits Produkte aus Aldol-Reaktionen, insbesondere gekreuzten Aldol-Reaktionen, zwischen primären oder sekundären aliphatischen Aldehyden, insbesondere Formaldehyd, und sekundären aliphatischen, sekundären cycloaliphatischen oder sekundären arylaliphatischen Aldehyden, wie beispielsweise Isobutyraldehyd, 2-Methylbutyraldehyd, 2-Ethylbutyraldehyd, 2-Methylvaleraldehyd, 2-Ethylcapronaldehyd, Cyclopentancarboxaldehyd, Cyclohexancarboxaldehyd, 1,2,3,6-Tetrahydrobenzaldehyd, 2-Methyl-3-phenylpropionaldehyd, 2-Phenylpropionaldehyd (Hydratropaldehyd) oder Diphenylacetaldehyd, dar. Geeignete 2,2-disubstituierte 3-Hydroxyaldehyde sind insbesondere 2,2-Dimethyl-3-hydroxypropanal, 2-Hydroxymethyl-2-methyl-butanal, 2-Hydroxymethyl-2-ethyl-butanal, 2-Hydroxymethyl-2-methyl-pentanal, 2-Hydroxymethyl-2-ethyl-hexanal, 1-Hydroxymethyl-cyclopentancarboxaldehyd, 1-Hydroxymethyl-cyclohexancarboxaldehyd 1-Hydroxymethyl-cyclohex-3-encarboxaldehyd, 2-Hydroxymethyl-2-methyl-3-phenyl-propanal, 3-Hydroxy-2-methyl-2-phenyl-propanal und 3-Hydroxy-2,2-diphenyl-propanal. Geeignete Dicarbonsäuren wurden bereits bei den Polyaldehyden **ALD3c** beschrieben. Die Herstellung von Polyaldehyden **ALD3d** kann insbesondere durch direkte Veresterung der genannten Hydroxyaldehyde mit den genannten Dicarbonsäuren erfolgen.

Als Polyaldehyde **ALD3** der Formel (VIII) geeignet sind in einer weiteren Ausführungsform Polyaldehyde **ALD3e** der Formel (VIII e).

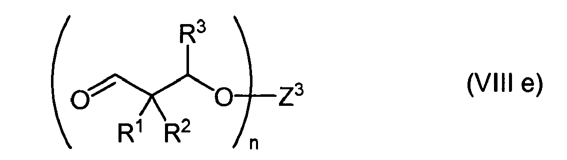

Hierbei weisen n, Z³, R¹, R² und R³ die bereits genannten Bedeutungen auf.

Polyaldehyde **ALD3e** der Formel (VIII e) stellen formal Ether von 2,2-disubstituierten 3-Hydroxyaldehyden und zwei- und mehrwertigen Alkoholen oder Phenolen dar. Geeignete 2,2-disubstituierte 3-Hydroxyaldehyde wurden bereits bei den Polyaldehyden **ALD3d** beschrieben

Geeignete zwei- und mehrwertige Alkohole oder Phenole sind beispielsweise 1,3-Propylenglykol, 1,2-Propylenglykol, 1,4-Butylenglykol, 1,6-Hexandiol, 1,8-Octandiol, 1,10-Decandiol, Neopentylglykol, Diethylenglykol, Triethylenglykol, die isomeren Dipropylenglykole und Tripropylenglykole, hydriertes Bisphenol A, 1,1,1-Trimethylolethan, 1,1,1-Trimethylolpropan, Glycerin, Pentaerythrit, niedrigmolekulare Alkoxylierungsprodukte der vorgenannten zwei- und mehrwertigen Alkohole, Resorcin, Bisphenol A, Bisphenol F, Bis-(4-hydroxy-3-methylphenyl)-methan, 4,4'-Dihydroxydiphenyl (DOD), 4,4'-Dihydroxybenzophenon, 1,5-Dihydroxy-naphthalin und Bis-(4-hydroxyphenyl)-ether. Die Herstellung von Polyaldehyden **ALD3e** kann beispielsweise durch gekreuzte Veretherung zwischen den genannten Hydroxyaldehyden und den genannten zwei- und mehrwertigen Alkoholen oder Phenolen erfolgen. Ein weiterer, beispielsweise in US 3,676,500 beschriebener Weg zur Herstellung von Polyaldehyden **ALD3e** führt über die thermisch induzierte Ringöffnung von geeigneten meta-Dioxanen.

Als Polyaldehyde **ALD3** der Formel (VIII) geeignet sind in einer weiteren Ausführungsform Polyaldehyde **ALD3f** der Formel (VIII f).

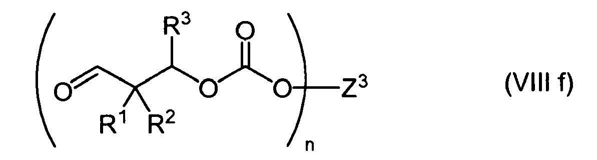

Hierbei weisen n, Z³, R¹, R² und R³ die bereits genannten Bedeutungen auf.

Polyaldehyde **ALD3f** der Formel (VIII f) stellen formal Dicarbonate von 2,2-disubstituierten 3-Hydroxyaldehyden und zwei- und mehrwertigen Alkoholen oder Phenolen dar. Geeignete 2,2-disubstituierte 3-Hydroxyaldehyde wurden bereits bei den Polyaldehyden **ALD3d** und geeignete zwei- und mehrwertige Alkohole oder Phenole bei den Polyaldehyden **ALD3e** beschrieben. Die Herstellung von Polyaldehyden **ALD3f** kann beispielsweise durch Umsetzung der genannten Hydroxyaldehyde mit Chlorameisensäureestern der genannten zwei- und mehrwertige Alkoholen oder Phenolen, wie zum Beispiel Ethylenbis-chloroformiat oder 1,6-Hexylenbis-chloroformiat, erfolgen.

Als Polyaldehyde **ALD3** der Formel (VIII) geeignet sind in einer weiteren Ausführungsform Polyaldehyde **ALD3g** der Formel (VIII g).

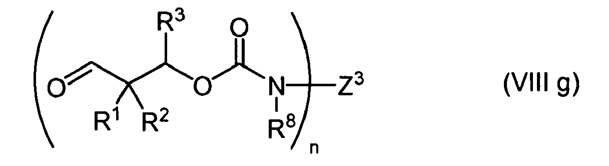

Hierbei weisen n, Z³, R¹, R², R³ und R⁸ die bereits genannten Bedeutungen auf.

Polyaldehyde **ALD3g** der Formel (VIII g) stellen formal Diurethane von 2,2-disubstituierten 3-Hydroxyaldehyden und Diaminen dar. Die Herstellung von Polyaldehyden **ALD3g** kann insbesondere durch Umsetzung der bereits bei den Polyaldehyden **ALD3d** beschrieben 2,2-disubstituierten 3-Hydroxyaldehyden mit Polyisocyanaten, gegebenenfalls gefolgt von einem Alkylierungsschritt, erfolgen. Für diese Umsetzung geeignete Polyisocyanate sind insbesondere die weiter hinten in diesem Dokument als Polyisocyanate **PI** beschriebenen Polyisocyanate.

Als Polyaldehyde **ALD3** der Formel (VIII) geeignet sind in einer weiteren Ausführungsform Polyaldehyde **ALD3h** der Formel (VIII h).

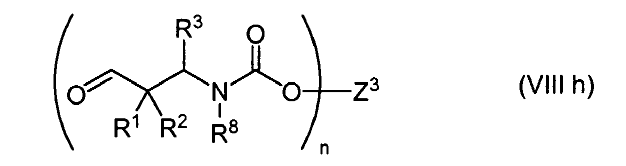

Hierbei weisen n, Z³, R¹, R², R³ und R⁸ die bereits genannten Bedeutungen auf.

Polyaldehyde **ALD3h** der Formel (VIII h) sind insbesondere erhältlich aus der Umsetzung der bereits beschriebenen Zwischenprodukte **ZW1** der Formel (XIII), bei welchen R¹⁹ für R⁸ stehen, mit Dicarbonaten, wie sie bereits zur Herstellung eines Polyaldehyds **ALD3f** der Formel (VIII f) erwähnt wurden, oder bevorzugt aus der Umsetzung mit Dichloriden solcher Dicarbonate, wie sie in Formel (XIV) dargestellt sind, wie zum Beispiel Ethylenbis-chloroformiat oder 1,6-Hexylenbis-chloroformiat.

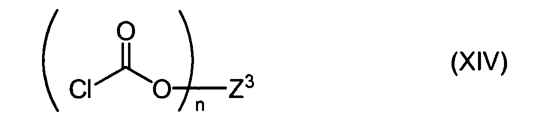

Hierbei weisen n und Z³ die bereits erwähnten Bedeutungen auf.

Als Polyaldehyde **ALD3** der Formel (VIII) geeignet sind in einer weiteren Ausführungsform Polyaldehyde **ALD3i** der Formel (VIII i).

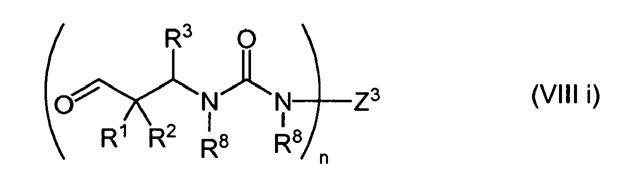

Hierbei weisen n, Z³, R¹, R², R³ und R⁸ die bereits genannten Bedeutungen auf.

Polyaldehyde **ALD3i** der Formel (VIII i) sind insbesondere erhältlich aus der Umsetzung der bereits beschriebenen Zwischenprodukte **ZW1** der Formel (XIII), bei welchen R¹⁹ für R⁸ steht, mit Polyisocyanaten, gegebenenfalls gefolgt von einem Alkylierungsschritt. Für diese Umsetzung geeignete Polyisocyanate sind die weiter hinten in diesem Dokument als Polyisocyanate **PI** beschriebenen Polyisocyanate.

Als Polyaldehyd **ALD** der Formel (V) bevorzugt sind Polyaldehyde **ALD1** der Formel (VI) und **ALD2** der Formel (VII). Besonders bevorzugt sind Polyaldehyde **ALD3** der Formel (VIII), davon insbesondere bevorzugt sind Polyaldehyde **ALD3b** der Formel (VIII b).

Die Polyaldehyde **ALD1** und die Polyaldehyde **ALD2** weisen in α-Stellung zum Carbonyl-C-Atom kein Wasserstoffatom auf. Dadurch können ihre Aldehydgruppen nicht zu Enolgruppen tautomerisieren und sind damit gegenüber Isocyanatgruppen unreaktiv.
Die Polyaldehyde **ALD2** sind geruchsfrei.

Eine alternative Herstellmethode für Aldimine **A3** der Formel (III a), bei welchen X für O oder S und Y³ für einen der Reste 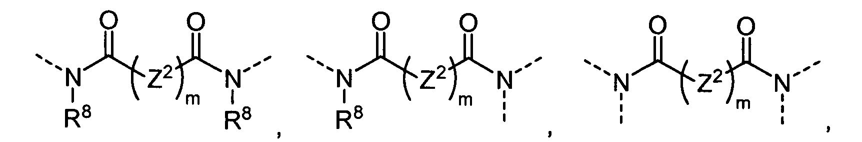 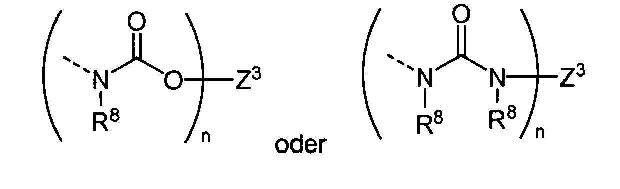 steht, führt über ein Zwischenprodukt **ZW2** der Formel (XV).

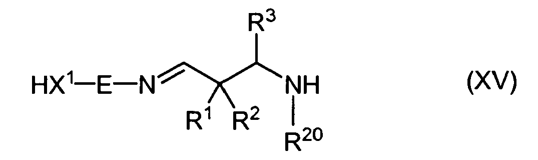

Hierbei steht X¹ für O oder S, R²⁰ für R⁸ oder für einen Rest R²¹ der Formel (XVI); und E, R¹, R², R³ und R⁸ weisen die bereits genannten Bedeutungen auf.

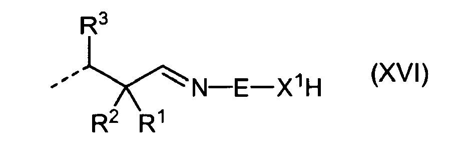

Das Zwischenprodukt **ZW2** wird dabei anstelle des Zwischenprodukts **ZW1** entweder mit mindestens einer Dicarbonsäure, bevorzugt in Form eines Dicarbonsäuredichlorids oder -diesters, oder mit mindestens einem Tetracarbonsäuredianhydrid, oder mit mindestens einem Dichlorid eines Dicarbonats der Formel (XIV), oder mit mindestens einem Polyisocyanat direkt zum entsprechenden Aldimin **A3** umgesetzt.
Das Zwischenprodukt **ZW2** ist insbesondere erhältlich durch die Umsetzung eines entsprechenden Zwischenproduktes **ZW1,** bei welchem X für O oder S steht, mit mindestens einem Amin **B** der Formel (IV), wobei das Amin **B** stöchiometrisch in Bezug auf die Aldehydgruppen des Zwischenproduktes **ZW1** eingesetzt wird.

Die Aldimine **A** der Formel (I) sind, wie erwähnt, geruchsarme oder insbesondere geruchsfreie Substanzen. Sie weisen n HX-Gruppen in Form von Hydroxyl-, sekundären Amino- oder Mercaptogruppen auf. Weiterhin weisen sie n Aldiminogruppen auf. Sie sind unter geeigneten Bedingungen lagerstabil. Bei Zutritt von Feuchtigkeit können ihre Aldiminogruppen über Zwischenstufen formal zu Aminogruppen hydrolysieren, wobei die entsprechenden, zur Herstellung der Aldimine verwendeten, Polyaldehyde **ALD** der Formel (V) freigesetzt werden. Da diese Hydrolysereaktion reversibel ist und das chemische Gleichgewicht deutlich auf der Aldiminseite liegt, ist davon auszugehen, dass in Abwesenheit von gegenüber Aminen reaktiven Verbindungen, insbesondere Isocyanaten, nur ein Teil der Aldiminogruppen teilweise oder ganz hydrolysieren. Die bei der Hydrolyse der Aldimine **A** freigesetzten Polyaldehyde **ALD** der Formel (V) sind ihrerseits geruchsarm oder geruchsfrei, bedingt durch ihr relativ hohes Molekulargewicht und ihre chemische Struktur.
Die bevorzugten Aldimine **A1** der Formel (II) und **A2** der Formel (III) weisen Aldiminogruppen auf, welche nicht zu Enaminogruppen tautomerisieren können. Für eine Verwendung zusammen mit Isocyanatgruppen stellen solche Aldiminogruppen besonders gut geschützte Aminogruppen dar.
Bei den besonders bevorzugten Aldiminen **A2** der Formel (III) handelt es sich um neue Verbindungen mit vorteilhaften Eigenschaften. Bei ihrer Hydrolyse werden Polyaldehyde **A2** der Formel (VII) freigesetzt, welche geruchsarm oder geruchsfrei sind und aufgrund ihrer geringen Flüchtigkeit weitgehend in der ausgehärteten Zusammensetzung verbleiben. Die am meisten bevorzugten Aldimine **A3** der Formel (III a) setzen bei ihrer Hydrolyse Polyaldehyde **ALD3** der Formel (VIII) frei. Diese weisen neben Aldehydgruppen zusätzliche funktionelle Gruppen auf, welche zur Ausbildung von Wasserstoffbrücken-Bindungen fähig sind.
Da die Polyaldehyde **ALD** zwei bis vier Aldehydgruppen aufweisen, haben sie trotz ihrer Grösse ein relativ niedriges Aldehyd-Equivalentgewicht. Dadurch bleibt die absolute Menge des freigesetzten Polyaldehyds **ALD** in der ausgehärteten Zusammensetzung in Gewichts-% relativ niedrig. In Verbindung mit der relativ hohen Viskosität bzw. dem festen Aggregatszustand vieler der Polyaldehyde **ALD** führt dies dazu, dass die Polyaldehyde **ALD** keine oder nur eine geringe Weichmacherwirkung auf die Zusammensetzung ausüben.

Die Zusammensetzung enthält weiterhin mindestens ein Polyisocyanat **P.**
Als Polyisocyanat **P** geeignet ist in einer Ausführungsform ein Polyisocyanat **PI** in der Form eines monomeren Di- oder Triisocyanates oder eines Oligomeren eines monomeren Diisocyanates oder eines Derivates eines monomeren Diisocyanates.

Als monomere Di- oder Triisocyanate geeignet sind beispielsweise die Folgenden: 1,4-Tetramethylendiisocyanat, 2-Methylpentamethylen-1,5-diisocyanat, 1,6-Hexamethylendiisocyanat (HDI), 2,2,4- und 2,4,4-Trimethyl-1,6-hexamethylendiisocyanat (TMDI), 1,10-Decamethylendiisocyanat, 1,12-Dodecamethylendiisocyanat, Lysin- und Lysinesterdiisocyanat, Cyclohexan-1,3-und -1,4-diisocyanat, 1-Methyl-2,4- und -2,6-diisocyanatocyclohexan und beliebige Gemische dieser Isomeren (HTDI oder H₆TDI), 1-Isocyanato-3,3,5-trimethyl-5-isocyanatomethyl-cyclohexan (=Isophorondiisocyanat oder IPDI), Perhydro-2,4'- und -4,4'-diphenylmethandiisocyanat (HMDI oder H₁₂MDI), 1,4-Diisocyanato-2,2,6-trimethylcyclohexan (TMCDI), 1,3- und 1,4-Bis-(isocyanatomethyl)-cyclohexan, m- und p-Xylylendiisocyanat (m- und p-XDI), m- und p-Tetramethyl-1,3- und -1,4-xylylendiisocyanat (m- und p-TMXDI), Bis-(1-Isocyanato-1-methylethyl)-naphthalin, Dimer- und Trimerfettsäureisocyanate wie 3,6-Bis-(9-isocyanatononyl)-4,5-di-(1-heptenyl)-cyclohexen (Dimeryldiisocyanat), α,α,α',α',α",α"-Hexamethyl-1,3,5-mesitylentriisocyanat, 2,4- und 2,6-Toluylendiisocyanat und beliebige Gemische dieser Isomeren (TDI), 4,4'-, 2,4'- und 2,2'-Diphenylmethandiisocyanat und beliebige Gemische dieser Isomeren (MDI), Gemische aus MDI und MDI-Homologen (polymeres MDI oder PMDI), 1,3- und 1,4-Phenylendiisocyanat, 2,3,5,6-Tetramethyl-1,4-diisocyanatobenzol, Naphthalin-1,5-diisocyanat (NDI), 3,3'-Dimethyl-4,4'-diisocyanatodiphenyl (TODI), Dianisidindiisocyanat (DADI), 1,3,5-Tris-(isocyanatomethyl)-benzol, Tris-(4-isocyanatophenyl)-methan und Tris-(4-isocyanatophenyl)-thiophosphat.
Als Polyisocyanat **PI** besonders geeignet sind Oligomere oder Derivate von monomeren Diisocyanaten, insbesondere von HDI, IPDI, TDI und MDI. Kommerziell erhältliche Typen sind insbesondere HDI-Biurete, beispielsweise als Desmodur^{®} N 100 und N 3200 (von Bayer), Tolonate^{®} HDB und HDB-LV (von Rhodia) und Duranate^{®} 24A-100 (von Asahi Kasei); HDI-Isocyanurate, beispielsweise als Desmodur^{®} N 3300, N 3600 und N 3790 BA (alle von Bayer), Tolonate^{®} HDT, HDT-LV und HDT-LV2 (von Rhodia), Duranate^{®} TPA-100 und THA-100 (von Asahi Kasei) und Coronate^{®} HX (von Nippon Polyurethane); HDI-Uretdione, beispielsweise als Desmodur^{®} N 3400 (von Bayer); HDI-Iminooxadiazindione, beispielsweise als Desmodur^{®} XP 2410 (von Bayer); HDI-Allophanate, beispielsweise als Desmodur^{®} VP LS 2102 (von Bayer); IPDI-Isocyanurate, beispielsweise in Lösung als Desmodur^{®} Z 4470 (von Bayer) oder in fester Form als Vestanat^{®} T1890/100 (von Degussa); TDI-Oligomere, beispielsweise als Desmodur^{®} IL (von Bayer); sowie gemischte Isocyanurate auf Basis TDI / HDI, zum Beispiel als Desmodur^{®} HL (von Bayer). Weiterhin besonders geeeignet sind bei Raumtemperatur flüssige Formen von MDI (sogenanntes "modifiziertes MDI"), welche Gemische von MDI mit MDI-Derivaten, wie beispielsweise MDI-Carbodiimiden bzw. MDI-Uretoniminen oder MDI-Urethanen darstellen, bekannt beispielsweise unter Handelsnamen wie Desmodur^{®} CD, Desmodur^{®} PF, Desmodur^{®} PC (alle von Bayer), sowie Gemische aus MDI und MDI-Homologen (polymeres MDI oder PMDI), erhältlich unter Handelsnamen wie Desmodur^{®} VL, Desmodur^{®} VL50, Desmodur^{®} VL R10, Desmodur^{®} VL R20 und Desmodur^{®} VKS 20F (alle von Bayer), Isonate^{®} M 309, Voranate^{®} M 229 und Voranate^{®} M 580 (alle von Dow) oder Lupranat^{®} M 10 R (von BASF).
Die vorgenannten oligomeren Polyisocyanate **PI** stellen in der Praxis üblicherweise Gemische von Substanzen mit unterschiedlichen Oligomerisationsgraden und/oder chemischen Strukturen dar. Vorzugsweise weisen sie eine mittlere NCO-Funktionalität von 2.1 bis 4.0 auf und enthalten insbesondere Isocyanurat-, Iminooxadiazindion-, Uretdion-, Urethan-, Biuret-, Allophanat-, Carbodiimid-, Uretonimin- oder Oxadiazintrion-Gruppen. Bevorzugt weisen diese Oligomere einen niedrigen Gehalt an monomeren Diisocyanaten auf.
Als Polyisocyanat **PI** bevorzugt sind bei Raumtemperatur flüssige Formen von MDI, sowie die Oligomeren von HDI, IPDI und TDI, insbesondere die Isocyanurate.

Als Polyisocyanat **P** geeignet ist in einer weiteren Ausführungsform ein Isocyanatgruppen aufweisendes Polyurethanpolymer **PUP.**

Ein geeignetes Isocyanatgruppen aufweisendes Polyurethanpolymer **PUP** ist erhältlich durch die Umsetzung von mindestens einem Polyol mit mindestens einem Polyisocyanat.

Als Polyole für die Herstellung eines Polyurethanpolymers **PUP** können beispielsweise die folgenden handelsüblichen Polyole oder Mischungen davon eingesetzt werden:
- Polyoxyalkylenpolyole, auch Polyetherpolyole oder Oligoetherole genannt, welche Polymerisationsprodukte von Ethylenoxid, 1,2-Propylenoxid, 1,2-oder 2,3-Butylenoxid, Oxetan, Tetrahydrofuran oder Mischungen davon sind, eventuell polymerisiert mit Hilfe eines Startermoleküls mit zwei oder mehreren aktiven Wasserstoffatomen wie beispielsweise Wasser, Ammoniak oder Verbindungen mit mehreren OH- oder NH-Gruppen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, Bisphenol A, hydriertes Bisphenol A, 1,1,1-Trimethylolethan, 1,1,1-Trimethylolpropan, Glycerin, Anilin, sowie Mischungen der vorgenannten Verbindungen. Eingesetzt werden können sowohl Polyoxyalkylenpolyole, die einen niedrigen Ungesättigtheitsgrad aufweisen (gemessen nach ASTM D-2849-69 und angegeben in Milliequivalent Ungesättigtheit pro Gramm Polyol (mEq/g)), hergestellt beispielsweise mit Hilfe von sogenannten Double Metal Cyanide Complex-Katalysatoren (DMC-Katalysatoren), als auch Polyoxyalkylenpolyole mit einem höheren Ungesättigtheitsgrad, hergestellt beispielsweise mit Hilfe von anionischen Katalysatoren wie NaOH, KOH, CsOH oder Alkalialkoholaten.
   Besonders geeignet sind Polyoxyalkylendiole oder Polyoxyalkylentriole, insbesondere Polyoxyethylen- und Polyoxypropylendi- und -triole.
   Speziell geeignet sind Polyoxyalkylendiole und -triole mit einem Ungesättigtheitsgrad tiefer als 0.02 mEq/g und mit einem Molekulargewicht im Bereich von 1'000 - 30'000 g/mol, sowie Polyoxypropylendiole und -triole mit einem Molekulargewicht von 400 - 8'000 g/mol.
   Ebenfalls besonders geeignet sind sogenannte Ethylenoxid-terminierte ("EO-endcapped", ethylene oxide-endcapped) Polyoxypropylenpolyole. Letztere sind spezielle Polyoxypropylenpolyoxyethylenpolyole, die beispielsweise dadurch erhalten werden, dass reine Polyoxypropylenpolyole, insbesondere Polyoxypropylendiole und -triole, nach Abschluss der Polypropoxylierungsreaktion mit Ethylenoxid weiter alkoxyliert werden und dadurch primäre Hydroxylgruppen aufweisen.
- Styrol-Acrylnitril- oder Acrylnitril-Methylmethacrylat-gepfropfte Polyetherpolyole.
- Polyesterpolyole, auch Oligoesterole genannt, hergestellt nach bekannten Verfahren, insbesondere der Polykondensation von Hydroxycarbonsäuren oder der Polykondensation von aliphatischen und/oder aromatischen Polycarbonsäuren mit zwei- oder mehrwertigen Alkoholen.
   Als Polyesterpolyole insbesondere geeignet sind solche, welche hergestellt sind aus zwei- bis dreiwertigen, insbesondere zweiwertigen, Alkoholen, wie beispielsweise Ethylenglykol, Diethylenglykol, Propylenglykol, Dipropylenglykol, Neopentylglykol, 1,4-Butandiol, 1,5-Pentandiol, 3-Methyl-1,5-hexandiol, 1,6-Hexandiol, 1,8-Octandiol, 1,10-Decandiol, 1,12-Dodecandiol, 1,12-Hydroxystearylalkohol, 1,4-Cyclohexandimethanol, Dimerfettsäurediol (Dimerdiol), Hydroxypivalinsäureneopentylglykolester, Glycerin, 1,1,1-Trimethylolpropan oder Mischungen der vorgenannten Alkohole, mit organischen Di- oder Tricarbonsäuren, insbesondere Dicarbonsäuren, oder deren Anhydride oder Ester, wie beispielsweise Bernsteinsäure, Glutarsäure, Adipinsäure, Trimethyladipinsäure, Korksäure, Azelainsäure, Sebacinsäure, Dodecandicarbonsäure, Maleinsäure, Fumarsäure, Dimerfettsäure, Phthalsäure, Phthalsäureanhydrid, Isophthalsäure, Terephthalsäure, Dimethylterephthalat, Hexahydrophthalsäure, Trimellithsäure und Trimellithsäureanhydrid, oder Mischungen der vorgenannten Säuren, sowie Polyesterpolyole aus Lactonen wie beispielsweise aus ε-Caprolacton und Startern wie den vorgenannten zwei- oder dreiwertigen Alkoholen.
   Besonders geeignete Polyesterpolyole sind Polyesterdiole.
- Polycarbonatpolyole, wie sie durch Umsetzung beispielsweise der oben genannten - zum Aufbau der Polyesterpolyole eingesetzten - Alkohole mit Dialkylcarbonaten, Diarylcarbonaten oder Phosgen zugänglich sind.
- Mindestens zwei Hydroxylgruppen tragende Blockcopolymere, welche mindestens zwei verschiedene Blöcke mit Polyether-, Polyester- und/oder Polycarbonatstruktur der oben beschriebenen Art aufweisen, insbesondere Polyetherpolyesterpolyole.
- Polyacrylat- und Polymethacrylatpolyole.
- Polyhydroxyfunktionelle Fette und Öle, beispielsweise natürliche Fette und Öle, inbesondere Ricinusöl; oder durch chemische Modifizierung von natürlichen Fetten und Ölen gewonnene - sogenannte oleochemische - Polyole, beispielsweise die durch Epoxidierung ungesättigter Öle und anschliessender Ringöffnung mit Carbonsäuren bzw. Alkoholen erhaltenen Epoxypolyester bzw. Epoxypolyether, oder durch Hydroformylierung und Hydrierung ungesättigter Öle erhaltene Polyole; oder aus natürlichen Fetten und Ölen durch Abbauprozesse wie Alkoholyse oder Ozonolyse und anschliessender chemischer Verknüpfung, beispielsweise durch Umesterung oder Dimerisierung, der so gewonnenen Abbauprodukte oder Derivaten davon erhaltene Polyole. Geeignete Abbauprodukte von natürlichen Fetten und Ölen sind insbesondere Fettsäuren und Fettalkohole sowie Fettsäureester, insbesondere die Methylester (FAME), welche beispielsweise durch Hydroformylierung und Hydrierung zu Hydroxyfettsäureestern derivatisiert werden können.
- Polykohlenwasserstoffpolyole, auch Oligohydrocarbonole genannt, wie beispielsweise polyhydroxyfunktionelle Polyolefine, Polyisobutylene, Polyisoprene; polyhydroxyfunktionelle Ethylen-Propylen-, Ethylen-Butylen- oder Ethylen-Propylen-Dien-Copolymere, wie sie beispielsweise von der Firma Kraton Polymers hergestellt werden; polyhydroxyfunktionelle Polymere von Dienen, insbesondere von 1,3-Butadien, welche insbesondere auch aus anionischer Polymerisation hergestellt sein können; polyhydroxyfunktionelle Copolymere aus Dienen wie 1,3-Butadien oder Diengemischen und Vinylmonomeren wie Styrol, Acrylonitril, Vinylchlorid, Vinylacetat, Vinylalkohol, Isobutylen und Isopren, beispielsweise polyhydroxyfunktionelle Acrylonitril/Butadien-Copolymere, wie sie beispielsweise aus Epoxiden oder Aminoalkoholen und carboxylterminierten Acrylonitril/Butadien-Copolymeren (beispielsweise kommerziell erhältlich unter dem Namen Hypro^{®} (früher Hycar^{®}) CTBN und CTBNX und ETBN von Nanoresins AG, Deutschland, bzw. Emerald Performance Materials LLC) hergestellt werden können; sowie hydrierte polyhydroxyfunktionelle Polymere oder Copolymere von Dienen.

Diese genannten Polyole weisen bevorzugt ein mittleres Molekulargewicht von 250 - 30'000 g/mol, insbesondere von 400 - 20'000 g/mol, und weisen bevorzugt eine mittlere OH-Funktionalität im Bereich von 1.6 bis 3 auf.
Unter "Molekulargewicht" versteht man bei Oligomeren oder Polymeren im vorliegenden Dokument stets das Molekulargewichtsmittel Mₙ.

Als Polyole bevorzugt sind Polyether-, Polyester-, Polycarbonat- und Polyacrylatpolyole, bevorzugt Di- und Triole. Besonders bevorzugt sind Polyetherpolyole, insbesondere Polyoxypropylen- und Polyoxypropylenpolyoxyethylenpolyole, sowie flüssige Polyesterpolyole und Polyetherpolyesterpolyole.

Zusätzlich zu diesen genannten Polyolen können kleine Mengen von niedrigmolekularen zwei- oder mehrwertigen Alkoholen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, hydriertes Bisphenol A, dimere Fettalkohole, 1,1,1-Trimethylolethan, 1,1,1-Trimethylolpropan, Glycerin, Pentaerythrit, Zuckeralkohole wie Xylit, Sorbit oder Mannit, Zucker wie Saccharose, andere höherwertige Alkohole, niedrigmolekulare Alkoxylierungsprodukte der vorgenannten zwei- und mehrwertigen Alkohole, sowie Mischungen der vorgenannten Alkohole bei der Herstellung des Polyurethanpolymers **PUP** mitverwendet werden. Ebenso können kleine Mengen an Polyolen mit einer mittleren OH-Funktionalität von mehr als 3 mitverwendet werden, beispielsweise Zuckerpolyole.

Als Polyisocyanate für die Herstellung eines Polyurethanpolymers **PUP** können aliphatische, cycloaliphatische oder aromatische Polyisocyanate, insbesondere Diisocyanate, verwendet werden, beispielsweise die monomeren Diisocyanate, welche bereits als geeignete Polyisocyanate **PI** erwähnt wurden, sowie Oligomere und Polymere dieser monomeren Diisocyanate, sowie beliebige Mischungen dieser Isocyanate. Bevorzugt sind monomere Diisocyanate, insbesondere MDI, TDI, HDI und IPDI.

Die Herstellung eines Polyurethanpolymers **PUP** erfolgt in bekannter Art und Weise direkt aus den Polyisocyanaten und den Polyolen, oder durch schrittweise Adduktionsverfahren, wie sie auch als Kettenverlängerungsreaktionen bekannt sind.
In einer bevorzugten Ausführungsform wird das Polyurethanpolymer **PUP** über eine Reaktion von mindestens einem Polyisocyanat und mindestens einem Polyol hergestellt, wobei die Isocyanatgruppen gegenüber den Hydroxylgruppen im stöchiometrischen Überschuss vorliegen. Vorteilhaft beträgt das Verhältnis zwischen Isocyanat- und Hydroxylgruppen 1.3 bis 10, insbesondere 1.5 bis 5.

Das Polyurethanpolymer **PUP** weist ein Molekulargewicht von vorzugsweise über 500 g/mol, insbesondere ein solches zwischen 1000 und 30'000 g/mol, auf.
Weiterhin weist das Polyurethanpolymer **PUP** bevorzugt eine mittlere NCO-Funktionalität im Bereich von 1.8 bis 3 auf.

Als Polyisocyanat **P** geeignet sind schliesslich auch Gemische enthaltend ein Polyurethanpolymer **PUP** und ein Polyisocyanat **PI,** insbesondere einerseits Gemische enthaltend ein MDI-basiertes Polyurethanpolymer **PUP** und monomeres und/oder polymeres MDI, sowie andererseits Gemische enthaltend ein IPDI-basiertes Polyurethanpolymer **PUP** und monomeres und/oder oligomeres IPDI.

Beim Kontakt des Aldimins **A** mit dem Polyisocyanat **P** reagieren die HX-Gruppen sowie gegebenenfalls vorhandene weitere gegenüber Isocyanatgruppen reaktive Gruppen mit den Isocyanatgruppen in einer Additionsreaktion, während die Aldiminogruppen in Abwesenheit von Wasser nicht mit den Isocyanatgruppen reagieren. Sobald die Aldiminogruppen jedoch mit Feuchtigkeit, insbesondere in Form von Luftfeuchtigkeit, in Kontakt kommen, beginnen sie formal zu Aldehydgruppen und primären Aminogruppen zu hydrolysieren, worauf letztere mit den Isocyanatgruppen reagieren und dabei Harnstoffgruppen bilden. Die Reaktion der Isocyanatgruppen mit den hydrolysierenden Aldiminogruppen muss dabei nicht notwendigerweise über freie primäre Aminogruppen erfolgen. Selbstverständlich sind auch Reaktionen mit Zwischenstufen der Hydrolysereaktion möglich. Beispielsweise ist es denkbar, dass eine hydrolysierende Aldiminogruppe in der Form eines Halbaminals direkt mit einer Isocyanatgruppe reagiert. Weiterhin können Isocyanatgruppen auch direkt mit Feuchtigkeit reagieren und bilden dabei Harnstoffgruppen. Je nach Stöchiometrie zwischen Isocyanatgruppen und der Summe der gegenüber Isocyanatgruppen reaktiven Gruppen härtet die Zusammensetzung als Ergebnis dieser Reaktionen aus, was auch als Vernetzung bezeichnet werden kann, oder es werden vergleichsweise niedrigmolekulare Additionsprodukte aus Aldiminen **A** und Polyisocyanaten **P** gebildet, welche für weiterführende Reaktionen verwendet werden können.
Für den Fall, dass bei dieser Reaktion ein deutlicher Überschuss an HX-Gruppen im Verhältnis zu den Isocyanatgruppen vorhanden ist, werden insbesondere Additionsprodukte **AV1** der Formel (XVII) gebildet, 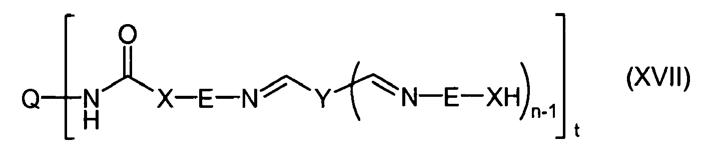 wobei
Q für den Rest eines Polyisocyanats **P** nach Entfernung von t Isocyanatgruppen steht,
t für 2 oder 3, insbesondere für 2, steht,
und n, E, X und Y die bereits erwähnten Bedeutungen aufweisen.
Die Additionsprodukte **AV1** können in der gleichen Weise verwendet werden wie die Aldimine **A**, insbesondere als Härter für Isocyanatgruppen aufweisende Zusammensetzungen.
Für den Fall, dass bei dieser Reaktion ein deutlicher Überschuss an Isocyanatgruppen im Verhältnis zu den HX-Gruppen vorhanden ist, werden unter Ausschluss von Wasser insbesondere Additionsprodukte **AV2** der Formel (XVIII) gebildet, 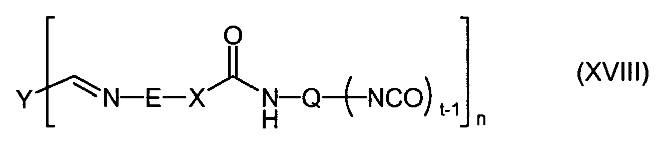 wobei n, t, E, Q, X und Y die bereits erwähnten Bedeutungen aufweisen.
Die Additionsprodukte **AV2** sind mit Feuchtigkeit härtbare Substanzen. Sie können insbesondere verwendet werden als Bestandteil von mit Feuchtigkeit härtbaren Zusammensetzungen.

Bevorzugt ist die beschriebene Zusammensetzung zweikomponentig.
Als "zweikomponentig" wird im vorliegenden Dokument eine Zusammensetzung bezeichnet, bei welcher die Bestandteile der Zusammensetzung in zwei verschiedenen Komponenten vorliegen, welche in voneinander getrennten Gebinden gelagert werden, und welche jeweils für sich lagerstabil sind. Erst kurz vor oder während der Applikation der Zusammensetzung werden die beiden Komponenten miteinander vermischt, worauf die vermischte Zusammensetzung aushärtet, wobei die Aushärtung unter Umständen erst durch die Einwirkung von Feuchtigkeit abläuft oder vervollständigt wird.

Bevorzugt ist die beschriebene Zusammensetzung eine härtbare zweikomponentige Zusammensetzung **ZS**, bestehend aus einer ersten Komponente **K1** enthaltend
a) mindestens ein Aldimin **A** der Formel (I), wie es vorgängig beschrieben wurde, und
b) mindestens eine Substanz **RS**, welche mindestens zwei gegenüber Isocyanatgruppen reaktive Gruppen aufweist, und/oder Wasser, und einer zweiten Komponente **K2** enthaltend
c) mindestens ein Polyisocyanat **P,** wie es vorgängig beschrieben wurde.

Als gegenüber Isocyanatgruppen reaktive Gruppen in der Substanz **RS** sind insbesondere Hydroxyl-, Mercapto- und primäre oder sekundäre Aminogruppen geeignet, wobei von den Aminogruppen die sekundären bevorzugt sind.

Als Substanz **RS,** welche mindestens zwei gegenüber Isocyanatgruppen reaktive Gruppen aufweist, geeignet sind insbesondere
- Polyole mit mindestens zwei Hydroxylgruppen, insbesondere die Polyole, welche vorgängig als zur Herstellung eines Polyurethanpolymers **PUP** geeignet beschrieben wurden, sowie niedrigmolekulare Alkohole, insbesondere die niedrigmolekularen Alkohole, welche vorgängig als zur Herstellung eines Polyurethanpolymers **PUP** geeignet beschrieben wurden;
- Polythiole mit mindestens zwei Mercaptogruppen, wie beispielsweise die unter dem Markennamen Thiokol^{®} bekannten flüssigen Mercapto-terminierten Polymere, beispielsweise die Typen LP-3, LP-33, LP-980, LP-23, LP-55, LP-56, LP-12, LP-31, LP-32 und LP-2 (von Morton Thiokol; beispielsweise erhältlich von SPI Supplies, USA, oder von Toray Fine Chemicals, Japan), sowie Polyester von Thiocarbonsäuren, beispielsweise Pentaerythritoltetramercaptoacetat, Trimethylolpropantrimercaptoacetat, Glykoldimercaptoacetat, Pentaerythritoltetra-(3-mercaptopropionat), Trimethylolpropantri-(3-mercaptopropionat) und Glykoldi-(3-mercaptopropionat);
- Thioalkohole mit mindestens einer Hydroxyl- und mindestens einer Mercaptogruppe, wie beispielsweise 2-Mercaptoethanol, 1-Mercapto-2-propanol, 3-Mercapto-1-propanol, 3-Mercapto-1,2-propandiol, 4-Mercapto-1-butanol, 6-Mercapto-1-hexanol;
- Polyamine mit mindestens zwei sekundären Aminogruppen, wie beispielsweise N,N'-Dibutylethylendiamin; N,N'-Di-tert.butyl-ethylendiamin, N,N'-Diethyl-1,6-hexandiamin, 1-(1-Methylethyl-amino)-3-(1-methylethyl-aminomethyl)-3,5,5-trimethylcyclohexan (Jefflink^{®} 754 von Huntsman), N4-Cyclohexyl-2-methyl-N2-(2-methylpropyl)-2,4-pentandiamin, N,N'-Dialkyl-1,3-xylylendiamin, Bis-(4-(N-alkylamino)-cyclohexyl)-methan, 4,4'-Trimethylen-dipiperidin sowie N-alkylierte Polyetheramine, beispielsweise die Jeffamine^{®}-Typen SD-231, SD-401, SD-404 und SD-2001 (von Huntsman);
- Aminoalkohole mit mindestens einer Hydroxyl- und mindestens einer sekundären Aminogruppe, wie beispielsweise 2-(Methylamino)ethanol, 2-(Ethylamino)ethanol, 2-(Butylamino)ethanol, 2-(Cyclohexylamino)ethanol, 3-Pyrrolidinol, 3- oder 4-Hydroxy-piperidin, 2-Piperidin-ethanol, 2-[2-(1-piperazyl)]ethanol, 2-[2-(1-Piperazyl)ethoxy]ethanol, N-Hydroxyethylanilin, Diethanolamin, Diisopropanolamin, 3-Methylamino-1,2-propandiol;
- Polyamine mit mindestens zwei primären Aminogruppen, insbesondere Polyoxyalkylen-Polyamine, welche Produkte aus der Aminierung von Polyoxyalkylen-Polyolen darstellen und beispielsweise unter dem Namen Jeffamine^{®} (von Huntsman), unter dem Namen Polyetheramin (von BASF) oder unter dem Namen PC Amine^{®} (von Nitroil) erhältlich sind; insbesondere geeignete Polyoxyalkylen-Diamine sind Jeffamine^{®} D-230, Jeffamine^{®} D-400, Jeffamine^{®} D-2000, Jeffamine^{®} D-4000, Jeffamine^{®} XTJ-568, Jeffamine^{®} XTJ-569, Jeffamine^{®} XTJ-523, Jeffamine^{®} XTJ-536, Jeffamine^{®} XTJ-542, Jeffamine^{®} XTJ-559, Jeffamine^{®} T-403, Jeffamine^{®} T-5000, Polyetheramin D 230, Polyetheramin D 400, Polyetheramin D 2000, Polyetheramin T403, Polyetheramin T5000; PC Amine^{®} DA 250, PC Amine^{®} DA 400, PC Amine^{®} DA 650, PC Amine^{®} DA 2000, PC Amine^{®} TA 403 und PC Amine^{®} TA 5000.

Als Substanz **RS** bevorzugt sind Polyole, welche ein mittleres Molekulargewicht von 250 bis 30'000 g/mol, insbesondere von 400 bis 20'000 g/mol, und eine mittlere OH-Funktionalität von 1.6 bis 3.0 aufweisen.

In einer bevorzugten Ausführungsform ist die härtbare zweikomponentige Zusammensetzung **ZS** eine Zusammensetzung **ZS1** bestehend aus einer ersten Komponente **K1'** enthaltend
a) mindestens ein Aldimin **A** der Formel (I),
b) mindestens ein Polyol **D** und gegebenenfalls Wasser,
   und einer zweiten Komponente **K2'** enthaltend
c) mindestens ein Polyisocyanat **P.**

Als Polyol **D** geeignet sind die Polyole, welche vorgängig als zur Herstellung eines Polyurethanpolymers **PUP** geeignet beschrieben wurden und welche ein mittleres Molekulargewicht von 250 bis 30'000 g/mol, insbesondere von 400 bis 20'000 g/mol, und eine mittlere OH-Funktionalität von 1.6 bis 3.0 aufweisen.
Als Polyol **D** besonders bevorzugt sind Polyole ausgewählt aus der Gruppe bestehend aus Polyetherpolyolen, Polyesterpolyolen, Polyetherpolyesterpolyolen, Polycarbonatpolyolen, Polyacrylatpolyolen, Polykohlenwasserstoffpolyolen und polyhydroxyfunktionellen Fetten und Ölen.
In der Zusammensetzung **ZS1** beträgt das Verhältnis zwischen der Anzahl der gegenüber Isocyanatgruppen reaktiven Gruppen und der Anzahl Isocyanatgruppen vorteilhaft 0.5 bis 1.1, bevorzugt 0.6 bis 1.0, besonders bevorzugt 0.65 bis 0.95, wobei die Aldiminogruppen zu den gegenüber Isocyanatgruppen reaktiven Gruppen gezählt werden und vorhandenes Wasser nicht zu den gegenüber Isocyanatgruppen reaktiven Gruppen gezählt wird.
In der Zusammensetzung **ZS1** beträgt das Verhältnis zwischen der Anzahl Aldiminogruppen und der Anzahl Isocyanatgruppen bevorzugt 0.05 bis 0.3. Das Verhältnis zwischen der Anzahl der Hydroxylgruppen und der Anzahl HX-Gruppen weist bevorzugt einen Wert von 1 bis 50, insbesondere von 2 bis 20, auf.

In einer weiteren bevorzugten Ausführungsform ist die härtbare zweikomponentige Zusammensetzung **ZS** eine Zusammensetzung **ZS2** bestehend aus einer ersten Komponente **K1"** enthaltend
a) mindestens ein Aldimin **A** der Formel (I),
b) Wasser und gegebenenfalls ein Tensid,
   und einer zweiten Komponente **K2"** enthaltend
c) mindestens ein Polyisocyanat **P,** insbesondere ein Polyurethanpolymer **PUP.**

Die Komponente **K2"** ist bevorzugt so formuliert, dass sie bei Kontakt mit Luftfeuchtigkeit auch allein zu einer ausgehärteten Zusammensetzung mit guten mechanischen Eigenschaften aushärtet; das heisst, sie ist dann auch allein verwendbar und stellt damit eine einkomponentige feuchtigkeitshärtende Zusammensetzung dar, beispielsweise einen einkomponentigen Polyurethan-Klebstoff oder -Dichtstoff, wie sie kommerziell breit erhältlich sind. Beispielsweise werden derartige Klebstoffe unter den Handelsnamen Sikaflex^{®} und SikaTack^{®} von Sika Schweiz AG angeboten.
Die Komponente **K1"** dient insbesondere zur Beschleunigung der Aushärtung der Komponente **K2",** gegebenenfalls auch zur Beeinflussung der Applikationseigenschaften und/oder der mechanischen Eigenschaften. Die Komponente **K1"** liegt bevorzugt als Emulsion vor.
In der Zusammensetzung **ZS2** beträgt das Verhältnis der gegenüber Isocyanatgruppen reaktiven Gruppen und der Anzahl Isocyanatgruppen vorteihaft 0.1 bis 0.7, insbesondere 0.1 bis 0.5, wobei die Aldiminogruppen zu den gegenüber Isocyanatgruppen reaktiven Gruppen gezählt werden und vorhandenes Wasser nicht zu den gegenüber Isocyanatgruppen reaktiven Gruppen gezählt wird.
In der Zusammensetzung **ZS2** liegt das Wasser bezogen auf die Isocyanatgruppen bevorzugt in einer ungefähr stöchiometrischen oder insbesondere deutlich überstöchiometrischen Menge vor.

Die härtbare zweikomponentige Zusammensetzung **ZS** enthält gegebenenfalls weitere Bestandteile, insbesondere in Polyurethanzusammensetzungen üblicherweise eingesetzte Hilfs- und Zusatzstoffe, beispielsweise die Folgenden:
- Weichmacher, insbesondere Carbonsäureester wie Phthalate, insbesondere Dioctylphthalat, Diisononylphthalat und Diisodecylphthalat, Adipate, insbesondere Dioctyladipat, Azelate und Sebacate, organische Phosphor- und Sulfonsäureester und Polybutene;
- nicht-reaktive thermoplastische Polymere, wie beispielsweise Homo- oder Copolymere von ungesättigten Monomeren, insbesondere aus der Gruppe umfassend Ethylen, Propylen, Butylen, Isobutylen, Isopren, Vinylacetat und Alkyl(meth)acrylate, insbesondere Polyethylene (PE), Polypropylene (PP), Polyisobutylene, Ethylenvinylacetat-Copolymere (EVA) und ataktische Poly-α-Olefine (APAO);
- Lösemittel;
- anorganische und organische Füllstoffe, zum Beispiel gemahlene oder gefällte Calciumcarbonate, welche gegebenenfalls mit Fettsäuren, insbesondere Stearaten, beschichtet sind, Baryt (BaSO₄, auch Schwerspat genannt), Quarzmehle, calcinierte Kaoline, Aluminiumoxide, Aluminiumhydroxide, Kieselsäuren, insbesondere hochdisperse Kieselsäuren aus Pyrolyseprozessen, Russe, insbesondere industriell hergestellte Russe (im Folgenden als "Russ" bezeichnet), PVC-Pulver und Hohlkugeln;
- Fasern, beispielsweise aus Polyethylen;
- Pigmente, beispielsweise Titandioxid oder Eisenoxide;
- Katalysatoren, welche die Hydrolyse von Aldiminen beschleunigen, insbesondere Säuren, beispielsweise organische Carbonsäuren wie Benzoesäure, Salicylsäure und 2-Nitrobenzoesäure, organische Carbonsäureanhydride wie Phthalsäureanhydrid, Hexahydrophthalsäureanhydrid und Hexahydromethylphthalsäureanhydrid, Silylester von organischen Carbonsäuren, organische Sulfonsäuren wie Methansulfonsäure, p-Toluolsulfonsäure und 4-Dodecylbenzolsulfonsäure, Sulfonsäureester, andere organische oder anorganische Säuren, oder Mischungen der vorgenannten Säuren und Säureester;
- Katalysatoren, welche die Reaktion der Isocyanatgruppen beschleunigen, beispielsweise Organozinnverbindungen wie Dibutylzinndiacetat, Dibutylzinndilaurat, Dibutylzinndichlorid, Dibutylzinndiacetylacetonat und Dioctylzinndilaurat, Bismutverbindungen wie Bismuttrioctoat und Bismut-tris(neodecanoat), und tertiäre Aminogruppen enthaltende Verbindungen wie 2,2'-Dimorpholinodiethylether und 1,4-Diazabicyclo[2.2.2]octan;
- Rheologie-Modifizierer wie beispielsweise Verdickungsmittel oder Thixotropiermittel, zum Beispiel Harnstoffverbindungen, Polyamidwachse, Bentonite oder pyrogene Kieselsäuren;
- blockierte Amine, beispielsweise in Form von Ketiminen, Oxazolidinen, Enaminen oder anderen Aldiminen;
- Trocknungsmittel, wie beispielsweise Molekularsiebe, Calciumoxid, hochreaktive Isocyanate wie p-Tosylisocyanat, Orthoameisensäureester, Alkoxysilane wie Tetraethoxysilan;
- Organoalkoxysilane, im Folgenden auch "Silane" genannt, wie beispielsweise Epoxysilane, (Meth)acrylsilane, Isocyanatosilane, Vinylsilane, Carbamatosilane, Alkylsilane, S-(Alkylcarbonyl)-mercaptosilane und Aldiminosilane, sowie oligomere Formen dieser Silane;
- Stabilisatoren gegen Wärme, Licht- und UV-Strahlung;
- flammhemmende Substanzen;
- oberflächenaktive Substanzen wie beispielsweise Netzmittel, Verlaufsmittel, Entlüftungsmittel oder Entschäumer;
- Biozide wie beispielsweise Algizide, Fungizide oder das Pilzwachstum hemmende Substanzen.

Es ist vorteilhaft, beim Einsatz solcher weiterer Bestandteile darauf zu achten, dass diese die Lagerstabilität der jeweiligen Komponente **K1** oder **K2** nicht stark beeinträchtigen. Falls solche Zusätze als Bestandteil der Komponente **K2** vorhanden sind, ist darauf zu achten, dass sie während der Lagerung die Vernetzung der Isocyanatgruppen nicht in signifikantem Ausmass auslösen. Insbesondere bedeutet dies, dass solchermassen verwendete Zusätze kein oder höchstens Spuren von Wasser enthalten sollten. Es kann sinnvoll sein, gewisse Zusätze vor dem Einmischen in die Komponente **K2** chemisch oder physikalisch zu trocknen.

Im Fall der Komponente **K1** sind neben diesen zusätzlich noch weitere Hilfs- und Zusatzstoffe möglich, welche zusammen mit freien Isocyanatgruppen nicht oder nur kurzzeitig lagerfähig sind. Beispielsweise können in der Komponente **K1** geringe Mengen von Polyaminen vorhanden sein, wie sie als Verbindungen **C2** vorgängig beschrieben wurden, um mit dem Vermischen der beiden Komponenten **K1** und **K2** unmittelbar ein strukturviskoses, weniger stark abfliessendes oder abrutschendes Material zu erhalten. Weiterhin können Katalysatoren vorhanden sein, wie insbesondere:
Verbindungen von Zink, Mangan, Eisen, Chrom, Cobalt, Kupfer, Nickel, Molybdän, Blei, Cadmium, Quecksilber, Antimon, Vanadium, Titan, Zirconium oder Kalium, wie Zink(II)-acetat, Zink(II)-2-ethylhexanoat, Zink(II)-laurat, Zink(II)-oleat, Zink(II)-naphthenat, Zink(II)-acetylacetonat, Zink(II)-salicylat, Mangan(II)-2-ethylhexanoat, Eisen(III)-2-ethylhexanoat, Eisen(III)-acetylacetonat, Chrom(III)-2-ethylhexanoat, Cobalt(II)-naphthenat, Cobalt(II)-2-ethyl-hexanoat, Kupfer(II)-2-ethylhexanoat, Nickel(II)-naphthenat, Phenylquecksilber-neodecanoat, Blei(II)-acetat, Blei(II)-2-ethylhexanoat, Blei(II)-neodecanoat, Blei(II)-acetylacetonat, Aluminiumlactat, Aluminiumoleat, Aluminium(III)-acetylacetonat, Diisopropoxytitan-bis-(ethylacetoacetat), Dibutoxytitan-bis-(ethylacetoacetat), Dibutoxytitan-bis-(acetylacetonat), Kalium-acetat, Kaliumoctoat; tertiäre Amine wie Triethylamin, Tributylamin, N-Ethyl-diisopropylamin, N,N,N',N'-Tetramethyl-ethylendiamin, Pentamethyl-diethylentriamin und höhere Homologe davon, N,N,N',N'-Tetramethyl-propylendiamin, Pentamethyl-dipropylentriamin und höhere Homologe davon, N,N,N',N'-Tetramethyl-1,3-butandiamin, N,N,N',N'-Tetramethyl-1,6-hexandiamin, Bis-(dimethylamino)-methan, N,N-Dimethylbenzylamin, N,N-Dimethylcyclohexylamin, N-Methyl-dicyclohexylamin, N,N-Dimethyl-hexadecylamin, Bis-(N,N-diethylaminoethyl)-adipat, N,N-Dimethyl-2-phenylethylamin, Tris-(3-dimethylaminopropyl)-amin, 1,4-Diazabicyclo[2.2.2]octan (DABCO), 1,8-Diazabicyclo[5.4.0]undec-7-en (DBU), 1,5-Diazabicyclo[4.3.0]non-5-en (DBN), N-Methylmorpholin, N-Ethylmorpholin, N-Cocomorpholin, N,N'-Dimethylpiperazin, N-Methyl-N'-dimethylaminoethyl-piperazin, Bis-(dimethylaminoethyl)-piperazin, 1,3,5-Tris-(dimethylaminopropyl)-hexahydrotriazin, Bis-(2-dimethylaminoethyl)-ether; stickstoffaromatische Verbindungen wie 4-Dimethylamino-pyridin, N-Methylimidazol, N-Vinylimidazol oder 1,2-Dimethylimidazol; Amidine und Guanidine wie 1,1,3,3-Tetramethylguanidin; tertiäre Amine enthaltend aktive Wasserstoffatome, wie Triethanolamin, Triisopropanolamin, N-Methyldiethanolamin, N,N-Dimethylethanolamin, 3-(Dimethylamino)-propyl-diisopropanolamin, Bis-(3-(dimethylamino)-propyl)-isopropanolamin, Bis-(3-dimethylaminopropyl)amin, 3-(Dimethylamino)-propylharnstoff, Mannich-Basen von Phenolen wie 2,4,6-Tris-(dimethylaminomethyl)-phenol und 2,4,6-Tris-(3-(dimethylamino)-propylaminomethyl)-phenol, Imidazole wie N-Hydroxypropylimidazol oder N-(3-Aminopropyl)-imidazol, sowie Alkoxylierungs- und Polyalkoxylierungsprodukte dieser Verbindungen, beispielsweise Dimethylaminoethoxyethanol; organische Ammoniumverbindungen wie Benzyltrimethylammoniumhydroxid oder alkoxylierte tertiäre Amine; sogenannte "delayed action"-Katalysatoren, welche Modifizierungen bekannter Metall- oder Aminkatalysatoren darstellen, wie Umsetzungsprodukte aus tertiären Aminen und Carbonsäuren oder Phenolen, beispielsweise aus 1,4-Diazabicyclo[2.2.2]octan oder DBU und Ameisensäure oder Essigsäure; sowie Kombinationen der genannten Verbindungen, insbesondere von Metallverbindungen und tertiären Aminen.

Bevorzugt enthält die Zusammensetzung **ZS** mindestens einen Katalysator in Form einer metallorganischen Verbindung und / oder eines tertiären Amins und / oder einer Säure, insbesondere einer organischen Carbon- oder Sulfonsäure.
Weiterhin bevorzugt enthält die Zusammensetzung **ZS** mindestens einen Füllstoff.

Die Herstellung der beiden Komponenten **K1** und **K2** erfolgt getrennt voneinander und, zumindest für die Komponente **K2,** unter Ausschluss von Feuchtigkeit. Die beiden Komponenten **K1** und **K2** sind getrennt voneinander lagerstabil, d.h. sie können jede in einer geeigneten Verpackung oder Anordnung, wie beispielsweise einem Fass, einem Beutel, einem Eimer, einer Kartusche oder einer Flasche, vor ihrer Anwendung während mehreren Monaten bis zu einem Jahr und länger aufbewahrt werden, ohne dass sie sich in ihren jeweiligen Eigenschaften in einem für ihren Gebrauch relevanten Ausmass verändern. Die Komponente **K1** ist auch dann lagerstabil, wenn sie eine gewisse Menge Wasser enthält, da die Hydrolyse der Aldimine **A** in Abwesenheit von Isocyanatgruppen nur ansatzweise abläuft.

Zur Verwendung der beschriebenen Zusammensetzung **ZS** werden die beiden Komponenten **K1** und **K2** miteinander vermischt. Dabei beginnen die HX-Gruppen des Aldimins **A**, die gegebenenfalls vorhandenen, gegenüber Isocyanatgruppen reaktiven Gruppen der Substanz **RS** sowie gegebenenfalls vorhandene weitere Reaktivgruppen enthaltend aktiven Wasserstoff mit den Isocyanatgruppen unmittelbar zu reagieren, wobei insbesondere Urethangruppen entstehen. Die Aldiminogruppen reagieren hingegen erst bei Kontakt mit Feuchtigkeit, indem die Aldiminogruppen, wie vorgängig bereits beschrieben, formal zu Aldehydgruppen und primären Aminogruppen hydrolysieren und letztere mit Isocyanatgruppen Harnstoffgruppen bilden. Diejenigen Isocyanatgruppen, die nicht mit einer gegenüber Isocyanatgruppen reaktiven Gruppe reagieren, können direkt mit Feuchtigkeit reagieren und bilden dabei Harnstoffgruppen. Als Ergebnis dieser Reaktionen härtet die Zusammensetzung **ZS** aus; dieser Prozess wird auch als Vernetzung bezeichnet.
Das Vermischen der beiden Komponenten **K1** und **K2** kann weitgehend homogen oder inhomogen erfolgen, wobei zum Vermischen insbesondere ein Statikmischer oder ein dynamischer Mischer eingesetzt wird und das Vermischen kontinuierlich oder batchweise erfolgen kann. Daraus resultiert eine vermischte oder teilvermischte Zusammensetzung **ZS.**
In einer bevorzugten Ausführungsform erfolgt das Vermischen der beiden Komponenten **K1** und **K2** im Wesentlichen homogen.
Dies kann insbesondere durch die Verwendung von dynamischen Mischern erreicht werden. Es ist auch möglich, durch die Verwendung von Statikmischern mit vielen Mischelementen zu einer im Wesentlichen homogenen Vermischung zu kommen.
Wie der Fachmann weiss, ist der Term "homogen vermischt", insbesondere im Kontext von pastösen Zusammensetzungen, nicht absolut zu verstehen. Der Term bedeutet in der üblichen Verwendung nämlich, dass von Auge keine Mischgrenzen mehr sichtbar sind, während dies beispielsweise unter dem Mikroskop durchaus noch möglich ist. Aufgrund von experimentellen Erkenntnissen kann davon ausgegangen werden, dass bei der Verwendung von Statikmischern, beispielsweise des Typs Sulzer Quadro^{®} (erhältlich von der Firma Sulzer Chemtech), mit 18 oder mehr Statikmischer-Elementen die Vermischung von zwei pastösen Komponenten im Wesentlichen homogen erfolgt.

Die vermischte Zusammensetzung **ZS** wird anschliessend auf ein Substrat appliziert, gegebenenfalls mittels eines geeigneten Applikationshilfsmittels. Dabei muss darauf geachtet werden, dass zwischen dem Vermischen der Komponenten und der Applikation nicht zuviel Zeit vergeht, da durch ein zu starkes Vorreagieren der Bestandteile der vermischten Zusammensetzung vor der Applikation die Funktion der ausgehärteten Zusammensetzung gestört sein kann, beispielsweise indem die Haftung zum Substrat nur ungenügend oder verzögert aufgebaut wird. Die maximale Zeitspanne, innerhalb welcher die vermischte Zusammensetzung appliziert sein sollte, wird als "Offenzeit" bezeichnet.

Mit dem Vermischen der Komponenten **K1** und **K2** beginnt die vermischte Zusammensetzung **ZS** über die bereits beschriebenen Reaktionen auszuhärten. Die zur Hydrolyse der Aldiminogruppen notwendige Feuchtigkeit dringt bevorzugt in Form von Luftfeuchtigkeit in die vermischte Zusammensetzung ein. In diesem Fall erfolgt die Reaktion der Aldiminogruppen verzögert, und zwar von aussen nach innen, parallel zum Eindringen der Luftfeuchtigkeit in die Zusammensetzung. Die Feuchtigkeit kann aber auch schon zu Beginn teilweise oder vollständig in der Zusammensetzung vorhanden sein, insbesondere indem sie Bestandteil der Komponente **K1** war. In diesem Fall reagieren die Aldiminogruppen etwas schneller mit vorhandenen Isocyanatgruppen, aber trotzdem deutlich langsamer als entsprechende freie Amine. Als Ergebnis dieser Reaktionen vernetzt die vermischte Zusammensetzung und härtet schliesslich zu einem festen Material aus.

Die Aushärtung erfolgt im Allgemeinen ohne die Bildung von Blasen, auch bei hoher Aushärtungsgeschwindigkeit. Die Aushärtungsgeschwindigkeit lässt sich über die Art und Menge eines oder mehrerer gegebenenfalls vorhandener Katalysatoren, über die bei der Aushärtung herrschende Temperatur sowie über die der Zusammensetzung zur Verfügung stehende Feuchtigkeit, insbesondere die Luftfeuchtigkeit, beeinflussen.

Die härtbare zweikomponentige Zusammensetzung **ZS**, wie sie vorgängig beschrieben wurde, weist eine Reihe von Vorteilen auf. Die Umsetzung der HX-Gruppen mit Isocyanatgruppen ist eine gut kontrollierbare und damit auf eine gut handhabbare Offenzeit der Zusammensetzung einstellbare Reaktion, insbesondere wenn die HX-Gruppen in Form der bevorzugten Hydroxylgruppen vorliegen. Bei Kontakt mit Feuchtigkeit reagieren die vorhandenen Aldiminogruppen dann aber sehr schnell mit Isocyanatgruppen. Dadurch baut die Zusammensetzung sehr schnell Festigkeit auf, deutlich schneller als eine vergleichbare Zusammensetzung, welche nur über ein Polyol aushärtet. Weiterhin wird durch die Hydrolyse der Aldiminogruppen die direkte Reaktion von Isocyanatgruppen mit Feuchtigkeit solange unterdrückt, bis die Aldiminogruppen mehrheitlich umgesetzt sind. Zu diesem Zeitpunkt weist die Zusammensetzung aber bereits eine so hohe Festigkeit auf, dass aus der direkten Reaktion von Isocyanatgruppen mit Feuchtigkeit freigesetztes CO₂ kaum mehr zur Bildung von Blasen führt. Bei der Hydrolyse der Aldiminogruppen der Aldimine **A** werden Polyaldehyde **ALD** mit 2 bis 4 Aldehydgruppen freigesetzt. Diese weisen nur eine relativ geringe Flüchtigkeit auf und verbleiben weitgehend in der ausgehärteten Zusammensetzung, wo sie nur einen geringen oder gar keinen Geruch verursachen. Überraschenderweise ist der freigesetzte Polyaldehyd **ALD** in der ausgehärteten Zusammensetzung sehr gut verträglich und hat nur eine sehr geringe weichmachende Wirkung auf die Zusammensetzung. Dies ist insbesondere dann von Vorteil, wenn die Zusammensetzung eine hohe Zugfestigkeit und ein hohes Elastizitätsmodul aufweisen soll. Besonders gut verträglich in der ausgehärteten Zusammensetzung sind die Polyaldehyde **ALD2,** und insbesondere die Polyaldehyde **ALD3.** Begünstigt werden diese vorteilhaften Eigenschaften der Aldimine **A** vermutlich von der speziellen Struktur der Aldimine **A.** Dadurch, dass der bei der Hydrolyse freigesetzte Polyaldehyd **ALD** 2 bis 4 Aldehydgruppen aufweist, ist sein Aldehyd-Equivalentgewicht relativ klein, und es wird insgesamt nur eine relativ geringe Aldehyd-Menge bezogen auf die gesamte Zusammensetzung frei. Weiterhin weisen die Polyaldehyde **ALD3,** welche aus den am meisten bevorzugten Aldiminen **A3** der Formel (III a) freigesetzt werden, funktionelle Gruppen auf, welche zur Ausbildung von Wasserstoffbrücken-Bindungen fähig sind. Möglicherweise sind die Polyaldehyde **ALD3** deshalb besonders gut verträglich und kaum weichmachend in der ausgehärteten Zusammensetzung. Zusammensetzungen mit Aldiminen **A3** der Formel (III a) zeigen kaum Schrumpf und Migrationseffekte.

Aldimine **A**, bei denen der Index n in Formel (I) für 3 oder 4 steht, zeigen zudem einen weiteren vorteilhaften Effekt. Dadurch, dass das Aldimin **A** bezüglich der HX-Gruppen eine Funktionalität von grösser als 2 aufweist, entsteht bei der Aushärtung sehr schnell eine hohe Vernetzung und somit ein besonders rascher Aufbau von Festigkeit. Bei der Hydrolyse der Aldiminogruppen reduziert sich die Funktionalität des Aldimin-Härters - jetzt das Amin **B** - dann aber auf 2, und es wird schliesslich eine ausgehärtete Zusammensetzung mit der gewünschten, gut einstellbaren Endfestigkeit und Elastizität gebildet. So sind Zusammensetzungen mit einer besonders hohen und schnellen Frühfestigkeit und einer guten Elastizität erhältlich.

Die vorgängig beschriebenen Zusammensetzungen weisen eine Reihe von Vorteilen auf. Sie verfügen über eine genügend lange Offenzeit, um eine gute Handhabung zu ermöglichen. Die Aushärtung verläuft dann aber sehr schnell und ohne dass es dabei zu einer starken Geruchsbelästigung kommt. Im ausgehärteten Zustand weisen die Zusammensetzungen hohe Zugfestigkeiten und Elastizitätsmodule und kaum Schrumpf und Migrationseffekte auf.
Falls die Zusammensetzungen nur die bevorzugten geruchsfreien Aldimine **A2** oder **A3** und auch sonst keine Inhaltsstoffe mit einem starken Geruch enthalten, entsteht vor, während und nach der Aushärtung kein störender Geruch, was für viele Anwendungen, insbesondere in Innenräumen, ein grosser Vorteil, wenn nicht sogar eine Voraussetzung ist.

Die vorgängig beschriebenen Zusammensetzungen, insbesondere die Zusammensetzung **ZS**, lassen sich insbesondere als Klebstoff, Dichtstoff, Vergussmasse, Beschichtung, Bodenbelag, Anstrich, Lack oder Primer verwenden.
Insbesondere geeignet sind sie für Anwendungen, bei welchen bei einer nicht zu kurzen Offenzeit eine schnelle Aushärtung und eine hohe Festigkeit erreicht werden sollen. Dies sind insbesondere Anwendungen als Klebstoff, Vergussmasse, Beschichtung, Bodenbelag, Anstrich und Lack.

Ein weiterer Aspekt der vorliegenden Erfindung betrifft ein Verfahren zum Verkleben von einem Substrat **S1** mit einem Substrat **S2,** welches die Schritte umfasst:
i) Applikation einer vorgängig beschriebenen Zusammensetzung auf ein Substrat **S1;**
ii) Kontaktieren der applizierten Zusammensetzung mit einem Substrat **S2** innerhalb der Offenzeit der Zusammensetzung;
   oder
i') Applikation einer vorgängig beschriebenen Zusammensetzung auf ein Substrat **S1** und auf ein Substrat **S2;**
ii') Kontaktieren der applizierten Zusammensetzung miteinander innerhalb der Offenzeit der Zusammensetzung;
wobei das Substrat **S2** aus dem gleichen oder einem unterschiedlichen Material wie das Substrat **S1** besteht.

Ein weiterer Aspekt der vorliegenden Erfindung betrifft ein Verfahren zum Abdichten. Dieses umfasst den Schritt:
i") Applikation einer vorgängig beschriebenen Zusammensetzung zwischen ein Substrat **S1** und ein Substrat **S2,** so dass die Zusammensetzung mit dem Substrat **S1** und dem Substrat **S2** in Kontakt steht;
wobei das Substrat **S2** aus dem gleichen oder einem unterschiedlichen Material wie das Substrat **S1** besteht.
Üblicherweise wird der Dichtstoff in eine sogenannte Fuge eingepresst.

Ein weiterer Aspekt der vorliegenden Erfindung betrifft ein Verfahren zum Beschichten eines Substrates **S1**. Dieses umfasst den Schritt:
i"') Applikation einer vorgängig beschriebenen Zusammensetzung auf ein Substrat **S1** innerhalb der Offenzeit der Zusammensetzung.

In diesen Verfahren sind geeignete Substrate **S1** und/oder **S2** insbesondere
- Glas, Glaskeramik, Beton, Mörtel, Backstein, Ziegel, Gips und Natursteine wie Granit oder Marmor;
- Metalle oder Legierungen wie Aluminium, Stahl, Eisen, Buntmetalle, verzinkte Metalle;
- Leder, Textilien, Papier, Holz, mit Harz gebundene Holzwerkstoffe, Harz-Textil-Verbundwerkstoffe und weitere sogenannte Polymer-Composites;
- Kunststoffe wie Polyvinylchlorid (Hart- und Weich-PVC), AcrylonitrilButadien-Styrol-Copolymere (ABS), SMC (Sheet Moulding Compounds), Polycarbonat (PC), Polyamid (PA), Polyester, Poly(methylmethacrylat) (PMMA), Polyester, Epoxidharze, Polyurethane (PUR), Polyoxymethylen (POM), Polyolefine (PO), Polyethylen (PE) oder Polypropylen (PP), Ethylen/Propylen-Copolymere (EPM) und Ethylen/Propylen/Dien-Terpolymere (EPDM), wobei die Kunststoffe bevorzugt mittels Plasma, Corona oder Flammen oberflächenbehandelt sein können;
- beschichtete Substrate wie pulverbeschichtete Metalle oder Legierungen; sowie Farben und Lacke.

Die Substrate können bei Bedarf vor dem Applizieren der Zusammensetzung vorbehandelt werden. Derartige Vorbehandlungen umfassen insbesondere physikalische und/oder chemische Reinigungsverfahren, beispielsweise Schleifen, Sandstrahlen, Bürsten oder dergleichen, oder Behandeln mit Reinigern oder Lösemitteln oder das Aufbringen eines Haftvermittlers, einer Haftvermittlerlösung oder eines Primers.
Die Applikation der vermischten Zusammensetzung kann in einem breiten Temperaturspektrum erfolgen. Beispielsweise kann die Zusammensetzung bei Raumtemperatur appliziert werden. Die Zusammensetzung kann aber auch bei tieferen wie auch bei höheren Temperaturen appliziert werden.
Aus diesen beschriebenen Verfahren zum Verkleben, Abdichten bzw. Beschichten - beziehungsweise aus der Verwendung einer der beschriebenen Zusammensetzungen als Klebstoff, Dichtstoff, Vergussmasse, Beschichtung, Bodenbelag, Anstrich, Lack oder Primer - entsteht ein Artikel.
Dieser Artikel ist insbesondere ein Bauwerk, insbesondere ein Bauwerk des Hoch- oder Tiefbaus, oder ein industrielles Gut oder ein Konsumgut, insbesondere ein Fenster, eine Haushaltmaschine, oder ein Transportmittel, insbesondere ein Fahrzeug zu Wasser oder zu Lande, bevorzugt ein Automobil, ein Bus, ein Lastkraftwagen, ein Zug oder ein Schiff, oder ein Anbauteil eines Transportmittels, oder ein Artikel der Möbel-, Textil- oder Verpackungsindustrie.

### Beispiele

### 1. Beschreibung der Messmethoden

**Infrarotspektren** wurden auf einem FT-IR Gerät 1600 von Perkin-Elmer gemessen (horizontale ATR-Messeinheit mit ZnSe-Kristall). Flüssige Proben wurden unverdünnt als Filme aufgetragen, feste Proben wurden in CH₂Cl₂ gelöst. Die Absorptionsbanden sind in Wellenzahlen (cm⁻¹) angegeben (Messfenster: 4000-650 cm⁻¹).
**¹H-NMR-Spektren** wurden auf einem Spektrometer des Typs Bruker DPX-300 bei 300.13 MHz gemessen; die chemischen Verschiebungen δ sind angegeben in ppm relativ zu Tetramethylsilan (TMS), Kopplungskonstanten *J* sind angegeben in Hz. Echte und Pseudo-Kopplungsmuster wurden nicht unterschieden.
Die Viskosität wurde auf einem thermostatisierten Kegel-Platten-Viskosimeter Physica UM (Kegeldurchmesser 20 mm, Kegelwinkel 1 °, Kegelspitze-Platten-Abstand 0.05 mm, Schergeschwindigkeit 10 bis 1000 s⁻¹) gemessen.
Der **Amingehalt,** das heisst der totale Gehalt an Aldiminogruppen und freien Aminogruppen in den hergestellten Verbindungen, wurde titrimetrisch bestimmt (mit 0.1N HClO₄ in Eisessig, gegen Kristallviolett) und ist stets angegeben in mmol N/g.

### 2. Herstellung von Polyaldehyden

### 1,3-Bis-(2,2-dimethyl-3-oxopropyl)-imidazolidin-2-on

In einem Rundkolben mit aufgesetztem Rückflusskühler wurden unter Stickstoffatmosphäre 21.5 g (0.25 mol) 2-Imidazolidinon, 41.7 g (0.50 mol) 36%-iger wässriger Formaldehyd und 37.2g (0.52 mol) Isobutyraldehyd vorgelegt unter gutem Rühren mit 5.0g conc. Salzsäure versetzt, wobei die Mischung heftig aufkochte. Nachdem sich das Kochen beruhigt hatte, wurde die Mischung im Ölbad(100°C) während 3Stunden zum Sieden erhitzt.Die farblose, klare Reaktionsmischung wurde mit 10N NaOH neutralisiert, zwei Mal mit Ethylacetat extrahiert, die vereinigten organischen Phasen mit Sole gewaschen, über MgSO₄ getrocknet und am Rotationsverdampfer vollständig eingeengt. Der erhaltene weisse Kristallkuchen wurde zerstossen und im Hochvakuum fraktioniert. Das Produkt destillierte bei einer Dampftemperatur von 138 °C und einem Druck von 4.10⁻² mbar. Ausbeute: 45.9 g (72% d.Th.) schneeweisse, geruchlose Kristalle mit einem Schmelzpunkt von 78-80 °C (unkorr.).
IR: 3433br, 2968, 2933, 2906, 2872, 2827, 2788, 2754, 2716 (CHO), 2685sh, 1757sh, 1719 (C=O Aldehyd), 1682 (C=O Harnstoff), 1493, 1471, 1447, 1420, 1400, 1379, 1365, 1347, 1332, 1273, 1249, 1202, 1168, 1141, 1116, 1106, 1050, 1028, 1021 sh, 992, 952, 909, 886, 870, 847sh, 775, 757, 671, 655.
¹H-NMR (CDCl₃, 300 K): δ 9.56 (*s*, 2 H, CHO), 3.27 (2×*s*, 2×4 H, NC*H*₂C(CH₃)₂ und 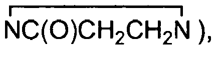 1.09 (*s*, 12 H, NCH₂C(C*H*₃)₂).

### N,N'-Bis(2,2-dimethyl-3-oxopropyl)-piperazin

In einem Rundkolben wurden unter Stickstoffatmosphäre 166.8 g (2.00 mol) 36%iger wässriger Formaldehyd und 150.4 g (2.08 mol) Isobutyraldehyd vorgelegt. Unter gutem Rühren und Eiskühlung wurden aus einem Eintropftrichter langsam 86.1 g (1.00 mol) Piperazin zugetropft, wobei darauf geachtet wurde, dass die Temperatur der Reaktionsmischung nicht über 20 °C anstieg. Nach erfolgter Zugabe liess man eine Stunde bei Raumtemperatur rühren. Die entstandene dickflüssige Suspension wurde im Ölbad bei 120 °C während 18 Stunden unter Rückfluss gerührt. Die klare, dunkelorange Reaktionsmischung wurde auf Raumtemperatur abgekühlt, wobei sie durchkristallisierte. Die feste Masse wurde mit einem Stössel zerkleinert, in Wasser suspendiert und abgenutscht. Das Rohprodukt wurde aus Ethylacetat umkristallisiert. Ausbeute (3 Fraktionen): 161.1 g (63% d.Th.) blassgelbe, geruchlose Kristallnadeln mit einem Amingehalt von 7.78 mmol N/g. IR:2964,2938,2871,2795,2752,2688(CHO),1719(C=O),1463,1400, 1374, 1360, 1341, 1323, 1283, 1152, 1123, 1054, 1017, 1005, 917, 869, 830, 775, 703. ¹H-NMR (CDCl₃, 300 K): δ 9.52 (*s*, 2 H, CHO), 2.44 (*s*, 4 H, NC*H*₂C(CH₃)₂), 2.39 (*s*, 8 H, NC*H*₂C*H*₂^{cycl.}), 1.04 (s, 12 H, CH₂C(C*H*₃)₂).

### N,N,N',N'-Tetrakis-(2,2-dimethyl-3-oxopropyl)-harnstoff

In einem Rundkolben mit aufgesetztem Rückflusskühler wurden unter Stickstoffatmosphäre 20.0 g (0.33 mol) Harnstoff, 111.1 g (1.33 mol) 36%iger wässriger Formaldehyd und 97.3 g (1.35 mol) Isobutyraldehyd vorgelegt und unter gutem Rühren mit 5.0 g conc. Salzsäure versetzt, wobei sich die Mischung deutlich erwärmte. Nach 10 Minuten wurde die Mischung im Ölbad (120 °C) während 18 Stunden zum Sieden erhitzt. Die weisse, trübe Reaktionsmischung wurde mit 10N NaOH neutralisiert, zwei Mal mit Dichlormethan extrahiert, die vereinigten organischen Phasen mit Sole gewaschen, über MgSO₄ getrocknet und am Rotationsverdampfer vollständig eingeengt. Das erhaltene dickflüssige Öl wurde mit Hilfe eines Dünnschichtverdampfers destilliert. Ausbeute: 33.1 g (25% d.Th.) blassgelbes, dickflüssiges, geruchloses Öl.
IR: 3370br, 2962, 2931 sh, 2912sh, 2870, 2709 (CHO), 1721 (C=O Aldehyd), 1640 (C=O Harnstoff), 1490, 1472, 1450, 1397, 1380, 1364, 1310, 1280, 1264, 1232, 1218, 1194, 1140, 1110, 1088, 1058, 1040, 1029sh, 1005, 986, 976sh, 948, 912, 894, 868, 844, 813, 772, 754, 735, 704.

### N,N'-Bis-(2,2-dimethyl-3-oxopropyl)-harnstoff

In einem Rundkolben mit aufgesetztem Rückflusskühler wurden unter Stickstoffatmosphäre 5.0 g (0.08 mol) Harnstoff, 13.9 g (0.17 mol) 36%iger wässriger Formaldehyd und 12.6 g (0.17 mol) Isobutyraldehyd vorgelegt und unter gutem Rühren mit 1.0 g conc. Salzsäure versetzt, wobei sich die Mischung erwärmte und ausklumpte. Nach 10 Minuten wurde die Mischung im Ölbad (120 °C) während 8 Stunden zum Sieden erhitzt. Die weisse, trübe Reaktionsmischung wurde mit 10N NaOH neutralisiert, zwei Mal mit Dichlormethan extrahiert, die vereinigten organischen Phasen mit Sole gewaschen, über MgSO₄ getrocknet und am Rotationsverdampfer vollständig eingeengt. Ausbeute: 18.2 g roher N,N'-Bis-(2,2-dimethyl-3-oxopropyl)-harnstoff als farb- und geruchloser, zäher Honig, welcher gemäss GC/MS-Analyse anteilig weitere Harnstoff-Aldehyde, insbesondere N,N-Bis-(2,2-dimethyl-3-oxopropyl)-harnstoff und N,N,N'-Tris-(2,2-dimethyl-3-oxopropyl)-harnstoff, aufwies. IR:3330br,3057,2967,2932,2911,2871,2716(CHO),1720(C=OAldehyd), 1637br (C=O Harnstoff),1490,1449,1403,1378,1367, 1304, 1265, 1204, 1141, 1086sh, 1055, 1039, 1029sh, 1003, 982, 953, 913, 894, 868, 838, 802, 759, 733, 701.

### 3. Herstellung von Aldiminen

### Beispiel 1: Aldimin A-1

In einem Rundkolben wurden unter Stickstoffatmosphäre 10.00 g (75 mmol) Terephthalaldehyd in 15.30 g 2-(2-Aminoethoxy)-ethanol (DGA; Diglycolamine^{®} Agent, Huntsman; Amingehalt 9.46 mmol N/g) suspendiert und unter Rühren erwärmt, wobei der Aldehyd sich rasch auflöste. Danach wurden die flüchtigen Bestandteile im Vakuum entfernt (10 mbar, 80 °C). Ausbeute: 22.7 g leicht trübes, braunorangefarbenes Öl mit einem Amingehalt von 6.19 mmol N/g, das beim Abkühlen auf Raumtemperatur innerhalb einiger Stunden durchkristallisierte. Schmelzpunkt: 47-49 °C (unkorr.).
IR: 3370br (OH), 2910sh, 2857, 1933br, 1697, 1639 (C=N), 1607, 1567, 1506, 1441, 1416, 1371 sh, 1355, 1339, 1298, 1216, 1162sh, 1120, 1058br, 1017sh, 967, 890br, 830, 813sh.

### Beispiel 2: Aldimin A-2

In einem Rundkolben wurden unter Stickstoffatmosphäre 10.00 g (39 mmol) 1,3-Bis-(2,2-dimethyl-3-oxopropyl)-imidazolidin-2-on in 7.84 g 2-(2-Aminoethoxy)-ethanol (DGA; Diglycolamine^{®} Agent, Huntsman; Amingehalt 9.46 mmol N/g) suspendiert, die Mischung unter Rühren erwärmt und die flüchtigen Bestandteile im Vakuum entfernt (zuerst 10 mbar, dann 4·10⁻² mbar, 80 °C), wobei sich der Aldehyd nach etwa 30 Minuten vollständig auflöste. Ausbeute: 16.48 g klarer, farbloser Honig mit einem Amingehalt von 4.46 mmol N/g und einer Viskosität von 10.3 Pa·s bei 20 °C.
IR: 3390br (OH), 2954, 2925, 2907, 2864, 2717, 1682sh (C=O), 1666 (C=N), 1493, 1444, 1425sh, 1391, 1362, 1335, 1275, 1253, 1204sh, 1162sh, 1123, 1060, 997sh, 928sh, 900sh, 892, 813, 786, 757, 677, 660.
¹H-NMR (CDCl₃, 300 K): δ 7.63 (*t*, *J* = 1.3, 2 H, CH=N), 3.70 und 3.56 (2×*m*, 2×8 H, HOC*H*₂C*H*₂OC*H*₂C*H*₂N), 3.38 (br s, 2 H, OH), 3.31 und 3.18 (2×*s*, 2x4 H, NC*H*₂C(CH₃)₂ und 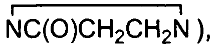 1.09 (*s*, 12 H, NCH₂C(C*H*₃)₂).

### Beispiel 3: Aldimin A-3

In einem Rundkolben wurden unter Stickstoffatmosphäre 10.00 g 2-(2-Aminoethoxy)-ethanol (DGA; Diglycolamine^{®} Agent, Huntsman; Amingehalt 9.46 mmol N/g) vorgelegt. Dazu wurden bei Raumtemperatur innerhalb von 5 Minuten 12.46 g (49 mmol) fein gemahlenes N,N'-Bis(2,2-dimethyl-3-oxo-propyl)-piperazin spatelweise zugegeben und die entstandene Suspension während 30 Minuten bei Raumtemperatur gerührt. Danach wurde die Mischung im Ölbad erwärmt und die flüchtigen Bestandteile im Vakuum entfernt (10 mbar, 85 °C). Ausbeute: 20.78 g schwach trübes, hellorangefarbenes Öl mit einem Amingehalt von 9.30 mmol N/g und einer Viskosität von 1.95 Pa.s bei 20 °C. IR: 3358br(OH),2929,2922,2904,2861, 2842, 2804, 1664 (C=N), 1458, 1441sh, 1378, 1358, 1336, 1320, 1280, 1235br, 1150, 1122, 1061, 1015, 890, 831. ¹H-NMR (CDCl₃, 300 K): δ 7.61 (s, 2 H, CH=N), 3.71 und 3.56 (2×*m*, 2x8 H, alle OCH₂), 2.42 (s, 8 H, NCH₂^{cycl}), 2.40 (br s, 2 H, OH), 2.33 (s, 4 H, NC*H*₂C(CH₃)₂), 1.04 (*s*, 12 H, CH₂C(C*H*₃)₂).

### Beispiel 4: Aldimin A-4

In einem Rundkolben wurden unter Stickstoffatmosphäre 10.00 g (25 mmol) N,N,N',N'-Tetrakis-(2,2-dimethyl-3-oxopropyl)-harnstoff vorgelegt und bei Raumtemperatur 10.66 g 2-(2-Aminoethoxy)-ethanol (DGA; Diglycolamine^{®} Agent, Huntsman; Amingehalt 9.46 mmol N/g) eingerührt. Danach wurde die Mischung im Ölbad erwärmt und die flüchtigen Bestandteile im Vakuum entfernt (zuerst 10 mbar/80 °C, dann 4·10⁻² mbar/100 °C). Ausbeute: 16.1 g blassgelber Honig mit einem Amingehalt von 3.07 mmol N/g und einer Viskosität von 13.5 Pa.s bei 50 °C.
IR: 3380br (OH), 2959, 2929, 2909, 2866, 1710, 1662sh (C=N), 1642, 1634, 1489, 1472, 1446, 1393, 1361, 1308, 1262, 1232, 1221sh, 1192, 1125, 1084sh, 1058, 1007, 991 sh, 977sh, 945, 908, 845, 804, 752, 733, 704, 662.

### Beispiel 5: Aldimin A-5

In einem Rundkolben wurden unter Stickstoffatmosphäre 15.00 g (66 mmol) roher N,N'-Bis-(2,2-dimethyl-3-oxopropyl)-harnstoff vorgelegt und bei Raumtemperatur 12.50 g 2-(2-Aminoethoxy)-ethanol (DGA; Diglycolamine^{®} Agent, Huntsman; Amingehalt 9.46 mmol N/g) eingerührt. Danach wurde die Mischung im Ölbad erwärmt und die flüchtigen Bestandteile im Vakuum entfernt (zuerst 10 mbar/80 °C, dann 4·10⁻² mbar/100 °C). Ausbeute: 25.9 g leicht trübes, blassgelbes Öl mit einem Amingehalt von 4.86 mmol N/g und einer Viskosität von 5.2 Pa.s bei 20 °C.
IR: 3350br (OH), 2948, 2924, 2910, 2862, 1661sh (C=N), 1642, 1490, 1451, 1378sh, 1365, 1352, 1303, 1274, 1203, 1122, 1058, 984,955,894,839,802,760.

### Vergleichsbeispiel 6: Aldimin A-6

In einem Rundkolben wurden unter Stickstoffatmosphäre 28.06 g (0.099 mol) 2,2-Dimethyl-3-lauroyloxy-propanal vorgelegt. Unter kräftigem Rühren wurden aus einem Eintropftrichter 10.00 g (0.095 mol) 2-(2-Aminoethoxy)-ethanol (DGA; Diglycolamine^{®} Agent, Huntsman; Amingehalt 9.46 mmol N/g) zugegeben. Danach wurden die flüchtigen Bestandteile im Vakuum entfernt (10 mbar, 80 °C). Ausbeute: 36.3 g farblose, bei Raumtemperatur dünnflüssige, klare und geruchlose Flüssigkeit mit einem Amingehalt von 2.58 mmol N/g.

### 4. Herstellung vonzweikomponentigen Polyrethanzusammensetzungen

### Beispiele 7 bis 9, Vergleichsbeispiele 10 und 11: Vergussmassen

Für jedes Beispiel wurden die jeweiligen Bestandteile der Komponente **K1** gemäss Tabelle 1 in den angegebenen Gewichtsteilen ohne vorherige Trocknung in einen Polypropylenbecher mit Schraubverschluss eingewogen und mittels eines Zentrifugalmischers (SpeedMixer™ DAC 150, FlackTek Inc.; 2 min. bei 3000 U/min) zu einer homogenen Creme gemischt. Dazu wurden die in der Tabelle 1 angegebenen Gewichtsteile an PMDI als Komponente **K2** zugegeben und homogen eingemischt (30 sec. bei 3000 U/min). Das Verhältnis zwischen den Isocyanatgruppen der Komponente **K2** und der Summe der Reaktivgruppen (Hydroxyl- und Aldimingruppen) der Komponente **K1** beträgt stets 1.1.
Das verwendete Ricinusöl war von Fluka und wies eine OH-Zahl von 165 mg KOH/g auf. Als Dimerfettsäurediol wurde Sovermol^{®} 908 von Cognis mit einer OH-Zahl von 200 mg KOH/g verwendet. Als Triol wurde Desmophen^{®} 4011 T von Bayer mit einer OH-Zahl von 550 mg KOH/g verwendet. Als Säurekatalysator wurde eine Lösung von 5 Gew.-% Salicylsäure in Dioctyladipat verwendet. Als Kreide wurde Omyacarb^{®} 5-GU von Omya eingesetzt. Das PMDI war Desmodur^{®} VKS 20 F von Bayer mit einem NCO-Gehalt von 30.0 Gew.-%.

**Tabelle 1: Zusammensetzung der Beispiele 7 bis 11.**

| Beispiel | **7** | **8** | **9** | **10 (Vgl.)** | **11 (Vgl.)** |
|---|---|---|---|---|---|
| **Komponente K1:** | | | | | |
| Ricinusöl | 22.5 | 22.5 | 22.5 | 22.5 | 22.5 |
| Dimerfettsäurediol | 17.5 | 17.5 | 17.5 | 17.5 | 17.5 |
| Triol | 4.75 | 4.75 | 4.75 | 4.75 | 4.75 |
| Aldimin | ***A-1*** 5.0 | ***A-2*** 5.0 | ***A-3*** 5.0 | ***A-6*** 5.0 | - |
| Tripropylenglykol | - | - | - | - | 5.0 |
| Säurekatalysator | 0.25 | 0.25 | 0.25 | 0.25 | 0.25 |
| Kreide | 50.0 | 50.0 | 50.0 | 50.0 | 50.0 |
| **Komponente K2:** | | | | | |
| PMDI | 36.5 | 33.9 | 34.0 | 31.0 | 35.0 |

Die so erhaltenen vermischten Zusammensetzungen (Vergussmassen) wurden auf Offenzeit, Aushärtegeschwindigkeit und mechanische Eigenschaften nach der Aushärtung geprüft. Die **Offenzeit** der Vergussmasse wurde als "gut" bezeichnet, wenn diese sich bis zu einem Zeitpunkt von maximal 3 Minuten nach dem Vermischen der beiden Komponenten **K1** und **K2** bei Raumtemperatur leicht ausgiessen und damit wie gewünscht verarbeiten liess, somit also keine die Verarbeitung behindernde oder verunmöglichende Viskositätserhöhung zeigte. Hinweise zur Aushärtegeschwindigkeit wurden zum einen durch Messen der **Zeit bis zur Klebefreiheit** ("tack-free time") - in der Tabelle als "Klebefreiheit" bezeichnet - erhalten. Dazu wurde die Vergussmasse unmittelbar nach dem Vermischen der beiden Komponenten **K1** und **K2** in einer Schichtdicke von ca. 2 mm auf Pappkarton aufgetragen und im Normklima (23±1 °C, 50±5% relative Luftfeuchtigkeit) die Zeit bestimmt, die es dauerte, bis beim leichten Antippen der Oberfläche der Vergussmasse mittels einer Pipette aus LDPE erstmals keine Rückstände auf der Pipette mehr zurückblieben. Zum anderen wurde der weitere Verlauf der Aushärtung (und damit indirekt des Festigkeitsaufbaus) durch periodisches Messen der **Shore D-Härte** nach DIN EN 53505 verfolgt. Als "getempert" wurde dabei eine Lagerung von 4 h bei 105 °C der während 7 Tagen im Normklima ausgehärteten Prüfkörper bezeichnet. Zur Prüfung der mechanischen Eigenschaften wurde die Vergussmasse als Film mit einer Schichtdicke von ca. 2 mm in eine plane PTFE-Form gegossen, der Film während 7 Tagen im Normklima ausgehärtet und nach DIN EN 53504 auf **Zugfestigkeit, Bruchdehnung** und Elastizitätsmodul (**E-Modul**) bei 0.5-1.0% Dehnung (Zuggeschwindigkeit: 10 mm/min) geprüft. In qualitativer Weise beurteilt wurde ausserdem die **Blasenbildung** (anhand der Menge Blasen, die während der Aushärtung des Films auftraten) sowie der **Geruch**.

Die Ergebnisse dieser Prüfungen sind in der Tabelle 2 aufgeführt.

**Tabelle 2: Eigenschaften der Beispiele 7 bis 11.**

| Beispiel | **7** | **8** | **9** | **10 (Vgl.)** | **11 (Vgl.)** |
|---|---|---|---|---|---|
| Offenzeit | gut | gut | gut | gut | gut |
| Klebefreiheit [min.] | 15 | 37 | 13 | 52 | 108 |
| Shore D nach 1 Tag | 92 | 83 | 91 | 62 | 71 |
| Shore D nach 3 Tagen | 96 | 92 | 96 | 84 | 84 |
| Shore D nach 7 Tagen | 96 | 95 | 97 | 88 | 86 |
| Shore D getempert | 98 | 96 | 98 | 92 | 94 |
| Zugfestigkeit [MPa] | 29.7 | 22.7 | 32.0 | 10.6 | 9.6 |
| Bruchdehnung [%] | 3 | 5 | 3 | 50 | 30 |
| E-Modul [MPa] | 1640 | 1050 | 1830 | 210 | 250 |
| Blasenbildung | keine | keine | keine | keine | viele |
| Geruch | schwach | kein | kein | kein | kein |

Aus der Tabelle 2 ist ersichtlich, dass die erfindungsgemässen Vergussmassen der Beispiele 7 bis 9 eine für die Verarbeitung ausreichend lange Offenzeit und eine sehr schnelle Aushärtung ohne Blasenbildung aufweisen. Sie erreichen nach wenigen Tagen im Normklima bereits ihre Endhärte. Eine Temperung ist nicht notwendig. Sie weisen sehr hohe Zugfestigkeiten und E-Module auf. Die Vergussmasse des Vergleichsbeispiels 10, welche eine gleiche Menge Aldimin abgeleitet von einem langkettigen Monoaldehyd enthält, härtet deutlich langsamer aus und weist eine geringere Zugfestigkeit und ein tieferes E-Modul auf. Die Vergussmasse des Vergleichsbeispiels 11, welche anstelle eines Aldimins eine gleiche Menge eines Glykols enthält, härtet ebenfalls langsamer aus, weist eine geringere Zugfestigkeit und ein tieferes E-Modul auf und zeigt bei der Aushärtung deutliche Blasenbildung. Die Vergussmasse des Beispiels 7 weist bei der Aushärtung einen schwachen Geruch auf, während jene der Beispiele 8 bis 11 geruchsfrei sind.

### Beispiele 12 bis 15 und Vergleichsbeispiel 16: Klebstoffe

Für jedes Beispiel wurden die jeweiligen Bestandteile der Komponente K1 gemäss Tabelle 3 in den angegebenen Gewichtsteilen ohne vorherige Trocknung in einen Polypropylenbecher mit Schraubverschluss eingewogen und mittels eines Zentrifugalmischers (SpeedMixer™ DAC 150, FlackTek Inc.; 2 min. bei 3000 U/min) zu einer homogenen Creme gemischt. Dazu wurden die in der Tabelle 3 angegebenen Gewichtsteile der Komponente **K2**, welche wie nachfolgend beschrieben hergestellt wurde, zugegeben und eingemischt (30 sec. bei 3000 U/min). Das Verhältnis zwischen den Isocyanatgruppen der Komponente **K2** und der Summe der Reaktivgruppen (Hydroxyl- und Aldimingruppen) der Komponente **K1** beträgt stets 1.1.
Die Komponente **K2** wurde wie folgt hergestellt:
In einem Mischer wurden unter Feuchtigkeitsausschluss 30 Gewichtsteile PMDI (Desmodur^{®} VKS 20 F, Bayer; NCO-Gehalt = 30.0 Gew.-%), 10 Gewichtsteile modifiziertes MDI (Desmodur^{®} CD, Bayer; NCO-Gehalt = 29.5 Gew.-%) und 60 Gewichtsteile Polyurethanpolymer ***P-1*** homogen gemischt.
Das Polyurethanpolymer ***P-1*** wurde wie folgt hergestellt:
1300 g Polyoxypropylen-Diol (Acclaim^{®} 4200 N, Bayer; OH-Zahl 28.5 mg KOH/g), 2600 g Polyoxypropylenpolyoxyethylen-Triol (Caradol^{®} MD34-02, Shell; OH-Zahl 35.0 mg KOH/g), 600 g 4,4'-Methylendiphenyldiisocyanat (4,4'-MDI; Desmodur^{®} 44 MC L, Bayer) und 500 g Diisodecylphthalat (DIDP; Palatinol^{®} Z, BASF) wurden nach bekanntem Verfahren bei 80 °C zu einem NCO-terminierten Polyurethanpolymeren mit einem Gehalt an freien IsocyanatGruppen von 2.05 Gewichts-% umgesetzt.
Als Ricinusöl, als Kreide und als Säurekatalysator wurden dieselben wie für das Beispiel 7 verwendet. Als Polyol wurde das Polypropylenglykol Acclaim^{®} 4200 von Bayer mit einer OH-Zahl von 28.5 mg KOH/g verwendet. Als Molekularsieb wurde Purmol^{®} 13 von Zeochem Europe (in aktivierter Form) verwendet. Als Aminkatalysator wurde DABCO^{®} 33-LV von Air Products verwendet.

**Tabelle 3: Zusammensetzung der Beispiele 12 bis 16.**

| Beispiel | **12** | **13** | **14** | **15** | **16 (Vgl.)** |
|---|---|---|---|---|---|
| **Komponente K1:** | | | | | |
| Ricinusöl | 9.0 | 9.0 | 9.0 | 9.0 | 9.0 |
| Polyol | 35.0 | 35.0 | 35.0 | 35.0 | 35.0 |
| Aldimin | ***A-2*** | ***A-3*** | ***A-4*** | ***A*-*5*** | ***A-6*** |
| | 5.0 | 5.0 | 5.0 | 5.0 | 5.0 |
| Kreide | 48.0 | 48.0 | 48.0 | 48.0 | 48.0 |
| Molekularsieb | 2.0 | 2.0 | 2.0 | 2.0 | 2.0 |
| Aminkatalysator | 0.1 | 0.1 | 0.1 | 0.1 | 0.1 |
| Säurekatalysator | 0.9 | 0.9 | 0.9 | 0.9 | 0.9 |
| **Komponente K2:** | 31.7 | 31.4 | 25.9 | 32.9 | 25.0 |

Die so erhaltenen vermischten Zusammensetzungen (Klebstoffe) wurden im Wesentlichen auf dieselbe Weise wie für das Beispiel 7 beschrieben geprüft. Anstelle der Shore D-Härte wurden aber die Shore A-Härte gemessen; die Zugfestigkeit, Bruchdehnung und das E-Modul wurden an Filmen mit einer Schichtdicke von 3 mm und mit einer Zuggeschwindigkeit von 200 mm/min geprüft, und das E-Modul wurde bei 0.5-5.0 % Dehnung gemessen. Die Ergebnisse dieser Prüfungen sind in der Tabelle 4 aufgeführt.

**Tabelle 4: Eigenschaften der Beispiele 12 bis 16.**

| Beispiel | **12** | **13** | **14** | **15** | **16 (Vgl.)** |
|---|---|---|---|---|---|
| Offenzeit | gut | gut | gut | gut | gut |
| Klebefreiheit [min.] | 60 | 25 | 30 | 50 | 105 |
| Shore A nach 8 Stunden | 20 | 39 | 33 | 33 | 12 |
| Shore A nach 1 Tag | 54 | 57 | 52 | 54 | 45 |
| Shore A nach 2 Tagen | 58 | 59 | 56 | 57 | 52 |
| Shore A nach 3 Tagen | 61 | 62 | 59 | 60 | 56 |
| Shore A nach 7 Tagen | 62 | 63 | 60 | 61 | 58 |
| Shore A getempert | 64 | 60 | 60 | 60 | 62 |
| Zugfestigkeit [MPa] | 2.1 | 2.0 | 2.2 | 1.2 | 1.6 |
| Bruchdehnung [%] | 100 | 115 | 100 | 95 | 75 |
| E-Modul [MPa] | 3.6 | 2.2 | 4.3 | 2.0 | 3.7 |
| Blasenbildung | keine | keine | keine | keine | keine |
| Geruch | kein | kein | kein | kein | kein |

Aus der Tabelle 4 ist ersichtlich, dass die erfindungsgemässen Klebstoffe der Beispiele 12 bis 15 eine für die Verarbeitung ausreichend lange Offenzeit, eine schnelle und blasenfreie Aushärtung sowie eine hohe Zugfestigkeit und ein hohes E-Modul aufweisen. Die Klebstoffe der Beispiele 13 und 14 weisen eine besonders hohe Aushärtungsgeschwindigkeit auf; beim Klebstoff des Beispiels 13 ist dies möglicherweise auf den zusätzlichen katalytischen Effekt der tertiären Aminogruppen im Aldehyd-Teil des Aldimins ***A-3*** zurückzuführen, während beim Klebstoff des Beispiels 14 möglicherweise die hohe OH-Funktionalität des Aldimins ***A-4*** für den raschen Festigkeitsaufbau mitverantwortlich ist.

## Patentansprüche

1. Zusammensetzung enthaltend
a) mindestens ein Aldimin **A** der Formel (I), 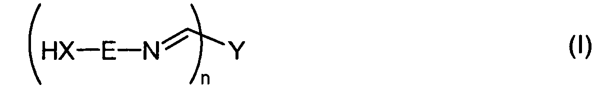 wobei n für 2 oder 3 oder 4 steht,
E entweder für einen zweiwertigen Kohlenwasserstoffrest mit 3 bis 20 C-Atomen steht, oder zusammen mit R¹¹ für einen dreiwertigen Kohlenwasserstoffrest mit 3 bis 20 C-Atomen steht, wobei E gegebenenfalls Heteroatome in der Form von Ether-Sauerstoff oder tertiärem Amin-Stickstoff enthält,
Y für einen n-wertigen organischen Rest mit 6 bis 30 C-Atomen, welcher gegebenenfalls Stickstoff- und/oder Sauerstoffatome in Form von tertiären Aminogruppen, Ether-, Ester-, Carbonat-, Amid-, Urethan- oder Harnstoffgruppen aufweist, steht,
X für O oder S oder N-R¹⁰ oder N-R¹¹ steht, wobei R¹⁰ für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht, und
R¹¹ zusammen mit E für einen dreiwertigen Kohlenwasserstoffrest mit 3 bis 20 C-Atomen, welcher gegebenenfalls Heteroatome in der Form von Ether-Sauerstoff oder tertiärem Amin-Stickstoff enthält, steht; und
b) mindestens ein Polyisocyanat **P**.

2. Zusammensetzung gemäss Anspruch 1, **dadurch gekennzeichnet, dass** das Aldimin **A** die Formel (II) aufweist 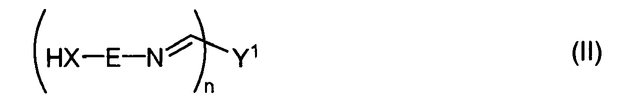 wobei Y¹ für einen n-wertigen, substituierten oder unsubstituierten Aryl- oder Heteroaryl-Rest, welcher eine Ringgrösse von 5 bis 8, bevorzugt 6, Atomen aufweist, steht.

3. Zusammensetzung gemäss Anspruch 1, **dadurch gekennzeichnet, dass** das Aldimin **A** die Formel (III)aufweist 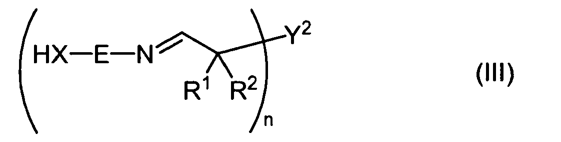 wobei
R¹ und R² entweder unabhängig voneinander jeweils für einen einwertigen Kohlenwasserstoffrest mit 1 bis 12 C-Atomen stehen,
oder zusammen für einen zweiwertigen Kohlenwasserstoffrest mit 4 bis 12 C-Atomen, der Teil eines, gegebenenfalls substituierten, carbocyclischen Rings mit 5 bis 8, bevorzugt 6, C-Atomen ist, stehen;
Y² für einen n-wertigen organischen Rest mit 1 bis 24 C-Atomen steht, welcher gegebenenfalls Stickstoff- und/oder Sauerstoffatome in Form von tertiären Aminogruppen, Ether-, Ester-, Carbonat-, Amid-, Urethan- oder Harnstoffgruppen aufweist.

4. Zusammensetzung gemäss Anspruch 3, **dadurch gekennzeichnet, dass** das Aldimin **A** die Formel (III a) aufweist 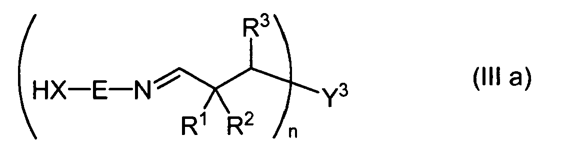 wobei R³ für ein Wasserstoffatom oder für einen Alkyl-, Cycloalkyl-, Arylalkyl- oder Alkoxycarbonylrest mit 1 bis 12 C-Atomen steht;
Y³ für einen n-wertigen Rest steht, welcher ausgewählt ist aus der Gruppe bestehend aus 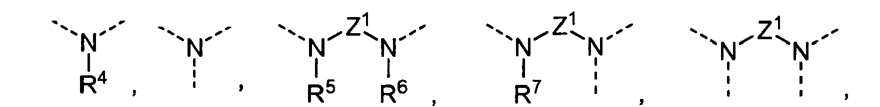 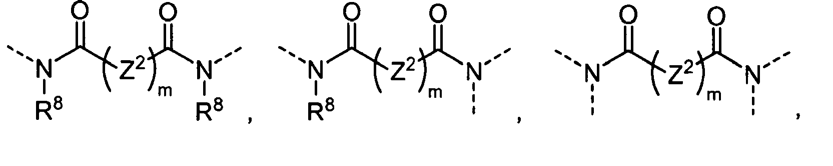 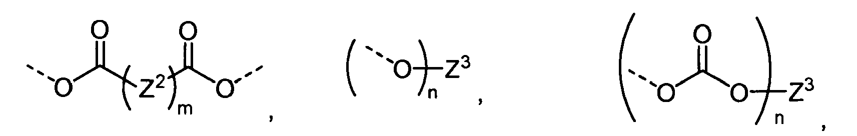 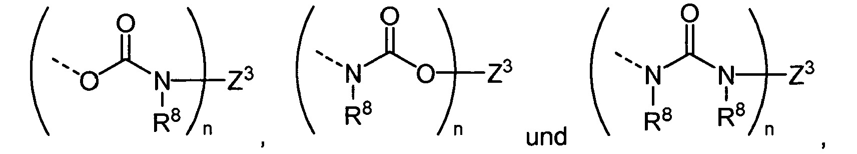 wobei
m für 0 oder 1 steht;
Z¹ entweder für eine Carbonylgruppe oder für einen Alkylenrest mit 2 bis 15 C-Atomen, welcher gegebenenfalls mindestens eine Ether- Gruppe aufweist, steht;
Z² für einen zweiwertigen Kohlenwasserstoffrest mit 1 bis 15 C-Atomen, welcher gegebenenfalls mindestens eine Ether-, Carbonyl- oder Carboxylgruppe aufweist, steht;
Z³ für einen n-wertigen Kohlenwasserstoffrest mit 2 bis 15 C-Atomen, welcher gegebenenfalls mindestens eine Ether- oder Carbonylgruppe aufweist, steht;
R⁴ für einen Alkyl-, Cycloalkyl- oder Arylalkylrest mit 1 bis 20 C-Atomen steht;
R⁵ und R⁶ entweder
unabhängig voneinander jeweils für einen Alkyl-, Cycloalkyl- oder Arylalkylrest mit 1 bis 12 C-Atomen stehen,
oder, für den Fall dass Z¹ für eine Carbonylgruppe steht, unabhängig voneinander jeweils für ein Wasserstoffatom oder für einen Alkyl-, Cycloalkyl- oder Arylalkylrest mit 1 bis 12 C- Atomen stehen;
oder zusammen für einen Alkylenrest mit 2 bis 20 C-Atomen, welcher zusammen mit N-Z¹-N einen 5- bis 12-gliedrigen Ring bildet und gegebenenfalls mindestens eine Ether-Gruppe aufweist, steht;
R⁷ entweder
für einen Alkyl-, Cycloalkyl- oder Arylalkylrest mit 1 bis 15 C-Atomen steht;
oder, für den Fall dass Z¹ für eine Carbonylgruppe steht, für ein Wasserstoffatom oder für einen Alkyl-, Cycloalkyl- oder Aryl- alkylrest mit 1 bis 15 C-Atomen steht;
und R⁸ für ein Wasserstoffatom oder für einen Alkyl-, Cycloalkyl- oder Arylalkylrest mit 1 bis 8 C-Atomen steht.

5. Zusammensetzung gemäss Anspruch 4, **dadurch gekennzeichnet, dass** das Y³ für den Rest 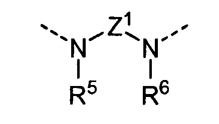 steht.

6. Zusammensetzung gemäss einem der vorhergehenden Ansprüche, **dadurch gekennzeichnet, dass** das Aldimin der Formel (I) aus der Umsetzung von mindestens einem Amin **B** der Formel (IV) mit mindestens einem Polyaldehyd **ALD** der Formel (V) hergestellt wird
HX-E-NH₂ (IV) 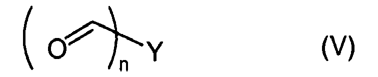
und das Amin **B** der Formel (IV) ausgewählt ist aus der Gruppe bestehend aus 5-Amino-1-pentanol, 6-Amino-1-hexanol oder höheren Homologen davon, 4-(2-Aminoethyl)-2-hydroxyethylbenzol, 3-Aminomethyl-3,5,5-trimethyl-cyclohexanol, 2-(2-Aminoethoxy)-ethanol, Triethylenglykol-monoamin oder höheren Homologen davon, 3-(2-Hydroxyethoxy)-propylamin, 3-(2-(2-Hydroxyethoxy)-ethoxy)-propylamin, 3-(6-Hydroxyhexyloxy)-propylamin, N-Methyl-1,3-propandiamin, N-Ethyl-1,3-propandiamin, N-Butyl-1,3-propandiamin, N-(2-Ethylhexyl)-1,3-propandiamin, N-Dodecyl-1,3-propandiamin, N-Cyclohexyl-1,3-propandiamin, N-Cocoalkyl-1,3-propandiamin, N-Oleyl-1,3-propandiamin, N-Soyaalkyl-1,3-propandiamin, N-Talg-alkyl-1,3-propandiamin und N-(C₁₆₋₂₂-Alkyl)-1,3-propandiamin.

7. Zusammensetzung gemäss einem der vorhergehenden Ansprüche, **dadurch gekennzeichnet, dass** die Zusammensetzung zweikomponentig ist und aus der Komponente **K1** enthaltend
a) mindestens ein Aldimin **A** der Formel (I) und
b) mindestens eine Substanz **RS**, welche mindestens zwei gegenüber Isocyanatgruppen reaktive Gruppen aufweist, und/oder Wasser, und der Komponente **K2** enthaltend
c) mindestens ein Polyisocyanat **P**
besteht.

8. Additionsprodukt **AV** erhalten aus der Reaktion eines Aldimin **A** der Formel (I) mit einem Polyisocyanat **P**, wie sie in einer Zusammensetzung gemäss einem der Ansprüche 1 bis 7 vorhanden sind.

9. Additionsprodukt gemäss Anspruch 8, **dadurch gekennzeichnet, dass** das Additionsprodukt **AV** die Formel (XVII) aufweist 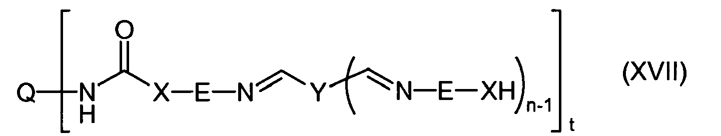 wobei Q für den Rest eines Polyisocyanats **P** nach Entfernung von t Isocyanatgruppen steht und t für 2 oder 3, insbesondere für 2, steht.

10. Additionsprodukt gemäss Anspruch 8, **dadurch gekennzeichnet, dass** das Additionsprodukt **AV** die Formel (XVIII) aufweist 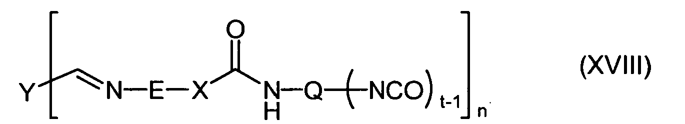 wobei Q für den Rest eines Polyisocyanats **P** nach Entfernung von t Isocyanatgruppen steht und t für 2 oder 3, insbesondere für 2, steht.

11. Aldimin der Formel (III) 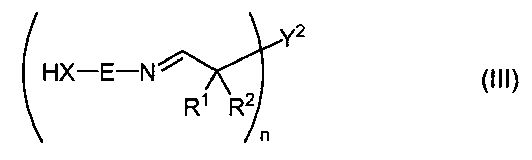 wobei
R¹ und R² entweder
unabhängig voneinander jeweils für einen einwertigen Kohlenwasserstoffrest mit 1 bis 12 C-Atomen stehen,
oder
zusammen für einen zweiwertigen Kohlenwasserstoffrest mit 4 bis 12 C-Atomen, der Teil eines, gegebenenfalls substituierten, carbocyclischen Rings mit 5 bis 8, bevorzugt 6, C-Atomen ist, stehen;
Y² für einen n-wertigen organischen Rest mit 1 bis 24 C-Atomen steht, welcher gegebenenfalls Stickstoff- und/oder Sauerstoffatome in Form von tertiären Aminogruppen, Ether-, Ester-, Carbonat-, Amid-, Urethan- oder Harnstoffgruppen aufweist;
E entweder für einen zweiwertigen Kohlenwasserstoffrest mit 3 bis 20 C-Atomen steht, oder zusammen mit R¹¹ für einen dreiwertigen Kohlenwasserstoffrest mit 3 bis 20 C-Atomen steht, wobei E gegebenenfalls Heteroatome in der Form von Ether-Sauerstoff oder tertiärem Amin-Stickstoff enthält,
X für O oder S oder N-R¹⁰ oder N-R¹¹ steht,
wobei
R¹⁰ für einen einwertigen Kohlenwasserstoffrest mit 1 bis 20 C- Atomen, welcher gegebenenfalls mindestens eine Carbon- säureester-, Nitril-, Nitro-, Phosphonsäureester-, Sulfon- oder Sulfonsäureester-Gruppe aufweist, steht, und
R¹¹ zusammen mit E für einen dreiwertigen Kohlenwasserstoffrest mit 3 bis 20 C-Atomen, welcher gegebenenfalls Heteroatome in der Form von Ether-Sauerstoff oder tertiärem Amin- Stickstoff enthält, steht;
und n für 2 oder 3 oder 4 steht.

12. Aldimin gemäss Anspruch 11, **dadurch gekennzeichnet, dass** das Aldimin die Formel (III a) aufweist 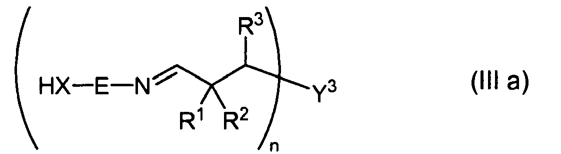 wobei
R³ für ein Wasserstoffatom oder für einen Alkyl-, Cycloalkyl-, Arylalkyl- oder Alkoxycarbonylrest mit 1 bis 12 C-Atomen steht;
Y³ für einen n-wertigen Rest steht, welcher ausgewählt ist aus der Gruppe bestehend aus 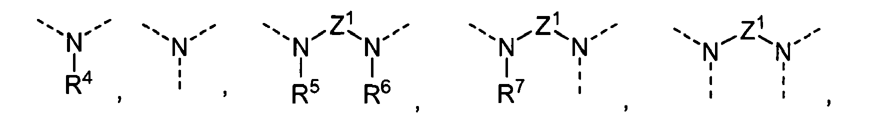 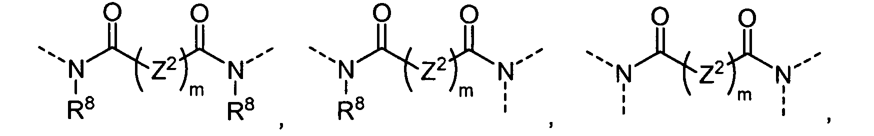 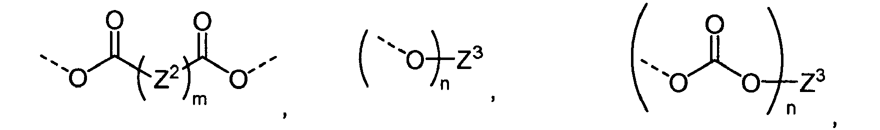 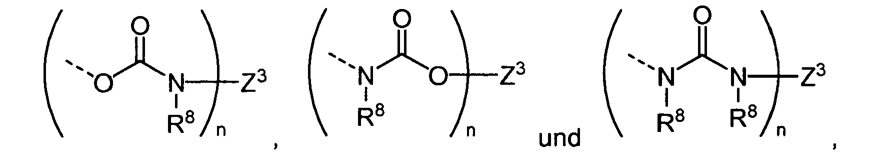 wobei
m für 0 oder 1 steht;
Z¹ entweder für eine Carbonylgruppe oder für einen Alkylenrest mit 2 bis 15 C-Atomen, welcher gegebenenfalls mindestens eine Ether- Gruppe aufweist, steht;
Z² für einen zweiwertigen Kohlenwasserstoffrest mit 1 bis 15 C-Atomen, welcher gegebenenfalls mindestens eine Ether-, Carbonyl- oder Carboxylgruppe aufweist, steht;
Z³ für einen n-wertigen Kohlenwasserstoffrest mit 2 bis 15 C-Atomen, welcher gegebenenfalls mindestens eine Ether- oder Carbonylgruppe aufweist, steht;
R⁴ für einen Alkyl-, Cycloalkyl- oder Arylalkylrest mit 1 bis 20 C-Atomen steht;
R⁵ und R⁶ entweder
unabhängig voneinander jeweils für einen Alkyl-, Cycloalkyl- oder Arylalkylrest mit 1 bis 12 C-Atomen stehen,
oder, für den Fall dass Z¹ für eine Carbonylgruppe steht, unabhängig voneinander jeweils für ein Wasserstoffatom oder für einen Alkyl-, Cycloalkyl- oder Arylalkylrest mit 1 bis 12 C- Atomen stehen;
oder zusammen für einen Alkylenrest mit 2 bis 20 C-Atomen, welcher zusammen mit N-Z¹-N einen 5- bis 12-gliedrigen Ring bildet und gegebenenfalls mindestens eine Ether-Gruppe aufweist, steht;
R⁷ entweder
für einen Alkyl-, Cycloalkyl- oder Arylalkylrest mit 1 bis 15 C-Atomen steht;
oder, für den Fall dass Z¹ für eine Carbonylgruppe steht, für ein Wasserstoffatom oder für einen Alkyl-, Cycloalkyl- oder Aryl- alkylrest mit 1 bis 15 C-Atomen steht;
und R⁸ für ein Wasserstoffatom oder für einen Alkyl-, Cycloalkyl- oder Arylalkylrest mit 1 bis 8 C-Atomen steht.

13. Verfahren zum Verkleben von einem Substrat **S1** mit einem Substrat **S2**, welches die Schritte umfasst
i) Applikation einer Zusammensetzung gemäss einem der Ansprüche 1 bis 7 auf ein Substrat **S1**;
ii) Kontaktieren der applizierten Zusammensetzung mit einem Substrat **S2** innerhalb der Offenzeit der Zusammensetzung;
oder
i') Applikation einer Zusammensetzung gemäss einem der Ansprüche 1 bis 7 auf ein Substrat **S1** und auf ein Substrat **S2**;
ii') Kontaktieren der applizierten Zusammensetzung miteinander innerhalb der Offenzeit der Zusammensetzung;
wobei das Substrat **S2** aus dem gleichen oder einem unterschiedlichen Material wie das Substrat **S1** besteht.

14. Verwendung einer Zusammensetzung gemäss einem der Ansprüche 1 bis 7 als Klebstoff, Dichtstoff, Vergussmasse, Beschichtung, Bodenbelag, Anstrich, Lack oder Primer.

15. Ausgehärtete Zusammensetzung erhalten aus einer Zusammensetzung gemäss einem der Ansprüche 1 bis 7 und Feuchtigkeit.

## Claims

1. Composition that contains
a) At least one aldimine **A** of Formula (I), 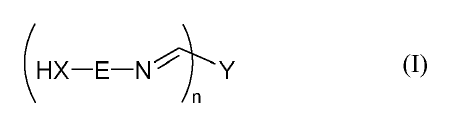 whereby n stands for 2 or 3 or 4,
E either stands for a divalent hydrocarbon radical with 3 to 20 C atoms, or together with R¹¹ for a trivalent hydrocarbon radical with 3 to 20 C atoms, whereby E optionally contains heteroatoms in the form of ether-oxygen or tertiary amine-nitrogen,
Y stands for an n-value organic radical with 6 to 30 C atoms, which optionally has nitrogen and/or oxygen atoms in the form of tertiary amino groups, or ether, ester, carbonate, amide, urethane or urea groups,
X stands for O or S or N-R¹⁰ or N-R¹¹, whereby
R¹⁰ stands for a monovalent hydrocarbon radical with 1 to 20 C atoms, which optionally has at least one carboxylic acid ester, nitrile, nitro, phosphonic acid ester, sulfonic or sulfonic acid ester group, and
R¹¹ together with E stands for a trivalent hydrocarbon radical
with
3 to 20 C atoms, which optionally contains heteroatoms in the form of ether-oxygen or tertiary amine-nitrogen; and
b) At least one polyisocyanate **P**.

2. Composition according to Claim 1, **characterized in that** the aldimine **A** has Formula (II) 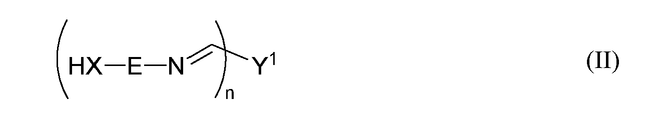 whereby Y¹ stands for an n-value, substituted or unsubstituted aryl or heteroaryl radical, which has a ring size of 5 to 8, preferably 6, atoms.

3. Composition according to Claim 1, wherein the aldimine A has Formula (III) 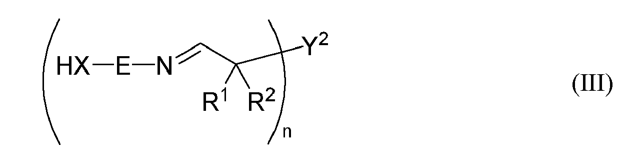 whereby
R¹ and R² either independently of one another in each case stand for a monovalent hydrocarbon radical with 1 to 12 C atoms,
or together stand for a divalent hydrocarbon radical with 4 to 12 C atoms, which is part of an optionally substituted, carbocyclic ring with 5 to 8, preferably 6, C atoms;
Y² stands for an n-value organic radical with 1 to 24 C atoms, which optionally
has nitrogen and/or oxygen atoms in the form of tertiary amino groups, or ether, ester, carbonate, amide, urethane or urea groups.

4. Composition according to Claim 3, wherein the aldimine **A** has Formula (III a) 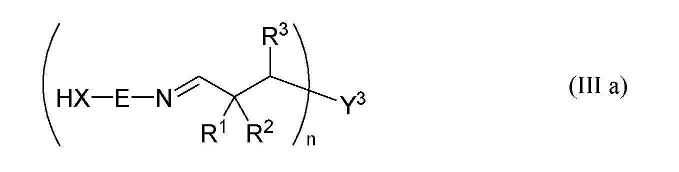 whereby R³ stands for a hydrogen atom or for an alkyl, cycloalkyl, arylalkyl or alkoxycarbonyl radical with 1 to 12 C atoms;
Y³ stands for an n-value radical, which is selected from the group that consists of 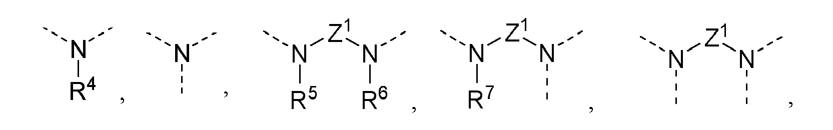 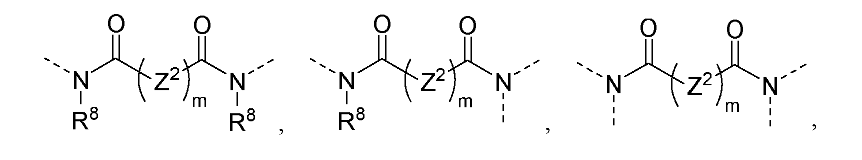 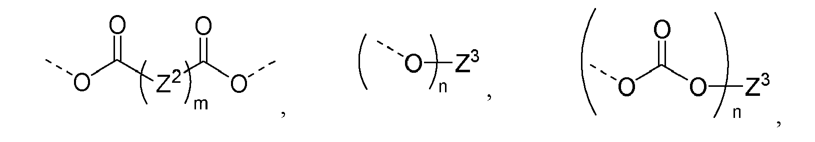 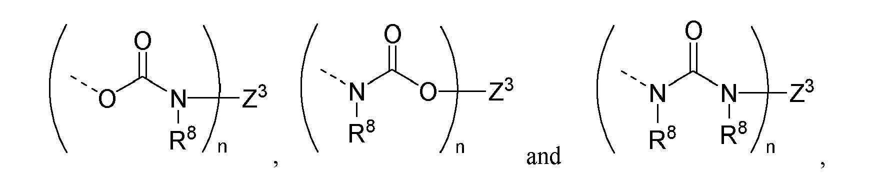 whereby
m stands for 0 or 1;
Z¹ either stands for a carbonyl group or for an alkylene radical with 2 to 15 C atoms, which optionally has at least one ether group;
Z² stands for a divalent hydrocarbon radical with 1 to 15 C atoms, which optionally has at least one ether, carbonyl or carboxyl group;
Z³ stands for an n-value hydrocarbon radical with 2 to 15 C atoms, which optionally has at least one ether or carbonyl group;
R⁴ stands for an alkyl, cycloalkyl or arylalkyl radical with 1 to 20 C atoms;
R⁵ and R⁶ cycloalkyl either
independently of one another in each case stand for an alkyl, or
arylalkyl radical with 1 to 12 C atoms, or, for the case that Z¹ stands for a carbonyl group, independently of one
another in each case stand for a hydrogen atom or for an alkyl, cycloalkyl or arylalkyl radical with 1 to 12 C atoms;
or together for an alkylene radical with 2 to 20 C atoms, which
together with N-Z¹-N form a 5- to 12-membered ring and optionally has at least one ether group;
R⁷ either
stands for an alkyl, cycloalkyl or arylalkyl radical with 1 to 15 C atoms;
or, for the case that Z¹ stands for a carbonyl group, for a hydrogen atom or
for an alkyl, cycloalkyl or arylalkyl radical with 1 to 15 C atoms;
and R⁸ stands for a hydrogen atom or for an alkyl, cycloalkyl or arylalkyl radical with 1 to 8 C atoms.

5. Composition according to Claim 4, wherein Y₃ stands for the radical

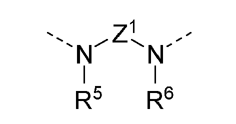

6. Composition according to one of the preceding claims, wherein the aldimine of Formula (I) from the reaction of at least one amine **B** of Formula (IV) is produced with at least one polyaldehyde **ALD** of Formula (V)
HX-E-NH₂ (IV) 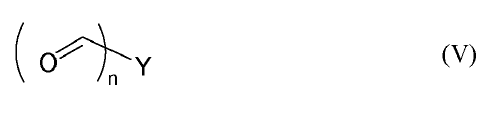
and the amine **B** of Formula (IV) is selected from the group that consists of 5-amino-1-pentanol, 6-amino-1-hexanol or higher homologs thereof, 4-(2-aminoethyl)-2-hydroxyethylbenzene, 3-aminomethyl-3,5,5-trimethyl-cyclohexanol, 2-(2-aminoethoxy)-ethanol, triethylene glycol-monoamine or higher homologs thereof, 3-(2-hydroxyethoxy)-propylamine, 3-(2-(2-hydroxyethoxy)-ethoxy)-propylamine, 3-(6-hydroxyhexyloxy)-propylamine, N-methyl-1,3-propanediamine, N-ethyl-1,3-propanediamine, N-butyl-1,3-propanediamine, N-(2-ethylhexyl)-1,3-propanediamine, N-dodecyl-1,3-propanediamine, N-cyclohexyl-1,3-propanediamine, N-coco alkyl-1,3-propanediamine, N-oleyl-1,3-propanediamine, N-soya alkyl-1,3-propanediamine, N-tallowalkyl-1,3-propanediamine and N-(C₁₆₋₂₂-alkyl)-1,3-propanediamine.

7. Composition according to one of the preceding claims, wherein the composition is two-component and consists of the component **K1** that contains
a) At least one aldimine **A** of Formula (I), and
b) At least one substance **RS**, which has at least two groups that are reactive to isocyanate groups, and/or water,
and the component **K2** that contains
c) At least one polyisocyanate **P.**

8. Addition product **AV** obtained from the reaction of an aldimine **A** of Formula (I) with a polyisocyanate **P**, as they are present in a composition according to one of Claims 1 to 7.

9. Addition product according to Claim 8, wherein the addition product **AV** has Formula (XVII) 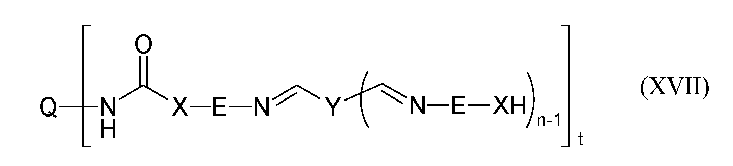 whereby Q stands for the radical of a polyisocyanate **P** after removal of t isocyanate groups, and t stands for 2 or 3, in particular for 2.

10. Addition product according to Claim 8, wherein the addition product **AV** has Formula (XVIII) 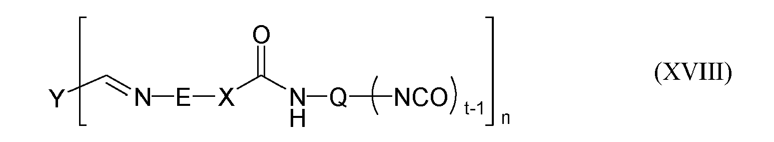 whereby Q stands for the radical of a polyisocyanate **P** after removal of t isocyanate groups, and t stands for 2 or 3, in particular for 2.

11. Aldimine of Formula (III) 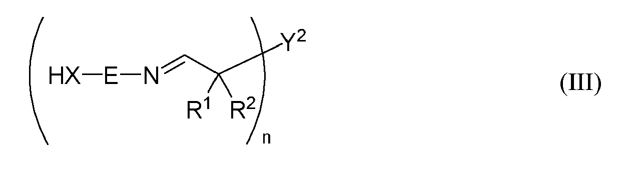 whereby
R¹ and R² either
independently of one another in each case stand for a monovalent hydrocarbon radical with 1 to 12 C atoms,
or together stand for a divalent hydrocarbon radical with 4 to 12 C atoms, which is part of an optionally substituted, carbocyclic ring with 5 to 8, preferably 6, C atoms;
Y² stands for an n-value organic radical with 1 to 24 C atoms, which optionally
has nitrogen and/or oxygen atoms in the form of tertiary amino groups, or ether, ester, carbonate, amide, urethane or urea groups;
E either stands for a divalent hydrocarbon radical with 3 to 20 C atoms,
or together with R¹¹ for a trivalent hydrocarbon radical with 3 to 20 C atoms, whereby E optionally contains heteroatoms in the form of ether- oxygen or tertiary amine-nitrogen,
X stands for O or S or N-R¹⁰ or N-R¹¹, whereby
R¹⁰ stands for a monovalent hydrocarbon radical with 1 to 20 C atoms, which optionally has at least one carboxylic acid ester, nitrile, nitro, phosphonic acid ester, sulfonic or sulfonic acid ester group, and
R¹¹ together with E stands for a trivalent hydrocarbon radical with 3 to 20
C atoms, which optionally contains heteroatoms in the form of ether-oxygen or tertiary amine-nitrogen;
and n stands for 2 or 3 or 4.

12. Aldimine according to Claim 11, wherein the aldimine has Formula (III a) 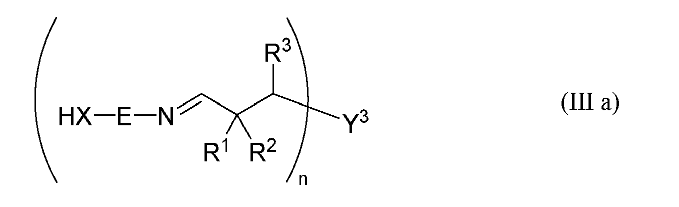 whereby
R³ stands for a hydrogen atom or for an alkyl, cycloalkyl, arylalkyl or alkoxycarbonyl radical with 1 to 12 C atoms;
Y³ stands for an n-value radical that is selected from the group that consists of 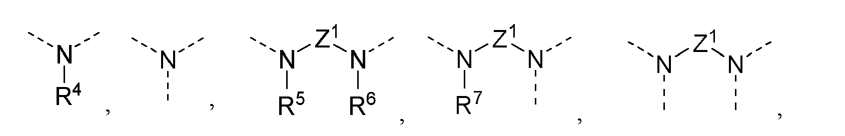 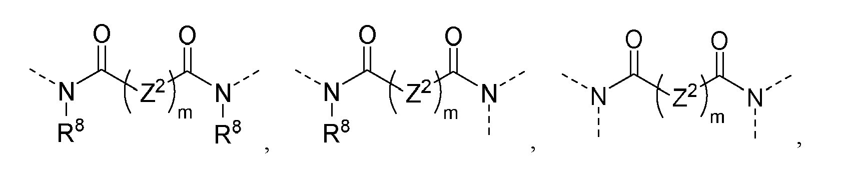 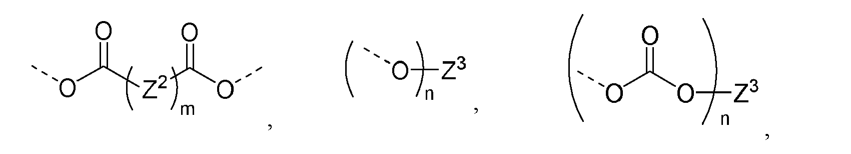 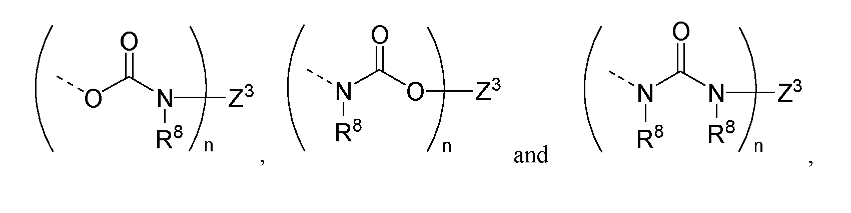
whereby
m stands for 0 or 1;
Z¹ either stands for a carbonyl group or for an alkylene radical with 2 to 15 C atoms, which optionally has at least one ether group;
Z² stands for a divalent hydrocarbon radical with 1 to 15 C atoms, which optionally has at least one ether, carbonyl or carboxyl group;
Z³ stands for an n-value hydrocarbon radical with 2 to 15 C atoms, which optionally has at least one ether or carbonyl group;
R⁴ stands for an alkyl, cycloalkyl or arylalkyl radical with 1 to 20 C atoms;
R⁵ and R⁶ cycloalkyl either independently of one another in each case stand for an alkyl, or
arylalkyl radical with 1 to 12 C atoms, or, for the case that Z¹ stands for a carbonyl group, independently of one
another in each case stand for a hydrogen atom or for an alkyl, cycloalkyl or arylalkyl radical with 1 to 12 C atoms;
or together for an alkylene radical with 2 to 20 C atoms, which
together with N-Z¹-N forms a 5- to 12-membered ring and optionally has at least one ether group;
R⁷ either
stands for an alkyl, cycloalkyl or arylalkyl radical with 1 to 15 C atoms;
or, for the case that Z¹ stands for a carbonyl group, for a hydrogen atom or
for an alkyl, cycloalkyl or arylalkyl radical with 1 to 15 C atoms;
and R⁸ radical stands for a hydrogen atom or for an alkyl, cycloalkyl or arylalkyl with 1 to 8 C atoms.

13. Method for gluing a substrate **S1** to a substrate **S2**, which comprises the steps:
i) Application of a composition according to one of Claims 1 to 7 to a sub strate
**S1**;
ii) Bonding of the applied composition to a substrate **S2** within the open time of
the composition;
or
i') Application of a composition according to one of Claims 1 to 7 to a sub strate
**S1** and to a substrate **S2**;
ii') Bonding of the applied composition together within the open time of the composition;
whereby the substrate **S2** consists of the same material or a different material such as the substrate **S1**.

14. Use of a composition according to one of Claims 1 to 7 as an adhesive, sealant, filling compound, coating, floor covering, paint, varnish or primer.

15. Cured composition obtained from a composition according to one of Claims 1 to 7 and moisture.

## Revendications

1. Composition contenant
a) au moins une aldimine **A** de formule (I) 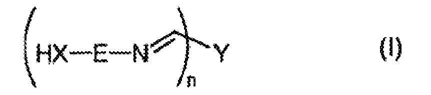 où n vaut 2 ou 3 ou 4,
E représente soit un radical hydrocarboné divalent comprenant 3 à 20 atomes de carbone, ou, ensemble avec R¹¹, un radical hydrocarboné trivalent comprenant 3 à 20 atomes de carbone, où E contient le cas échéant des hétéroatomes sous forme d'oxygène de fonction éther ou d'azote de fonction amine tertiaire,
Y représente un radical organique n-valent, comprenant 6 à 30 atomes de carbone, qui présente le cas échéant des atomes d'azote et/ou d'oxygène sous forme de groupes amino tertiaire, de groupes éther, ester, carbonate, amide, uréthane ou urée,
X représente O ou S ou N-R¹⁰ ou N-R¹¹, où
R¹⁰ représente un radical hydrocarboné monovalent comprenant 1 à 20 atomes de carbone, qui présente le cas échéant au moins un groupe ester d'acide carboxylique, nitrile, nitro, ester d'acide phosphonique, sulfone ou ester d'acide sulfonique, et
R¹¹ représente, ensemble avec E, un radical hydrocarboné trivalent comprenant 3 à 20 atomes de carbone, qui contient le cas échéant des hétéroatomes sous forme d'oxygène de fonction éther ou d'azote de fonction amine tertiaire ; et
b) au moins un polyisocyanate **P**.

2. Composition selon la revendication 1, **caractérisée en ce que** l'aldimine **A** présente la formule (II) 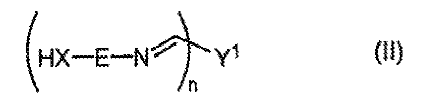 où Y¹ représente un groupe aryle ou hétéroaryle n-valent, substitué ou non substitué, qui présente une taille de cycle de 5 à 8, de préférence de 6, atomes.

3. Composition selon la revendication 1, **caractérisée en ce que** l'aldimine **A** présente la formule (III) 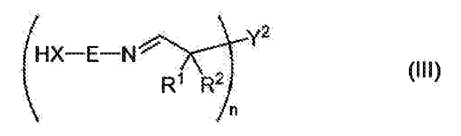 où
R¹ et R² représentent soit, indépendamment l'un de l'autre, à chaque fois un radical hydrocarboné monovalent comprenant 1 à 12 atomes de carbone, ou ensemble, un radical hydrocarboné divalent comprenant 4 à 12 atomes de carbone, qui fait partie d'un cycle carbocyclique, le cas échéant substitué, comprenant 5 à 8, de préférence 6, atomes de carbone ;
Y² représente un radical organique n-valent comprenant 1 à 24 atomes de carbone, qui présente le cas échéant des atomes d'azote et/ou d'oxygène sous forme de groupes amino tertiaire, de groupes éther, ester, carbonate, amide, uréthane ou urée.

4. Composition selon la revendication 3, **caractérisée en ce que** l'aldimine **A** présente la formule (IIIa) 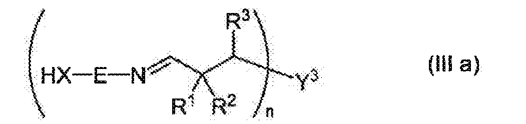
où R³ représente un atome d'hydrogène ou un groupe alkyle, cycloalkyle, arylalkyle ou alcoxycarbonyle comprenant 1 à 12 atomes de carbone ;
Y³ représente un radical n-valent, qui est choisi dans le groupe constitué par 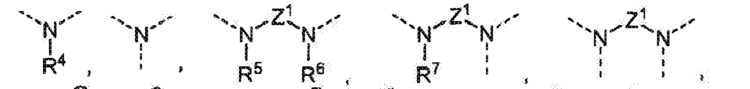 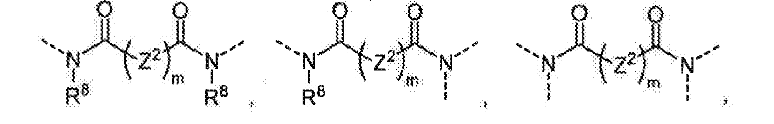 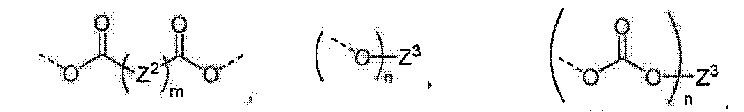 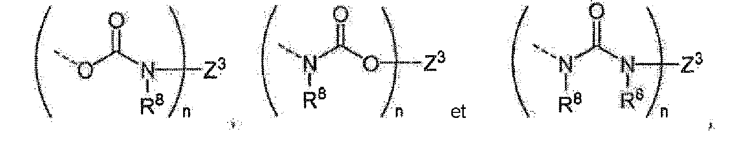
où
m vaut 0 ou 1 ;
Z¹ représente soit un groupe carbonyle, soit un radical alkylène comprenant 2 à 15 atomes de carbone, qui présente le cas échéant au moins un groupe éther ;
Z² représente un radical hydrocarboné divalent comprenant 1 à 15 atomes de carbone, qui présente le cas échéant au moins un groupe éther, carbonyle ou carboxyle ;
Z³ représente un radical hydrocarboné n-valent comprenant 2 à 15 atomes de carbone, qui présente le cas échéant au moins un groupe éther ou carbonyle ;
R⁴ représente un radical alkyle, cycloalkyle ou aralkyle comprenant 1 à 20 atomes de carbone ;
R⁵ et R⁶ représentent soit, indépendamment l'un de l'autre, un radical alkyle, cycloalkyle ou aralkyle comprenant 1 à 12 atomes de carbone, ou pour le cas où Z¹ représente un groupe carbonyle, indépendamment l'un de l'autre à chaque fois un atome d'hydrogène ou un radical alkyle, cycloalkyle ou arylalkyle comprenant 1 à 12 atomes de carbone ; ou
ensemble un radical alkylène comprenant 2 à 20 atomes de carbone, qui forme ensemble avec N-Z¹-N un cycle de 5 à 12 chaînons et qui présente le cas échéant au moins un groupe éther ;
R⁷ représente soit un radical alkyle, cycloalkyle ou aralkyle comprenant 1 à 15 atomes de carbone ; ou pour le cas où Z¹ représente un groupe carbonyle, un atome d'hydrogène ou un radical alkyle, cycloalkyle ou arylalkyle comprenant 1 à 15 atomes de carbone ; et
R⁸ représente un atome d'hydrogène ou un radical alkyle, cycloalkyle ou aralkyle comprenant 1 à 8 atomes de carbone.

5. Composition selon la revendication 4, **caractérisée en ce que** Y³ représente le radical

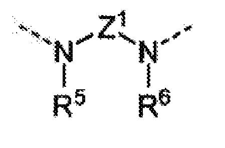

6. Composition selon l'une quelconque des revendications précédentes, **caractérisée en ce que** l'aldimine de formule (I) est préparée à partir de la transformation d'au moins une amine **B** de formule (IV) avec au moins un polyaldéhyde **ALD** de formule (V)
HX-E-NH₂ (IV) 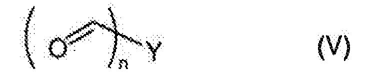
et l'amine **B** de formule (IV) est choisie dans le groupe constitué par le 5-amino-1-pentanol, le 6-amino-1-hexanol ou ses homologues supérieurs, le 4-(2-aminoéthyl)-2-hydroxyéthylbenzène, le 3-aminométhyl-3,5,5-triméthylcyclohexanol, le 2-(2-aminoéthoxy)-éthanol, la triéthylèneglycolmonoamine ou ses homologues supérieurs, la 3-(2-hydroxyéthoxy)-propylamine, 1 a 3-(2-(2-hydroxyéthoxy)-éthoxy)-propylamine, la 3-(6-hydroxyhexyloxy)-propylamine, la N-méthyl-1,3-propanedi aminé , la N-éthyl-1,3-propanediamine, la N-butyl-1,3-propanediamine, la N-(2-éthylhexyl) -1,3-propanediamine, la N-dodécyl-1, 3-propanediamine, la N-cyclohexyl-1,3-propanediamine, la N-coco-alkyl-1,3-propanediamine, la N-oléyl-1,3-propanediamine, la N-soja-alkyl-1,3-propanediamine, la N-suif-alkyl-1,3-propanediamine et la N-(C₁₆₋₂₂-alkyl)-1,3-propanediamine.

7. Composition selon l'une quelconque des revendications précédentes, **caractérisée en ce que** la composition est à deux composants et constituée par le composant **K1** contenant
a) au moins une aldimine **A** de formule (I) et
b) au moins une substance **RS**, qui présente au moins deux groupes réactifs par rapport à isocyanate et/ou de l'eau,
le composant **K2** contenant
c) au moins un polyisocyanate **P**.

8. Produit d'addition **AV** obtenu à partir de la réaction d'une aldimine **A** de formule (I) avec un polyisocyanate **P**, tels qu'ils sont présents dans une composition selon l'une quelconque des revendications 1 à 7.

9. Produit d'addition selon la revendication 8, **caractérisé en ce que** le produit d'addition **AV** présente la formule (XVII) 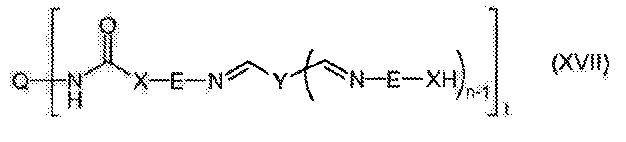 où Q représente le radical d'un polyisocyanate **P** après élimination de t groupes isocyanate et t vaut 2 ou 3, en particulier 2.

10. Produit d'addition selon la revendication 8, **caractérisé en ce que** le produit d'addition **AV** présente la formule (XVIII) 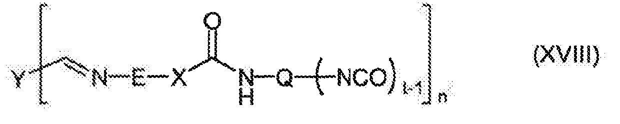 où Q représente le radical d'un polyisocyanate **P** après élimination de t groupes isocyanate et t vaut 2 ou 3, en particulier 2.

11. Aldimine de formule (III) 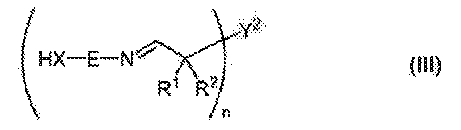 où
R¹ et R² représentent soit, indépendamment l'un de l'autre, à chaque fois un radical hydrocarboné monovalent comprenant 1 à 12 atomes de carbone, ou ensemble, un radical hydrocarboné divalent comprenant 4 à 12 atomes de carbone, qui fait partie d'un cycle carbocyclique, le cas échéant substitué, comprenant 5 à 8, de préférence 6, atomes de carbone ;
Y² représente un radical organique n-valent, comprenant 1 à 24 atomes de carbone, qui présente le cas échéant des atomes d'azote et/ou d'oxygène sous forme de groupes amino tertiaire, de groupes éther, ester, carbonate, amide, uréthane ou urée ;
E représente soit un radical hydrocarboné divalent comprenant 3 à 20 atomes de carbone, ou, ensemble avec R¹¹, un radical hydrocarboné trivalent comprenant 3 à 20 atomes de carbone, où E contient le cas échéant des hétéroatomes sous forme d'oxygène de fonction éther ou d'azote de fonction amine tertiaire,
X représente O ou S ou **N-R¹⁰** ou N-R¹¹, où
R¹⁰ représente un radical hydrocarboné monovalent comprenant 1 à 20 atomes de carbone, qui présente le cas échéant au moins un groupe ester d'acide carboxylique, nitrile, nitro, ester d'acide phosphonique, sulfone ou ester d'acide sulfonique, et
R¹¹ représente, ensemble avec E un radical hydrocarboné trivalent comprenant 3 à 20 atomes de carbone, qui contient le cas échéant des hétéroatomes sous forme d'oxygène de fonction éther ou d'azote de fonction amine tertiaire ; et
n vaut 2 ou 3 ou 4.

12. Aldimine selon la revendication 11, **caractérisée en ce que** l'aldimine présente la formule (III a) 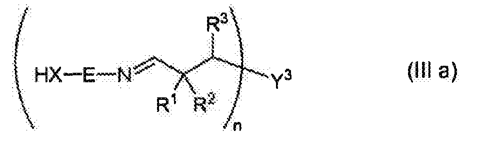 où
R³ représente un atome d'hydrogène ou un groupe alkyle, cycloalkyle, arylalkyle ou alcoxycarbonyle comprenant 1 à 12 atomes de carbone ;
Y³ représente un radical n-valent, qui est choisi dans le groupe constitué par 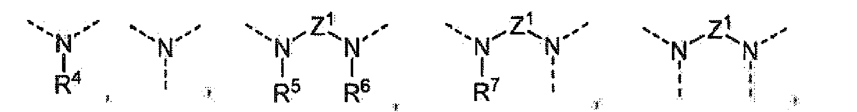 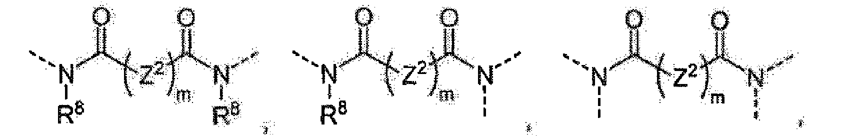 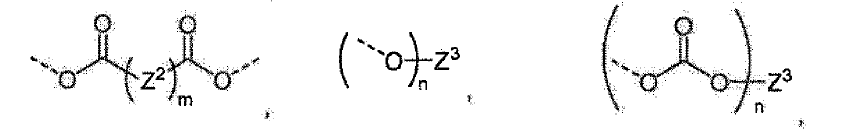 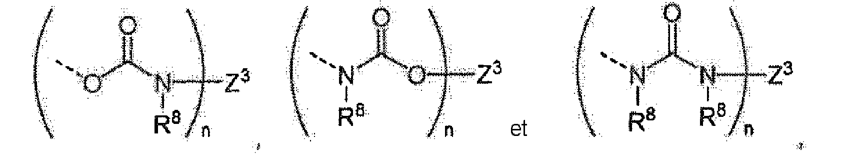 où
m vaut 0 ou 1 ;
Z¹ représente soit un groupe carbonyle, soit un radical alkylène comprenant 2 à 15 atomes de carbone, qui présente le cas échéant au moins un groupe éther ;
Z² représente un radical hydrocarboné divalent comprenant 1 à 15 atomes de carbone, qui présente le cas échéant au moins un groupe éther, carbonyle ou carboxyle ;
Z³ représente un radical hydrocarboné n-valent comprenant 2 à 15 atomes de carbone, qui présente le cas échéant au moins un groupe éther ou carbonyle ;
R⁴ représente un radical alkyle, cycloalkyle ou aralkyle comprenant 1 à 20 atomes de carbone ;
R⁵ et R⁶ représentent soit, indépendamment l'un de l'autre, un radical alkyle, cycloalkyle ou aralkyle comprenant 1 à 12 atomes de carbone, ou pour le cas où Z¹ représente un groupe carbonyle, indépendamment l'un de l'autre à chaque fois un atome d'hydrogène ou un radical alkyle, cycloalkyle ou arylalkyle comprenant 1 à 12 atomes de carbone ; ou
ensemble un radical alkylène comprenant 2 à 20 atomes de carbone, qui forme ensemble avec N-Z¹-N un cycle de 5 à 12 chaînons et qui présente le cas échéant au moins un groupe éther ;
R⁷ représente soit un radical alkyle, cycloalkyle ou aralkyle comprenant 1 à 15 atomes de carbone ; ou pour le cas où Z¹ représente un groupe carbonyle, un atome d'hydrogène ou un radical alkyle, cycloalkyle ou arylalkyle comprenant 1 à 15 atomes de carbone ;
et R⁸ représente un atome d'hydrogène ou un radical alkyle, cycloalkyle ou aralkyle comprenant 1 à 8 atomes de carbone.

13. Procédé pour le collage d'un substrat **S1** avec un substrat **S2**, qui comprend les étapes
i) application d'une composition selon l'une quelconque des revendications 1 à 7 sur un substrat **S1** ;
ii) mise en contact de la composition appliquée avec un substrat **S2** pendant le temps ouvert de la composition ; ou
i') application d'une composition selon l'une quelconque des revendications 1 à 7 sur un substrat **S1** et sur un substrat **S2** ;
ii') mise en contact de la composition appliquée l'une avec l'autre pendant le temps ouvert de la composition ;
le substrat **S2** étant constitué par le même matériau que le substrat **S1** ou par un matériau différent du substrat **S1**.

14. Utilisation d'une composition selon l'une quelconque des revendications 1 à 7 comme adhésif, masse d'étanchéité, masse de scellement, revêtement, revêtement de sol, enduit, laque ou apprêt.

15. Composition durcie obtenue à partir d'une composition selon l'une quelconque des revendications 1 à 7 et de l'humidité.
